(19) Europäisches Patentamt
European Patent Office
Office européen des brevets

(11) Publication number: **0 128 536**
**B1**

# (12) EUROPEAN PATENT SPECIFICATION

(45) Date of publication of patent specification: **13.09.89**

(51) Int. Cl.[4]: **C 07 D 498/04,** A 61 K 31/535

(21) Application number: **84106512.1**

(22) Date of filing: **07.06.84**

## (54) Fluoromethylthiooxacephalosporin derivatives.

(30) Priority: **14.06.83 JP 106293/83**

(43) Date of publication of application: **19.12.84 Bulletin 84/51**

(45) Publication of the grant of the patent: **13.09.89 Bulletin 89/37**

(84) Designated Contracting States: **DE FR IT**

(56) References cited:
**DE-A-3 041 160**
**US-A-4 122 262**

(73) Proprietor: **SHIONOGI & CO., LTD.**
**1-8, Doshomachi 3-chome Chuo-ku**
**Osaka 541 (JP)**

(72) Inventor: **Tsuji, Teruji**
**1-14-9, Yanagawa-cho**
**Takatsuki-shi Osaka (JP)**
Inventor: **Sato, Hisao**
**12-4, Higashimatsugaoka**
**Ikoma-shi Nara (JP)**
Inventor: **Hamashima, Yoshio**
**26-20, Hitsujisaru-cho Katsura**
**Nishikyo-ku Kyoto-shi Kyoto (JP)**

(74) Representative: **Hranitzky, Wilhelm Max et al**
**NOVAPAT - CABINET CHEREAU 9, rue du Valais**
**CH-1202 Genève (CH)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 128 536 B1**

Courier Press, Leamington Spa, England.

**Description**

This invention relates to a novel antibacterial, 7 β-(fluorinated methylthioacetamido)-7α-methoxy-3-(nucleophilic group-substituted methyl)-1-dethia-1-oxa-3-cephem-4-carboxylic acid (I) or its pharmaceutically acceptable salts and esters represented by the following formula

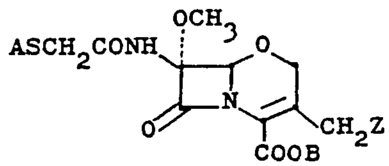

(1)

(wherein

A is monofluoromethyl or difluoromethyl and

B is hydrogen or a metal forming a physiologically acceptable ion or a pharmaceutically acceptable carboxy protecting ester forming group having 1 to 20 carbon atoms; and

Z is a halogen or an acyloxy or heterocyclyl thio group having up to 15 carbon atoms, wherein the heterocyclic part of the heterocyclyl thio group is a mono or di-cyclic group provided Z is not 1-(optionally protected hydroxyalkyl)-1H-tetrazol-5-ylthio.

Preferably A group is difluoromethyl.

The group B is preferably hydrogen or a salt forming atom or carboxy protecting ester forming group.

Carboxy-protecting ester forming group has up to 20C atoms and is known in the penicillin and cephalosporin fields and can be introduced or deprotected without adverse effect on other part of molecule (aralkyl ester (e.g., benzyl, methylbenzyl, dimethylbenzyl, p-methoxybenzyl, ethoxybenzyl, p-nitrobenzyl, aminobenzyl, diphenylmethyl, phthalidyl., phenacyl esters), substituted alkyl ester (e.g. 2,2,2-trichloroethyl, t-butyl, allyl, esters), aryl ester (e.g., pentachlorophenyl, indanyl, esters), N-hydroxyamino ester (ester with e.g., acetone oxim, acetophenone oxim, acetaldoxim, N-hydroxysuccinimide, N-hydroxyphthalimide), anhydride with carbonic or carboxylic acid.

The medical ester forming group is a potent antibacterial on taking orally or parenterally including a well known substituted alkyl ester (e.g., acetoxymethyl, pivaloyloxymethyl, ethoxyformyloxymethyl, 4-methyl-2-oxo-1,3-dioxolenylmethyl esters), substituted aralkyl ester (e.g., phenacyl, phthalidyl esters) or substituted aryl ester (e.g., phenyl, xylyl, indanyl esters).

A preferable light metal salt is that forms a physiologically acceptable ion and belongs to the 1st and 3rd group, 2nd and 4th series of the Periodical Table. Lithium, sodium, potassium, magensium, calcium and aluminum are preferable.

In the formula (I), Z has up to 15C atoms. Halogen represented by Z can be chlorine or bromine; acyloxy represented by Z can be alkanoyloxy or carbamoyloxy; and the heterocyclic part in Z can be a mono- or di-cyclic group having nitrogen, oxygen or sulfur hetero atom. The hetereocycle may be optionally hydrogenated or substituted by optionally substituted alkyl, optionally protected carboxy, cyano, optionally substituted phenyl, heterocyclic group, amino, acylamino, hydroxy, alkoxy, oxo, mercapto or alkylthio.

Representative heterocyclic part in Z is imidazole, pyrazole, oxazole, isoxazole, thiazole, isothiazole, triazole, oxadiazole, thiadiazole, tetrazole, pyrimidine, triazine, triazolopyrimidine, pyrazolotetrazole, pyrazolotriazole, pyrazolotriazin, tetrazolopyridazine or benzotriazole. Most preferable are tetrazole, thiadiazole, triazole, and triazine. Alkyl part of said substituted or unsubstituted alkyl may be a straight, branched, unsaturated, cyclic, or partially cyclic alkyl and preferably be 1 to 4C alkyl. The said substituent can be halogen (e.g., chlorine, bromine, fluorine), or preferably up to 15C sulfur function (e.g., mercapto, acylthio, alkylthio, carbamoylalkylthio, arylthio, heterthio, sulfamoyl, sulfo), oxygen function (e.g., hydroxy, acyloxy, alkoxy, carboxyalkoxy, oxo), nitrogen function (e.g., alkanoylamino, aroylamino, alkylamino, dialkylamino, ureido), carbon function (e.g., vinyl, allyl, aryl, cyano, acyl, carboxy, protected carboxy, carbalkoxy, carbaralkoxy, carbamoyl, carbonyl, alkoxycarbamoyl, alkylcarbamoyl, arylcarbamoyl, N-heterocyclic carbamoyl, heterocyclic ureidocarbonyl). If these functions have some part unsuitable for synthesis or use, they can conventionally be protected to give other members of this group.

Said acyl part is straight, branched, or cyclic alkanoyl, alkenoyl, carbalkoxy, carbamoyl, sulfo, alkylsulfonyl, sulfamoyl, monocyclic or dicyclic aroyl, aralkanoyl, aryalkenyol, carbaralkoxy or arylsulfonyl in which aryl part can be heterocyclic having nitrogen, oxygen or sulfur hetero atom.

The aryl part may be phenyl or said heterocyclic group.

The said groups can be substituted. They can be one or more of carbon function (e.g., alkyl, alkenyl, alkylidene, alkinyl, aralkyl, aryl, carboxy, protected carboxy, carbamoyl, alkanoyl, alkenoyl, aralkanoyl, aroyl, aminoalkyl, cyano), nitrogen function (e.g., amino, hydrazinyl, azido, diazo, alkylamino, arylamino, acylated amino, alkylideneamino, imino, nitroso, nitro), oxygen functions (e.g., hydroxy, alkoxy, aralkoxy, aryloxy, acyloxy, oxo), sulfur group (e.g., mercapto, alkylthio, arylthioi, acylthio, thioxo, sulfinyl, sulfonyl, sulfo, protected sulfo), phosphorus function (e.g., phospho) or halogen atom (e.g., fluorine, chlorine, bromine, iodo) and may form a cycle.

Said group having a reactive functional group can be protected conventionally for protecting from an adverse change during the reactions: e.g, hydrocarbon group, acyl, alkylated silyl, alkoxy, silyl, and alkylphospinyl for hydroxy, amino or mercapto; e.g., ester, acid anhydride, amide, hydrazide for carboxy or sulfo; and other conventional protective groups, especially those introduced or removed without adverse effect on other part of the molecule.

Preferable Z group has heterocyclic group selected from tetrazolyl, thiadiazolyl, triazinyl, or triazolyl.

More preferable Z group is 1-subsituted 1H-tetrazol-5-yl-thio, in which the substitution is selected substituent is one of methyl, vinyl, carbamoylmethyl, 2-carbamoylethyl, cyanomethyl, 2-cyanoethyl, 2-oxo-imidazolidin-1-yl, 2-dimethylaminoethyl, chloromethyl, difluoromethyl, 2-sulfamoylethyl, and 2-methanesulfonylaminoethyl.

Other preferable Z is acetoxy, carbamoyloxy, N-protected carbamoyloxy, or halo.

Compound (I) is antibacterial against aerobic or anaerobic and Gram-positive (e.g., Staphylococci) or Gram-negative (e.g., Escherichia coli) bacteria and useful as a bacteriocidal or bacteriostatic human or veterinary medicine for preventing or treating infection, as a disinfectant, preservative, or antiperishable.

The Compound (I) is stable and superior due to its activity against bacteria resistant to other drugs, and its pharmacological characters (e.g., absorption, distribution, metabolism, excretion).

For medical use, it is administered externally, orally, topically or by injecting to prevent or treat an infection caused by sensitive bacteria at a daily dose of 10 micrograms to 1 milligram externally, 0.2 to 5 gram intravenously, or 1 to 2 gram orally of Compound (I), if required formulating with conventional additives.

Such pharmaceutical preparation can be an ampoule, vial, powder, pellet, granule, capsule, tablet, dry syrup, suspension, solution, emulsion, ointment, injection, oral drug, inhalant, pap, ocular solution, nasal preparation, ear solution, trouch, suppository or spray for enteral, parenteral, topical, or local application.

The carboxylic acid or its light metal salt as Compound (I) can be used as an intravenous injection, drip, intramuscular or subcutaneous injection (e.g., vial, ampoule), if required in admixture with an excipient (e.g., stabilizer, solubilizer). A pharmacological ester can be used orally (e.g., powder, pellet, granule, capsule, dry syrup, solution, tablet, suspension) or externally or topically (e.g., solution, ointment, emulsion, suppository, spray).

Compound (I) can also be used as a raw material for assaying sensitivity of bacteria or a starting material for synthesizing other antibacterial within or without the scope of this invention.

Similar compounds having the same nucleus have been known by Japanese patent application Kokai 49—133594 and 51—41385, but these are not superior to the compounds of this invention in its broadness and intensity of antibacterial spectra.

Other similar compounds having the same nucleus (1-oxadethiacephalosporins), but differing in the substitution of the 7 β position, are disclosed in German patent application DE—A—3 041 160.

7-acylamino-8-oxo-3-oxa-1-azabicyclo [4.2.0]-octane-2-carboxylic acid derivatives are described in United States patent US—A—4 122 262.

The corresponding 1-thia analogs (having conventional cephem ring) are better antibacterials than the compounds of this invention.

Compound (I) can be synthesized, for example, by the following methods.

(1) Salt formation

Compound (I) having 4-carboxy on cephem nucleus can form a corresponding light metal salt by reacting with a base or by exchange decomposition with a light metal salt of other carboxylic acid. The procedure can be those conventional in the art, e.g., by neutralizing the free acid with light metal hydrocarbonate, or by treating with alkali metal lower carboxylate in a polar organic solvent (e.g., alcohol, ketone, ester) and then adding sparingly soluble solvent. The reaction ends usually in 1 to 10 minutes at lower than 50°C. The time may be longer, if no side reaction occurs.

Antibacterial preparations can be made by separating thus produced salts as solids (e.g., crystals, powders) or by lyophilizing.

(2) Deprotection of carboxy-protecting groups

Following conventional deprotections of protected carobxy in Compound (I) gives Carboxy compounds (I):

a) Highly reactive ester, amide, or anhydride as a carboxy protecting group can be deprotected in an aqueous solvent with an acid, base, buffer solution, or ion exchange resin. When the reactivity is insufficient, man can increase it in a conventional manner to deprotect more easily (by treatment of trichloroethyl, p-nitrobenzyl, phenacyl esters with e.g., metal or acid, catalytic hydrogenation, dithionate).

b) Aralkyl esters can be deprotected by catalytic reduction. The reaction can be done conventionally with hydrogen in the presence of a catalyst, e.g., palladium nickel.

c) Aralkyl, cyclopropylmethyl, sulfonylethyl esters can be deprotected by solvolyzing with [e.g., mineral acid, Lewis acid (e.g., aluminium chloride, tin chloride, titanium tetrachloride), sulfonic acid (e.g., methanesulfonic acid, trifluoromethanesulfonic acid), strong carboxylic acid (e.g., trifluoroacetic acid)], if required in the presence of a cation scavenger.

d) Phenacyl, alkenyl, hydroxyaralkyl esters can be deprotected with a base or nuclepholic reagent. Some phenacyl esters are deprotected by irradiation.

e) 2-Alkenyl ester is deprotected with an alkali metal alkanoate and palladium-triphenylphosphine complex giving alkali metal salt.

f) and other equivalent carboxy deprotections.

(3) Introduction of the 3-substituent

Compounds of the formula (I) but having 3-methyl substituted by a leaving group or atom can be treated with heteroaromatic thiol, aromatic base or its reactive derivative giving the objective compound (I). Here the leaving group is preferably reactive halogen, sufonyloxy, alkanoyloxy, dihaloacetoxy, trihaloacetoxy, or the like. Preferable reactive derivatives of said thiol is alkali metal salts, ammonium salt, carboxylate ester, and the like. The reaction goes at 0 to 60°C even in anhydrous or aqueous solvent. This reaction is accelerated with a dehydrating reagent, phosphoryl chlorides, rhodanates, or the like.

Compounds (I) having 3-carbamoyloxymethyl can be produced by reacting the 4-carboxy protected 3-hydroxy-methyl compound (I) with a reactive derivative of N-protected carbamic acid and the product is deprotected keeping the carbamoyloxy group.

(4) Amidation

A reaction of Amine (II) or its reactive with Carboxylic acid (III) or its reactive derivative gives Amide (I) or its derivatives.

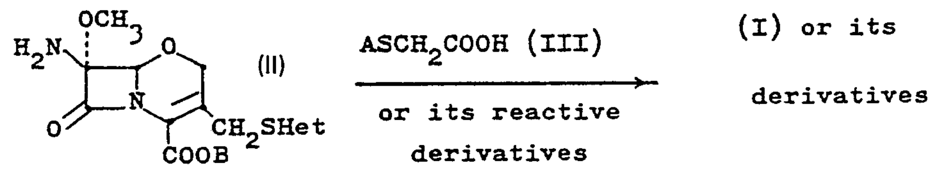


The reactive derivative of Amine (II) is that having 7-amino activated by silyl (e.g., trimethylsilyl, methoxydimethylsilyl, t-butyldimethylsilyl), stannyl (e.g., trimethylstannyl), alkylene (as a part of enamino of the amino with, e.g., aldehyde, acetone, acetylacetone, acetoacetate, acetoacetonitrile, acetoacetanilide, cyclopentanedione, acetylbutyrolactone), alkylidene (e.g., 1-haloalkylidene, 1-haloaralkylidene, 1-alkoxyalkylidene, 1-alkoxy-1-phenoxyalkylidene, alkylidene), acid (e.g., mineral acid, carboxylic acid or sulfonic acid as a salt of the amino), easily removable acyl (e.g., alkanoyl) or that protected at other functions of the molecule.

The reactive derivatives of Carboxylic acid (III) can be acid anhydride, halide, reactive ester, reactive amide or azide, conventional derivative for acylation.

Followings are procedures for said acylation.

i) Free acid (III) — In the presence of a condensing reagent [carbodiimide (e.g., N,N'-diethylcarbodiimide, N,N'-dicyclohexylcarbodiimide), carbonyl compound (e.g., carbonyl diimidazole), isoxazolinium salt, acylamino compound (e.g., 2-ethoxy-1-ethoxycarbonyl-1,2-dihydroquinoline) or amidase], Amine (II) is treated with Acid (III) preferably in an aprotic solvent (e.g., halohydrocarbon, nitrile, ether or amide solvent or the mixture), preferably 1 to 2 molar equivalents of Carboxylic acid (III) and 1 to 2 molar equivalents of the condensing reagent against Amine (II).

ii) Acid anhydride — This includes a symmetric anhydride and mixed anhydride [with e.g., mineral acid (e.g., phosphoric acid, sulfuric acid, carbonic half ester), organic acid (e.g., alkanoic acid, aralkanoic acid, sulfonic acid), intramolecular anhydride (e.g., ketene, isocyanate), and hydrohalogenic acid (i.e., acid halide)] of Carboxylic acid (III).

Preferably, Amine (II) or its reactive derivative is amidated with 1 to 2 molar equivalents of the acid anhydride in the presence of 0 to 1 molar equivalent of an acid scavenger [e.g., inorganic base (e.g., oxide, hydroxide, carbonate or hydrogen carbonate, of alkali metal or alkaline earth metal), organic base (e.g., tertiary amine, aromatic base); oxirane (e.g., alkylene oxide, aralkylene oxide); pyridinium salt (e.g., tripyridiniumtriazine trichloride); adsorbing agent (e.g., Celite); preferably in an aprotic solvent (e.g., halohydrocarbon, nitrile, ether or amide solvent or a mixture) thereof.

iii) Acid halide — This is a mixed anhydride of Carboxylic acid (III) with hydrogen halide. Thus, 1 to 2 molar equivalents of this acid halide is preferably reacted with 1 molar equivalent of Amine (II) or its reactive derivative as given above item ii) in the presence of 1 to 10 molar equivalents of said acid scavenger in a solvent (e.g., halohydrocarbon, nitrile, ether, ester, ketone, dialkylamide or water solvent or a mixture thereof).

iv) Reactive ester — This includes an enol ester (e.g., vinyl ester, isopropenyl ester), aryl ester (e.g., phenyl ester, halophenyl ester, nitrophenyl ester), heterocyclic ester (e.g., 1-hydroxybenzotriazole ester), an ester with N-hydroxycompound, diacylhydroxylamine ester or thioester having conventional reactive ester group. This is used as described below. Enzymatically reactive esters (e.g., lower alkyl ester) can be used conventionally in an aqueous solvent in the presence of an amidase.

v) Reactive amide — This includes an aromatic amide (amide with e.g., imidazole, triazole, 2-ethoxy-1,2-dihydroquinoline) or diacylanilide of Acid (III) treated as given below and

vi) Formimino compound (N,N-dimethylformimido ester halide, etc.) of Acid (III).

The reaction from iv) to vi) are carried out by treating 1 molar equivalent of Amine (II) or its reactive derivative with 1 to 2 molar equivalents of Carboxylic acid (III) or its reactive derivative in an aprotic solvent (halohydrocarbon, ether, ketone, nitrile, amide, ester solvents or mixture) for 1 to 5 hours at −20°C to 40°C.

(5) Methoxylation

Compounds of the formula (I) but having 7-hydrogen is treated with an N-halogenating reagent, dehydrohalogenating reagent, and methanol to give the corresponding Compound (I) from either 7α- or 7β-hydrogen compound.

The procedures are, for example, as follows:

a) Reacting with alkyl hypochlorite (e.g., t-butyl hypochlorite) and alkali metal methoxide (e.g., lithium methylate, sodium methylate) in methanol.

b) Reacting with molecular halogen and a base (metal alkoxide e.g. lithium methoxide, sodium methoxide, magnesium methoxide, DBU, triethylamine, picoline) in methanol.

c) Reacting with N-halogenating reagent (e.g., hypohalite salt, hypohalite ester, N-haloamide, N-haloimide) and dehydrohalogenating reagent (e.g., alkali metal alkoxide, aryl alkali metal), and then treating with methanol.

(6) Protection of carboxy or other reactive functions

When Compound (I) is subjected to a chemical reaction to make other Compound (I), functional groups other than objective group are sometimes protected by conventional methods in the art as given in various literatures.

Protection and deprotection of said functional groups are described in, e.g., J. F. W. McOmie ed., "Protective Groups in Organic Chemistry", p. 183 (1973) Pleum Press, N>Y>, S. Patai ed., "The Chemistry of Functional Groups", p. 505 (1969), Interscience Publ., John Wiley & Sons Ltd., London; Flynn ed., "Cephalosporins and Penicillins", Academic Press, N.Y. (1972), and the like books and various patent literatures.

For example, hydroxy is protected by e.g., acylation, etherification; amino is protected by e.g., acylation, enamine formation, silyl introduction; carboxy is protected by e.g., esterification, amidation, acid anhydride formation; each in a conventional manner. Introduction of physiologically active ester to improve pharmacological character is also included in this reaction, e.g., by treating Carboxylic acid (I) with a base to form a salt and then with halide to give the objective ester (I).

(7) Reaction Conditions

The said reactions (1) to (6) can usually be carried out conventionally in an inert solvent at −30°C to 100°C, preferably at −20°C to 50°C for 10 minutes to 5 hours in a solvent, if required in a dry condition. The reaction solvent can be a hydrocarbon, halohydrocarbon, ether, ketone, ester, nitrohydrocarbon, nitrile, amide, sulfoxide, carboxylic acid, organic base, alcohol or water industrial solvent or the mixture.

Similarly, Compound (I) having Z as halogen or acyloxy or its derivatives at carboxy can also be prepared analogously by the methods as shown above.

(8) Work up

The products can be obtained from the reaction mixture by removing contaminants (e.g., solvents, unreacted starting materials, by-products) by e.g., extracting, evaporating, washing, concentrating, precipitating, filtrating or drying and isolating the product by usual work up (e.g., adsorbing, eluting, distilling, precipitating, separating, chromatographing).

(9) Examples

Following examples illustrate the embodiments of this invention.

Physiochemical constants of the products are summarized in Tables at the end of Examples. In the Tables, IR shows $cm^{-1}$ values, NMR shows δ-values in ppm scale and J values show coupling constants in Hz scale. In the Examples, "part" shows part by weight and "equivalent" shows molar equivalent of the beta-lactam starting material.

Work-up procedure is usually as follows:

The reaction mixture is, if required after addition of solvent, e.g., water, dichloromethane, washed with water, dried, and concentrated. Residue is crystallized, precipitated, of filtrated to obtain the product is required after chromatography. Physical constants of the product is identified with those of authentic samples.

Abbreviations used are as follows:

AOM is acetoxymethyl, ECE is for 1-(ethoxycarbonyloxy)ethyl, Ph is for phenyl, and POM is for pivaloyloxymethyl.

In the Tables, infrared absorption spectra (IR) and nuclear magnetic resonance spectra (NMR) are taken in chloroform or deuterio chloroform, unless otherwise specified.

Example 1 (Acylation)

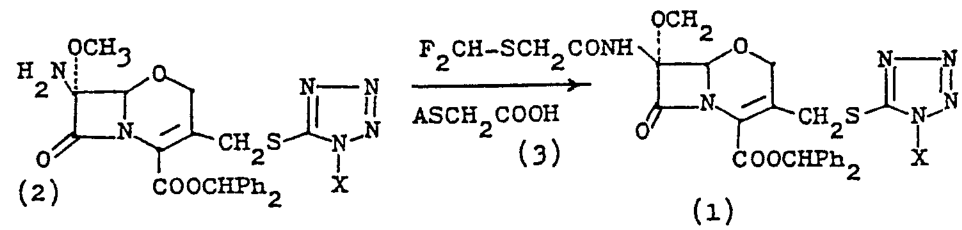


(2) (1)

To a solution of 7β - amino - 7α - methoxy - 3 - (1 - X - substituted - 5 - tetrazolyl)thiomethyl - 1 - dethia - 1 - oxa - 3 - cephem - 4 - carboxylic acid diphenylmethyl ester (2 millimoles) in dichloromethane (1 to 3 ml) cooled at −40°C are added difluoromethylthioacetic acid chloride (1 to 2 equivalents) and pyridine (1 to 2 equivalents), and the mixture is stirred for 1 to 3 hours. The reaction mixture is diluted with ethyl acetate, washed with aqueous 5% sodium hydrogen carbonate and water, dried and concentrated under reduced pressure. The residue is chromatographed over silica gel and the fractions eluted with ethyl acetate are concentrated in vacuum to give the objective 7β - difluoromethylthioacetamido - 7α - methoxy - 3 - (1 - X - substituted - 5 - tetrazolylthiomethyl) - 1 - dethia - 1 - oxa - 3 - cephem - 4 - carboxylic acid diphenylmethyl ester.

By employing this reaction, the compounds of Table 1 are produced.

Example 2 (Amidation)

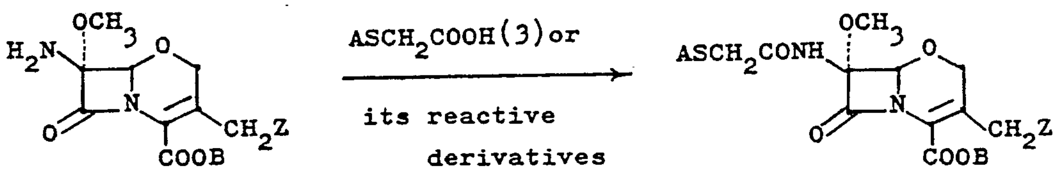


(2) (1)

7β-Amino compound (2) (1 equivalent) is treated with Carboxylic acid corresponding to the 7α-side chain (3) or its reactive derivative to give Amide (1), for example, by a method as examplified below:

1) In a mixture of dichloromethane (10 volumes), 2-ethoxy-1-ethoxycarbonyl-1,2-dihydroquinoline (1.1. equivalent), N,N'-dicyclohexylcarbodiimide (1.1. equivalent), pyridine (1.5 equivalents), and Carboxylic acid (3) (1.1 equivalent), stirred for 1 to 6 hours at 0°C to room temperature.

2) In a mixture of ethyl acetate (10 volumes), di-2-pyridyl disulfide (1.1 equivalent), triphenylphospine (1.1 equivalent), and Carboxylic acid (3) (1.1 equivalent), stirred for 2 to 6 hours at 10 to 50°C.

3) In a mixture of dichloromethane (3 volumes), 1,3,5-tripyridiniumtriazine trichloride (4 equivalents), and Carboxlic acid (3) (1.1 equivalent), stirred for 1 to 5 hours at −10 to 10°C.

4) In a mixture of carbon tetrachloride (30 volumes), 4-methlmorpholine (1.5 equivalents), trisdiethylaminophosphine (1.1 equivalent), and Carboxylic acid (3) (1.1 equivalent), kept for 1 to 5 hours at −20 to 10°C.

5) In a mixture of chloroform (10 volumes) and dimethoxyethane (10 volumes), pyridine (1.5 moles), and mixed anydride of Carboxylic acid (3) and isobutoxyformic acid, stirred at a temperature between −5 to 10°C over a 30 minutes and 6 hours time.

6) In a mixture of ethyl acetate (10 volumes) 1,2-dichloroethane (10 volumes), 4-methylmorpholine (1.5 equivalents), and the symmetric anhydride of Carboxylic acid (3) (1.1 equivalent), refluxed for 10 minutes to 2 hours.

7) In a mixture of dichloromethane (10 volumes), pyridine (1.5 equivalents), and mixed anydride of Carboxylic acid (3) and methanesulfonic acid (1.1 equivalent), kept at 0°C to room temperature over 1 to 3 hours.

8) In a mixture of ethyl acetate (10 volumes), pyridine (1.5 equivalents) and a mixed anydride of diethyl hydrogen phosphate and Carboxylic acid (3) (1.5 equivalents), stirred at 0°C to 10°C for 1 to 5 hours.

9) In a mixture of ethyl acetate (7 volumes), dichloromethane (10 volumes), pyridine (1 equivalent), and mixed anhydride of Carboxylic acid (3) and dichlorophosphoric acid (1.1 equivalent), stirred for 1 to 3 hours at 0°C to room temperature.

10) In a mixture of lutidine (1.5 equivalents), dichloromethane (10 volumes), and the mixed anhydride (1.1 to 2 equivalents) of Carboxylic acid (3) and monochlorophosphoric acid dimethylamide, stirred for 1 to 4 hours at 0 to 30°C.

11) In a mixture of dichloromethane (5 volumes), trifluroacetic anhydride (1.5 equivalents), pyridine (3 equivalents), and Carboxylic acid (3) (1.5 equivalents), stirred for 1 to 5 hours at 0°C to room temperature.

12) In a mixture of dichloromethane (10 volumes), bromide of diethyl hydrogen phosphate (1.2

equivalents), 4-methylmorpholine (2.5 equivalents), and Carboxylic acid (3) (1.2 equivalents), stirred for 1 to 3 hours at 0°C to room temperature.

13) Amine (2) having carboxy at position 4 is dissolved in aqueous (10 volumes) sodium hydrogen carbonate (2.5 equivalents). Carboxylic acid (3) chloride (1.1 equivalent) is dropwise added thereof. The mixture is kept at −5°C to room temperature for 30 minutes to 2 hours.

14) Amine (2) having carboxy at position 4 is treated with trimethylsilyl chloride and triethylamine (1.2 equivalents each) to O-silylate, and then treated with pyridine (4 equivalents) and Carboxylic acid (3) chloride (1.1 equivalents) at −30°C for between 30 minutes and 2 hours, and then the obtained silyl ester is hydrolyzed with acid.

15) In a solution of picoline (4 equivalents) and Carboxylic acid (3) chloride (1.2 equivalents) in dichloromethnane (20 volumes) at 0°C to −30°C over 30 minutes and 2 hours.

16) In a mixture of dimethylformamide (2 volumes) and ethyl acetate (10 volumes), stirred with triethylamine (1.1 equivalent) and Carboxylic acid (3) chloride (1.1 equivalent) at 0°C to −20°C for between 30 minutes and 3 hours.

17) In a mixture of dichloromethane (30 volumes), cyanuric chloride (1.1 equivalent), pyridine (4 equivalents), and Carboxylic acid (3) (1.1 equivalent), stirred for 30 minutes to 2 hours at −30°C to 10°C.

18) In a mixture of dichloromethane (3 volumes), phosphorus oxychloride (1.1 equivalent), pyridine (1.5 equivalents), and Carboxylic acid (3) (1.1 equivalent), stirred for 20 minutes to 2 hours at −10°C to 10°C.

19) Amine (2) is treated with trimethylsilyl chloride to obtain the correspoinding N-trimethylsilyl compound, and this is treated with phosphorus oxychloride (1.5 equivalents), Carboxylic acid (3) (1.2 equivalents), and pyridine (4 equivalents) in dichloromethane (5 parts) for 30 minutes to 2 hours at 0°C to room temperature.

20) In a mixture of dichloromethane (8 volumes), thionyl chloride (1.5 equivalents), pyridine (2.5 equivalents), and Carboxylic acid (3) (1.1 equivalents), stirred for 1 to 5 hours at −30 to 0°C.

21) In a mixture of chloroform (3 volumes), toluene (1 volume), picoline (2 equivalents), oxalyl chloride (1 equivalent), and Carboxylic acid (3) (1.1 equivalent), stirred for 10 minutes to 2 hours at −50°C to 10°C.

22) In a mixture of dichloromethane (20 volumes), pyridine (3 equivalents), and 1-oxabenzotriazolyl ester of Carboxylic acid (3) (3 equivalents), stirred for 5 to 30 hours at 10 to 50°C.

23) In a mixture of dichloromethane (20 volumes), 1-hydroxybenzotriazole (2.1 equivalent), and Carboxylic acid (3) (2 equivalents), stirred at room temperature for 1 to 15 hours.

24) In a mixture of dioxane (10 volumes), N,N'-dicyclohexylcarbodiimide (2 equivalents), and phthalimide of Carboxylic acid (3) (2 equivalents), stirred for 2 to 8 hours at 10 to 50°C.

25) In a mixture of methyl isobutyl ketone (10 volumes), N,N'-dicylcohexylcarbodiimide (1.5 equivalents), and succinimide of Carboxylic acid (3) (1.5 equivalents), stirred for 2 to 9 hours at 0 to 40°C.

26) In a mixture of carbonyldiimidazole (1.1 equivalent), tetrahydrofuran (10 volumes), dimethylacetamide (5 volumes), and Carboxylic acid (3) (1.1 equivalent), stirred for 1 to 5 hours at 0°C to room temperature.

27) In a mixture of dimethylformamide (5 volumes), diemthylaniline (1.3 equivalents) and the Vilsmeyer reagent made from Carboxylic acid (3) and dimethylformamide (1.1 equivalent), stirred at room temperature for 1 to 5 hours.

28) In a mixture of dichloromethane (10 volumes), dimethylformamide (5 volumes), N,N-dicyclohexylcarbodiimide (1.1. equivalent), picoline (1.2 equivalents), and Carboxylic acid (3) (1.1. equivalent), heated for 2 hours to 24 hours.

The product is isolated, if required after diluting with a solvent, (dichloromethane, etc), adjusting pH, washing with water, drying, and concentrating, then purifying by crystallization, adsorption, etc, if required after chromatography over silica gel. Physiochemical constants of Amides are identified by comparison with the product of other route.

Physical constants of amides are given on Table 4-1.

Example 3 (Heterothio introduction)

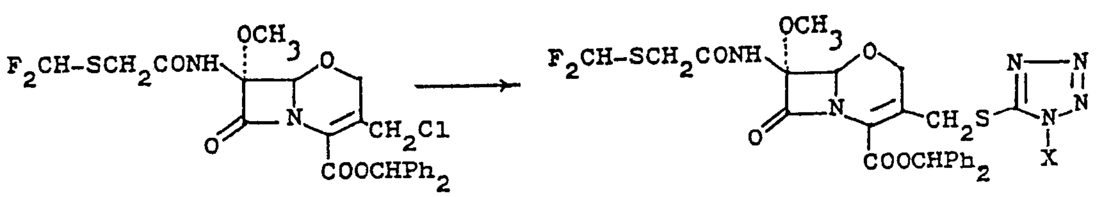

1) To a solution of 7β - difluoromethylthioacetamido - 7α - methoxy - 3 - chloromethyl - 1 - dethia - 1 - oxo - 3 - cephem - 4 - carboxylic acid diphenylmethyl ester (2 milli moles) in N,N-dimethylformamide (5 ml) is added a solution of 1-(X-substituted)-5-mercaptotetrazole sodium salt (1 to 3 equivalents), N,N-dimethylformamide (1 to 3 ml). The mixture is cooled to 0°C and stirred for 20 minutes to 2 hours. The reaction mixture is poured into water and extracted with ethyl acetate. The extract is washed with water, dried, and concentrated. The residue is chromatographed over silica gel. The fractions eluting with ethyl acetate is concentrated in vacuum to give the objective 7β - difluoromethylthioacetamido - 7α -

methoxy - 3 - (1 - X - substituted - 5 - tetrazolyl)thiomethyl - 1 - dethia - 1 - oxa - 3 - cephem - 4 - carboxylic acid diphenylmethyl ester.

By employing this method, Compounds of Table 2 are prepared.

2) To a solution of 7β - difluoromethylthioacetamido - 7α - methoxy - 3 - chloromethyl - 1 - dethia - 1 - oxa - 3 - cephem - 4 - carboxylic acid diphenylmethyl ester (1 part) in N,N-dimethylformamide (3 to 20 parts) is added 2-X-substituted-1,2,3,4-thiadiazole-5-thiol sodium salt (1 to 2 equivalent). After stirring at a temperature between −5°C and 10°C for 10 minutes to 3 hours, the mixture is poured into water and extracted with ethyl acetate. The extract is washed with water, dried, and concentrated. The residue is recrystallized from a mixture of benzene and ethyl acetate to give the corresponding 7β - difluoromethylthioacetamido - 7α - methoxy - 3 - (2 - X - substituted - 1,3,4 - thiadiazol - 5 - yl)thiomethyl - 1 - dethia - 1 - oxa - 3 - cephem - 4 - carboxylic acid diphenylmethyl ester (60—98%).

By employing this method, Compounds of Table 3 are produced.

The starting material for above ie, 7β - difluoromethylthioacetamido - 7α - methoxy - 3 - chloromethyl - 1 - dethia - 1 - oxa - 3 - cephem - 4 - carboxylic acid diphenylmethyl ester is produced as follows.

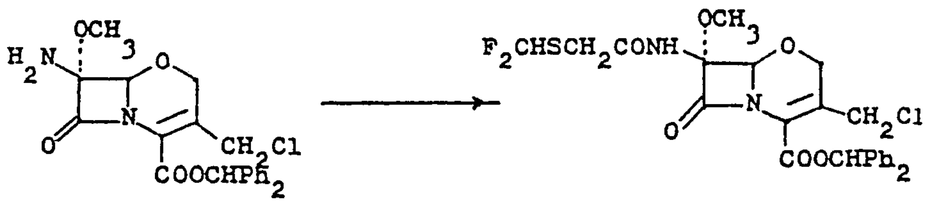

To a solution of 7β - amino - 7α - methoxy - 3 - chloromethyl - 1 - dethia - 1 - oxa - 3 - cephem - 4 - carboxylic acid diphenylmethyl ester in dichloromethane (1 L) cooled at −20°C are added pyridine (4.4 equivalents) and difluoromethylthioacetyl chloride (1.1 equivalent) under nitrogen. After stirring for 30 minutes, the mixture is poured into cold 1% hydrochloric acid and resulting organic layer is taken. This is washed with aqueous 4% sodium hydrogen carbonate, 1% hydrochloric acid, and water, dried, and concentrated in vacuum. The separating crystals are collected by filtration, washed with a mixture of benzene and ether, and dried to give 7β - difluoromethylthioacetamido - 7α - methoxy - 3 - chloromethyl - 1 - dethia - 1 - oxa - 3 - cephem - 4 - carboxylic acid diphenylmethyl ester. (39.8 g).

mp. 189—190.5°C.

IR ($CHCl_3$): 3390, 1792, 1732, 1705 $cm^{-1}$.

Example 4

Under the condition similar to that of Example 3, the corresponding 7β - difluoromethylthioacetamido - 7α - methoxy - 3 - chloromethyl - 1 - dethia - 1 - oxa - 3 - cephem - 4 - carboxylic acid diphenylmethyl ester is treated with a sodium salt of heterocyclic thiol to give Compounds of Table 4 (part 2).

Example 5 (Deesterification)

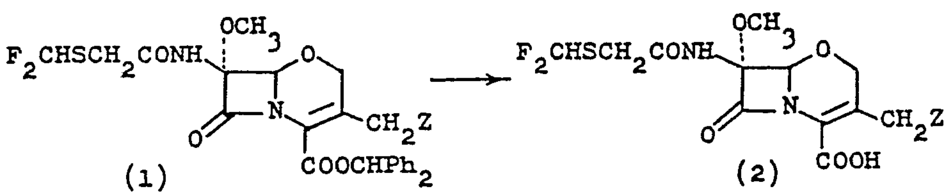

1) A solution of the p-methoxybenzyl ester or diphenylmethyl ester (1) (1 part) in a mixture of dichloromethane (0.3 to 3 parts), trifluoroacetic acid (0.3 to 3 parts) and anisole (0.5 to 5 parts) is stirred at a temperature between −10 and 40°C over 10 minutes to 3 hours period. The reaction mixture is concentrated in vacuum to remove the solvent and reagents. The residue is washed with benzene to give the corresponding free acid (2). Yield: 70 to 90%.

2) To a solution of above starting material (1) (1 part) is a mixture of dichloromethane (5 to 9 parts) and anisole (2 to 8 parts) is added aluminium chloride or titanium tetrachloride (2 to 4 equivalents). The mixture is stirred for 1 to 3 hours at −10 and 10°C. The reaction mixture is washed with diluted hydrochloric acid and water, dried, and concentrated to give the corresponding free acid (2). Yield: 80 to 95%.

The products of above methods are listed on Tables 5 to 7.

Example 6 (Pharmacologically acceptable esters)

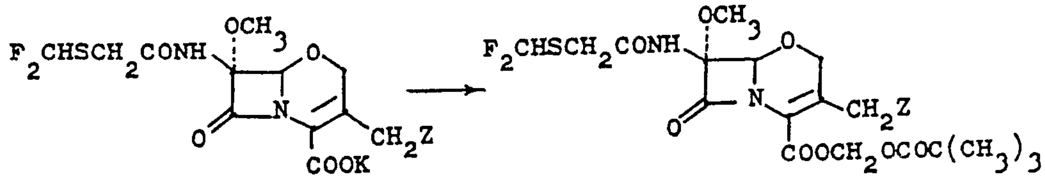

(1) (2)

1) To a solution of Carboxylic acid (1) potassium salt (1 millimole) in N,N-dimethylformamide (2 to 5 parts) is added iodomethyl pivalate (1 to 2 equivalents) under ice cooling. After 15 minutes to 2 hours' stirring, the mixture is diluted with ethyl acetate, washed with ice water and aqueous sodium hydrogen carbonate, dried, and concentrated in vacuum. The residue is recrystallized from ethyl acetate to give the pivaloyloxymethyl ester (2).

2) The potassium salt of above section 1) is replaced by sodium salt to give the same products under same conditions.

3) Pivaloyloxymethyl ester (2) of above section 1) (250 mg), corn starch (150 mg), and magnesium stearate (5 mg) are mixed, granulated, and encapsulated in a conventional manner.

This capsule (1 to 3 capsules) is given orally to treat a patient suffering from infection caused by sensitive Escherichia coli JC-2.

4) In place of iodomethyl pivalate of above 1), iodomethyl acetate or iodoethyl ethoxyformate is used under the same condition to give the corresponding acetoxymethyl ester (2) or ethoxycarbonyloxyethyl ester (2).

Physical constants of the products are listed in Table 8, 10, and 11.

Example 7 (Sodium salt, preparation, use)

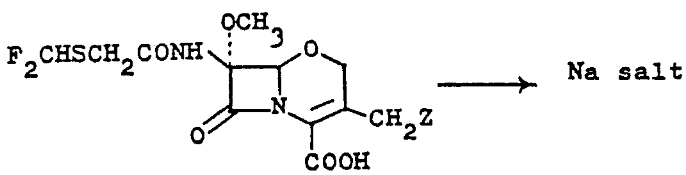


(1) (2)

A solution of Carboxylic acid (1) (1 g) in aqueous 0.5% sodium hydrogen carbonate (5 ml) adjusted to pH 7 with hydrochloric acid is washed with ethyl acetate, desalted, and poured into 10 ml vials. This is lyophilized conventionally to give the corresponding sodium salt (2) as powder.

The sodium salt (1 g) produced under sterile condition is dissolved in sterile water (4 g) is injected twice a day intravenously to a patient suffering from Staphylococcus aureus infection for treating said disease. The sodium salt (2) is assayed for MIC by the standard method of Japan Society of Chemotherapy to give values less than 0.1 g/ml against Streptococcus pyogenes C-203 and less than 0.5 g/ml Escherichia coli JC-2.

Sodium or potassium salt produced by above mentioned are listed on Table 12.

Example 8 (Methoxylation)

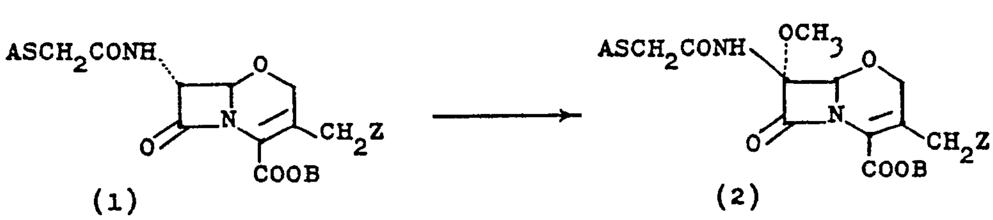

(1) (2)

To a solution of 7α-amido-substituted methyl-1-dethia-1-oxa-3-cephem-4-carboxylic acid derivative (1) (1 part) in dichloromethane (10 parts) is added tert-butyl hypochlorite (1.1. equivalent). After standing for 3 hours at −20°C, a solution of lithium methoxide (1.2 equivalents) in methanol is added to the mixture and let react for 30 minutes. The reaction mixture is acidified with acetic acid and diluted with dichloromethane. This is washed with water, dried, and concentrated in vacuum to give the corresponding 7β-amido-7α-methoxy-3-substituted methyl-1-dethia-1-oxa-3-cephem-4-carboxylic acid derivative (2) in 40 to 85% yield.

Products (I) made by this method are listed on Table 9.

Example 9

Compounds (I) produced by the methods of Examples 1—8 or its combination are listed on tables 13 and 14.

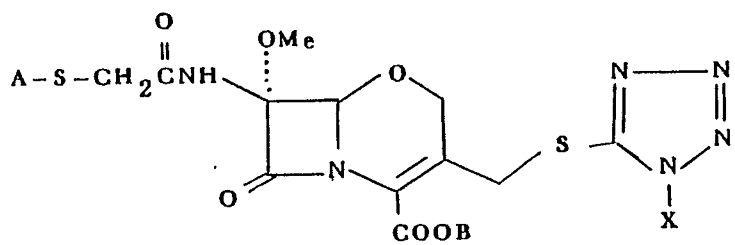

Table 1-1

| No. | A | B | X | IR (νmax $cm^{-1}$) | NMR (δ) |
|---|---|---|---|---|---|
| 1-1 | $CF_2H$ | $CHPh_2$ | $CH_2CH_2N(CH_3)_2$ | (KBr)3260, 1785, 1725, 1700, 1618 mp: 124.0~125.5℃ | 2.18 (s, 6H), 2.66 (t, 6Hz, 2H), 3.53 (s, 3H), 3.53 (s, 2H), 4.17 (t, 6Hz, 2H), 4.21 (s, 2H), 4.59 (s, 2H), 5.03 (s, 1H), 6.92 (s, 1H), 6.92 (t, 56Hz, 1H) |
| 1-2 | $CF_2H$ | $CHPh_2$ | H | 1790, mp: 166-71℃ | 3.55 (s, 5H), 3.80 (s, 3H), 4.25 (s, 2H), 4.63 (s, 2H), 5.07 (s, 1H), 3.58 (s, 1H), 3.58 (t, 56Hz, 1H), 7.23-7.67 (m, 10H) |
| 1-3 | $CF_2H$ | $CHPh_2$ | $CH_2CN$ | mp: 169-171℃ | 4.58 (s, 2H), 5.00 (s, 2H), 5.05 (s, 1H), 6.85 (s, 1H), 6.87 (t, 1H, 56Hz), 7.2-7.6 (m, 10H) |
| 1-4 | $CF_2H$ | $CHPh_2$ | $CH_2CH_2CN$ | 3375, 2245, 1785, 1715, 1706, 1392, 1258, 1072 | 2.87 (t, 2H, 6.5Hz), 3.55 (s, 5H), 4.23 (s, 2H), 4.33 (t, 2H, 6.5Hz), 4.60 (s, 2H), 5.08 (s, 1H), 6.87 (s, 1H), 6.91 (t, 1H, 56Hz), 7.25-7.70 (m, 10H) |
| 1-5 | $CF_2H$ | $CHPh_2$ | $CH_2CO_2CH_3$ | | 3.58 (s, 5H), 3.78 (s, 3H), 4.25 (s, 2H), 4.62 (s, 2H), 4.97 (s, 2H), 5.08 (s, 1H), 6.93 (t, 1H, 56Hz), 6.93 (s, 1H), 7.2-7.6 (m, 10H) |

Table 1-2

| № | A | B | X | IR($\nu$max $cm^{-1}$) | NMR($\delta$) |
|---|---|---|---|---|---|
| 1-6 | $CF_2H$ | $CHPh_2$ | $CH_2CO_2C_2H_5$ | 3280,1786,1757, 1720,1708,1395, 1260,1077 | 1.23(t,3H,5Hz),3.55(s,5H),4.18(q,2H,5Hz), 4.22(s,2H),4.58(s,2H),4.92(s,2H), 5.05(s,1H),6.88(s,1H),6.90(t,1H,56Hz), 7.20-7.55(m,10H) |
| 1-7 | $CF_2H$ | $CHPh_2$ | $CH_2CONH_2$ | 3360,2950,1785, 1720,1695,1390, 1256,1075 | 3.55(s,5H),4.20(bs,2H),4.53(bs,2H), 4.88(bs,2H),5.08(s,1H),6.91(s,1H), 6.91(t,1H,56Hz),7.3-7.7(m,10H) |
| 1-8 | $CF_2H$ | $CHPh_2$ | $CH_2CONHCH_3$ | 3375,1785,1760, 1705,1395,1070 | 2.70(d,3H,5Hz),3.53(s,5H),4.17(bs,2H), 4.57(bs,2H),4.85(bs,2H),5.07(s,1H), 6.70(d,1H,5Hz),6.87(s,1H),6.90(t,1H,56Hz), 7.2-7.6(m) |
| 1-9 | $CF_2H$ | $CHPh_2$ | $CH_2CON(CH_3)_2$ | 3375,1785,1700, 1675,1395,1070 | 2.90(s,3H),3.00(s,3H),3.55(s,5H), 4.20(s,2H),4.57(bs,2H),5.02(s,2H), 5.07(s,1H),6.88(s,1H),6.92(t,1H,56Hz), 7.2-7.6(m,10H) |
| 1-10 | $CF_2H$ | $CHPh_2$ | $CH_2COCH_3$ | 3375,1785,1730, 1717,1706,1255, 1070 | 2.17(s,3H),3.53(s,5H),4.16(s,2H), 4.53(s,2H),4.97(s,2H),5.0(s,1H),6.85(s,1H), 6.87(t,1H,56Hz),7.20-7.27(m,10H) |

Table 1-3

| No. | A | B | X | IR($\nu$max $cm^{-1}$) | NMR ($\delta$) |
|---|---|---|---|---|---|
| 1-12 | $CF_2H$ | $CHPh_2$ | $CH_2CH_2CO_2CHPh_2$ | 3375,1786,1730,<br>1700,1390,1255,<br>1072 | 3.0 (t, 2H, 7.0Hz), 3.50 (s, 5H), 4.15 (s, 2H),<br>4.32 (t, 2H, 7.0Hz), 4.53 (s, 2H), 5.02 (s, 1H),<br>6.83 (s, 1H), 6.88 (s, 1H), 6.88 (t, 1H, 56Hz),<br>7.22-7.58 (m, 20H) |
| 1-13 | $CF_2H$ | $CHPh_2$ | $CH_2CO_2CHPh_2$ | 3380,1786,1754,<br>1712,1700sh,<br>1392,1257,1076 | 3.60 (s, 5H), 4.16 (s, 2H), 4.53 (s, 2H),<br>5.03 (s, 3H), 6.90 (s, 2H), 6.90 (t, 1H, 56Hz),<br>7.2-7.6 (m, 20H) |
| 1-14 | $CF_2H$ | $CHPh_2$ | $CH_2CH_2CO_2CH_3$ | 3375,1790,1735,<br>1720,1700,1390,<br>1070 | 2.93 (t, 2H, 7Hz), 3.57 (s, 3H), 3.60 (s, 2H),<br>3.68 (s, 3H), 4.27 (s, 2H), 4.38 (t, 2H, 7Hz),<br>4.63 (s, 2H), 5.10 (s, 1H), 6.92 (s, 1H),<br>6.93 (t, 1H, 56Hz), 7.2-7.6 (m, 10H) |
| 1-15 | $CF_2H$ | $CHPh_2$ | $CH_2CH_2CO_2C_2H_5$ | 3370,1790,1725,<br>1715,1700,1390,<br>1070 | 1.22 (t, 3H, 7Hz), 2.92 (t, 2H, 7Hz), 3.57 (s, 3H),<br>3.58 (s, 2H), 4.13 (q, 2H, 7Hz), 4.27 (s, 2H),<br>4.37 (t, 2H, 7Hz), 4.63 (s, 2H), 5.08 (s, 1H),<br>6.90 (s, 1H), 6.90 (t, 1H, 55Hz), 7.2-7.6 (m, 10H). |
| 1-16 | $CF_2H$ | $CHPh_2$ | $CH_2CH_2CONH_2$ | 3480,3390,1790,<br>1720,1698,1260,<br>1078 | 2.77 (t, 2H, 7.5Hz), 3.50 (s, 5H), 4.17 (s, 2H),<br>4.33 (t, 2H, 7.5Hz), 4.53 (s, 2H), 5.05 (s, 1H),<br>6.00 (bs, 2H), 6.87 (s, 1H), 6.88 (t, 1H, 56Hz),<br>7.18-7.50 (m, 10H), 7.75 (bs, 1H) |

Table 1-4

| № | A | B | X | IR($\nu$max $cm^{-1}$) | NMR ($\delta$) |
|---|---|---|---|---|---|
| 1-17 | $CF_2H$ | $CHPh_2$ | $CH_2CH_2SO_2NH_2$ | 3250.1790.1730.<br>1700.1670 | 3.55 (s, 3H), 3.70 (t, 2H, 7Hz), 3.68 (s, 2H),<br>4.30 (s, 2H), 4.67 (s, 2H), 4.92 (t, 2H, 7Hz),<br>5.13 (s, 1H), 6.88 (s, 1H), 7.20 (t, 1H, 56Hz),<br>7.2-7.6 (m, 10H), |
| 1-18 | $CF_2H$ | $CHPh_2$ | $-CH_2CONH-$ 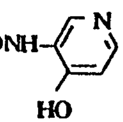 | 1690.1723.1785 | (acetone) 3.50 (s, 3H), 3.73 (s, 2H), 4.10+4.42<br>(ABq, 14Hz, 2H), 4.63 (s, 2H), 5.12 (s, 1H),<br>5.55 (s, 2H), 6.38 (d, 1H, 7Hz),<br>7.20 (t, 1H, 56Hz), 6.87 (s, 1H),<br>7.13~8.12 (m, 11H), 8.85 (s, 1H) |
| 1-19 | $CF_2H$ | $CHPh_2$ | $-CH_2CONH-$ 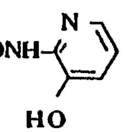 | 1685.1720.1784 | |
| 1-20 | $CF_2H$ | $CHPh_2$ | $-CH_2CONHCONH_2$ | (Nujol) 1705.<br>1780 | ($d_6$-acetone) 3.53 (s, 3H), 3.73 (S, 2H),<br>4.27 (s, 2H), 4.67 (s, 2H), 5.13 (s, 1H),<br>5.38 (s, 2H), 6.90 (s, 1H), 7.20 (t, 1H, 56Hz),<br>7.10~7.83 (10H) |
| 1-21 | $CF_2H$ | $CHPh_2$ | $-CH_2SO_3H$ | | ($CDCl_3-CD_3OD$) 3.53 (s, 5H), 4.23 (S, 2H),<br>4.60 (s, 2H), 5.10 (s, 1H), 5.20 (s, 2H),<br>6.93 (s, 1H), 7.03 (t, 56Hz, 1H), 7.2~7.6 (m, 10H) |

Table 1-5

| № | A | B | X | IR($\nu$max $cm^{-1}$) | NMR($\delta$) |
|---|---|---|---|---|---|
| 1-22 | $CF_2H$ | $CHPh_2$ | $-C(CH_3)_2COOH$ | 1790.1720 | 183(s.3H).1.93(s.3H).3.52(s.5H).<br>4.23(s.2H).4.37(s.2H).4.98(s.1H).<br>6.87(t.1H.55Hz)6.92(s.1H).<br>7.7~7.0(m.11H) |
| 1-23 | $CF_2H$ | $CHPh_2$ | $-CH=CH_2$ | | 3.52(s.5H).4.27(s.2H).4.60(s.2H).<br>5.03(s.1H).5.30(dd.1H.2Hz.9Hz).<br>5.80(dd.1H.2Hz.16Hz).4.10(s.1H).<br>4.10(dd.1H.9Hz.16Hz)<br>4.12(t.1H.54Hz).7.0-7.8(m.10H) |
| 1-24 | $CF_2H$ | $CHPh_2$ | $-CH_2CH_2NHCO-$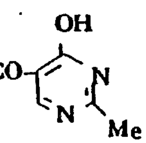 | 1780 | 2.41(s.3H).3.56(s.3H).3.61(s.2H).<br>5.03(s.1H).6.93(t.56.7Hz.1H).<br>6.87(s.1H).8.80(s.1H) |
| 1-25 | $CF_2H$ | $CHPh_2$ | $-CH_2CONHOCH_3$ | 1715.1780 | 3.52(s.5H).3.63(s.3H).4.15(s.2H).<br>4.52(s.2H).4.85(s.2H).5.05(s.1H).<br>6.87(s.1H).6.90(t.1H.56Hz).<br>7~8(m.12H) |
| 1-26 | $CF_2H$ | $CHPh_2$ | $-CH_2NHCONH_2$ | 1690.1780.3370 | 3.50(s.5H).4.33(s.2H).4.50(s.2H).<br>4.83(s.2H).5.02(s.1H).6.86(s.1H).<br>6.86(t.1H.56Hz).7~7.9(m.14H) |

Table 1-6

| No. | A | B | X | IR(νmax $cm^{-1}$) | NMR (δ) |
|---|---|---|---|---|---|
| 1-27 | $CF_2H$ | $CHPh_2$ | 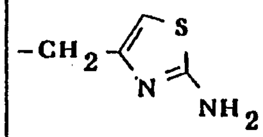 | | 3.53 (s, 5H), 4.22 (s, 2H), 4.54 (s, 2H), 5.03 (s, 1H), 5.12 (s, 2H), 5.60 (s, 2H), 6.30 (s, 1H), 6.90 (s, 1H), 6.90 (t, 1H, 56Hz), 7.1~7.7 (m, 11H) |
| 1-28 | $CF_2H$ | $CHPh_2$ | $-CH_2C(=NOH)-CH_3$ | 1710, 1780, 3250 | 1.81 (s, 3H), 3.50 (s, 5H), 4.22 (s, 2H), 4.53 (s, 2H), 4.82 (s, 2H), 5.03 (s, 1H), 6.89 (s, 1H), 6.89 (t, 1H, 56Hz), 7.05~7.65 (m, 1H), 8.51 (s, 1H) |
| 1-29 | $CF_2H$ | $CHPh_2$ | $CH_2C(=O)NH_2$ | | 3.51 (s, 5H), 4.15 (s, 2H), 4.50 (s, 2H), 4.83 (s, 2H), 5.01 (s, 1H), 6.86 (s, 1H), 6.86 (t, 1H, 56Hz), 7.1~7.8 (m, 13H) |
| 1-30 | $CF_2H$ | $CHPh_2$ | $CH_2CH_2C(=O)NH_2$ | | ($CDCl_3$+$CD_3OD$) 2.81 (t, 2H, 7Hz), 3.55 (s, 5H), 4.23 (s, 2H), 4.38 (t, 2H, 7Hz), 4.56 (s, 2H), 5.09 (s, 1H), 6.89 (s, 1H), 6.96 (t, 1H, 56Hz), 7.2~7.8 (m, 11H) |
| 1-31 | $CF_2H$ | $CHPh_2$ | $CH_2CH_2NHSO_2CH_3$ | 1170, 1321, 1390, 1698, 1780 | 2.83 (s, 3H), 3.55 (s, 7H), 4.15~4.4 (4H), 4.58 (s, 2H), 5.07 (s, 1H), 6.87 (s, 1H), 6.91 (t, 1H, 56Hz), 7.2~7.7 (m, 12H) |
| 1-32 | $CF_2H$ | $CHPh_2$ | 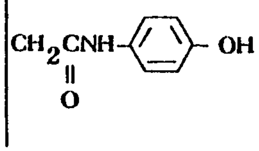 | 1665, 1710, 1768, 3250 | ($d_6$-acetone) 3.53 (s, 3H), 3.68 (s, 2H), 4.26 (q, 2H, 13Hz), 4.63 (s, 2H), 5.13 (s, 1H), 5.21 (s, 2H), 6.55~7.90 (m, 19H) |

Table 1-7

| No. | A | B | X | IR(νmax $cm^{-1}$) | NMR(δ) |
|---|---|---|---|---|---|
| 1-33 | $CF_2H$ | $CHPh_2$ | 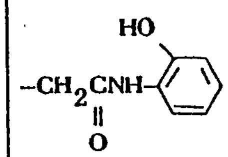 | | ($d_6$-acetone) 3.54 (s, 3H), 3.73 (s, 2H), 4.31 (q, 2H, 13Hz), 4.66 (s, 2H), 5.16 (s, 1H), 5.46 (s, 2H), 6.60~8.10 (m, 19H) |
| 1-34 | $CF_2H$ | $CHPh_2$ | 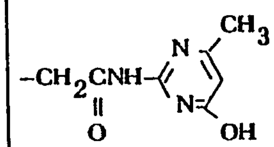 | 1670, 1695, 1788 3300 | 2.13 (s, 3H), 3.50 (s, 5H), 4.15 (s, 2H), 4.48 (s, 2H), 5.03 (s, 1H), 5.18 (s, 2H), 5.88 (s, 1H), 6.82 (s, 1H), 6.90 (t, 1H, 56Hz), 7.0~7.70 (m, 13H) |

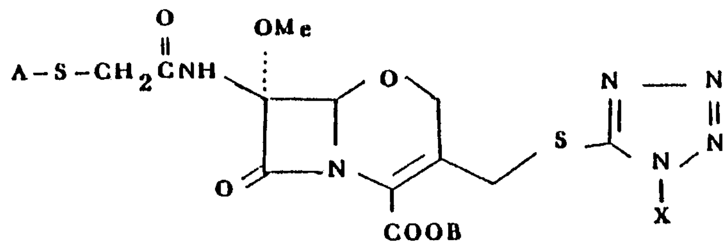

Table 2-1

| No | A | B | X | IR($\nu max$ $cm^{-1}$) | NMR($\delta$) |
|---|---|---|---|---|---|
| 2-1 | $CF_2H$ | $CHPh_2$ | $C_2H_5$ | 3390, 1790, 1709, 1633<br>mp 140.0~142.5°C | 1.43 (t, 7Hz, 3H), 3.25 (s, 5H), 4.13 (q, 7Hz, 2H), 4.25 (s, 2H), 4.62 (s, 2H) 5.03 (s, 1H), 6.92 (s, 1H), 6.92 (t, 56Hz, 1H), 7.2~7.7 (m, 11H) |
| 2-2 | $CF_2H$ | $CHPh_2$ | 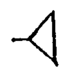 | | 1.0~1.3 (m, 4H), 3.0~3.6 (m, 1H), 3.53 (s, 5H), 4.27 (s, 2H) 4.63 (s, 2H), 5.03 (s, 1H), 6.90 (t, 56Hz, 1H), 6.93 (s, 1H), 7.2~7.7 (m, 11H) |
| 2-4 | $CF_2H$ | $CHPh_2$ | $CH_2CH_2SCH_3$ | 3390, 1790, 1709, 1631 | 1.97 (s, 3H), 2.80 (t, 6Hz, 2H), 3.50 (s, 2H), 3.53 (s, 3H), 4.22 (s, 2H), 4.22 (t, 6Hz, 2H), 4.57 (s, 2H), 5.03 (s, 1H), 6.85 (t, 56Hz, 1H), 6.90 (s, 1H), 7.1~7.7 (m) |

Table 2-2

| No. | A | B | X | IR($\nu$max $cm^{-1}$) | NMR ($\delta$) |
|---|---|---|---|---|---|
| 2-5 | $CF_2H$ | $CHPh_2$ | $CHF_2$ | 3370,1785,1700 | 3.57 (s, 5H), 4.35 (s, 2H), 4.65 (s, 2H), 5.08 (s, 1H), 6.93 (t, 55Hz, 1H), 6.93 (s, 1H), 7.43 (t, 58Hz, 1H), 7.07~7.67 (m, 11H) |
| 2-6 | $CF_2H$ | $CHPh_2$ | $CH_2CH(CH_3)_2$ | 3375,1785,1705, 1690(sh) | 0.93 (d, 7Hz, 6H), 2.20 (q, 7Hz, 1H), 3.58 (s, 5H), 3.97 (d, 7Hz, 2H), 4.47 (s, 2H), 4.67 (s, 2H), 5.08 (s, 1H), 6.93 (s, 56Hz, 2H), 7.2~7.6 (m, 10H) |

Table 3-1

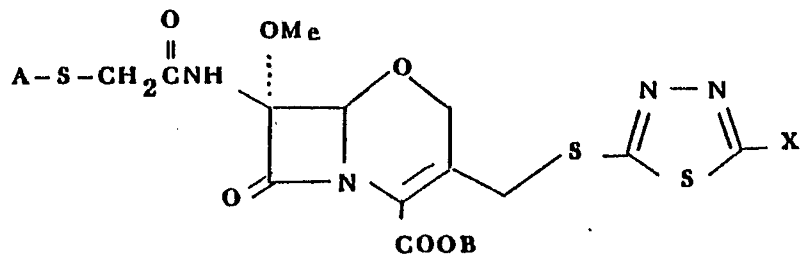

| No. | A | B | X | IR($\nu max$ $cm^{-1}$) | NMR ($\delta$) |
| --- | --- | --- | --- | --- | --- |
| 3-1 | $CF_2H$ | $CHPh_2$ | H | 1785,1720,1700 | ($CD_3OD-CDCl_3$)<br>3.53 (s), 3.57 (s) (5H, OMe, $SCH_2$);<br>4.40 (brs, 2H-$CH_2S$ ); 4.62 (s, 2H )<br>5.08 (s, 1H, $C_6$-H); 6.90 (s, 1H, $C\underline{H}Ph_2$)<br>6.97 (t, 1H J=56Hz $CF_2H$);<br>7.2~7.7 (m, 11H) |
| 3-2 | $CF_2H$ | $CHPh_2$ | $CH_2NHBOC$ | 1785,1715,1705 | 1.47 (s, 9H, tBu), 3.55 (s,5H, OMe, $SCH_2$),<br>4.32 (ABq, 2H J=14Hz),<br>4.63 (broods, 4H, $CH_2$×2),<br>5.10 (s, 1H, $C_6$-H),<br>6.97 (s, 2H, $C\underline{H}Ph_2$, $CF_2H$),<br>7.3~7.6 (m, 10H) |
| 3-3 | $CF_2H$ | $CHPh_2$ | $CH_2OH$ | 1790,1740,1700 | ($CDCl_3-CD_3OD$)<br>3.53 (s, 5H, OMe+$SCH_2$), 4.33(s, 2H, $CH_2$),<br>4.60 (s, 2H, $CH_2$), 4.90 (s, 2H, $CH_2$),<br>5.07 (s, 1H, $C_6$-H), 6.87 (s, 1H)<br>6.97 (t, 1H, J=56Hz), 7.3~7.7 (m, 10H) |

Table 3-2

| No. | A | B | X | IR($\nu max$ $cm^{-1}$) | NMR($\delta$) |
|---|---|---|---|---|---|
| 3-4 | $CF_2H$ | $CHPh_2$ | $CH_2OCH_2CO_2Na$ | 1785,1750,1720<br>1710 | 3.53(s, 5H, OMe+$SCH_2$),<br>4.27(s, 2H, $CH_2$), 4.33(ABq, 2H, $CH_2$),<br>4.60(s, 2H, $CH_2$), 4.93(s, 2H, $CH_2$),<br>5.07(s, 1H, $C_6$-H)<br>6.92, 6.96(s×2, t, 3H), 7.2~7.7(m, 20H) |
| 3-5 | $CF_2H$ | $CHPh_2$ | $CH_3$ | 1785,1720,1700 | ($CDCl_3$-$d_4$MeOH): $\delta$ 2.70(s, 3H),<br>3.57(s, 5H, OMe-$SCH_2$),<br>4.30(brs, 2H), 4.60(brs, 2H),<br>5.07(s, 1H), 6.93(s, 1H), 7.00(t, 1H, J=56Hz)<br>7.2~7.6(m, 10H) |
| 3-6 | $CF_2H$ | $CHPh_2$ | $CH_2CN$ | 1780,1720,1700 | ($d_6$-DMSO)<br>3.43(s, 3H), 3.67(s, 2H), 4.33(ABq, 2H),<br>4.60(brs, 4H, $CH_2$×2), 5.22(s, 1H),<br>6.87(s, 1H), 7.30(t, J=56Hz, 1H)<br>7.2~7.8(m, 10H) |
| 3-7 | $CF_2H$ | $CHPh_2$ | $CH_2CO_2Na$ | (Nujol) 1780,<br>1740,1710,1670 | ($d_6$-acetone)<br>3.57(s, 3H), 3.73(s, 5H, OMe, $sCH_2$)<br>4.22(s, 2H), 4.43(ABq, 2H), 4.68(s, 2H),<br>5.13(s, 1H), 6.93(s, 1H),<br>7.23(t, J=56Hz, 1H), 7.2~7.8(m, 10H) |

Table 3-3

| No | A | B | X | IR($\nu max$ $cm^{-1}$) | NMR($\delta$) |
|---|---|---|---|---|---|
| 3-8 | $CF_2H$ | $CHPh_2$ | $NH_2$ | 1780,1740 | ($d_6$-acetone-$d_6$·DMSO)<br>3.53(s,3H), 3.72(s,2H), 4.17(ABq,J=14Hz,2H),<br>4.63(s,2H), 5.30(s,1H), 6.88(s,1H),<br>7.28(t,J=56Hz,1H), 7.2~7.8(m,10H) |
| 3-9 | $CF_2H$ | $CHPh_2$ | $SCH_3$ | 1780,1720,1695 | ($CDCl_3$-$d_4MeOH$)<br>2.70(s,3H), 3.53(s,5H,OMe+$SCH_2$),<br>4.20(ABq,J=14Hz,2H), 4.53(brS,2H),<br>5.03(s,1H), 6.83(s,1H), 6.93(t,J=56Hz,1H),<br>7.3~7.7(m,10H) |
| 3-10 | $CF_2H$ | $CHPh_2$ | $COOC_2H_5$ | 1785,1740(sh)<br>1715,1700(sh) | 1.47(t,J=7Hz,3H), 3.57(s,5H,OMe,$SCH_2$),<br>4.43(ABq,J=14Hz,2H),<br>450(q,J=7Hz,2H), 4.63(brs,2H),<br>5.07(s,1H), 6.93(s+t,J=56Hz,2H),<br>7.2-7.7(m,10Hz) |

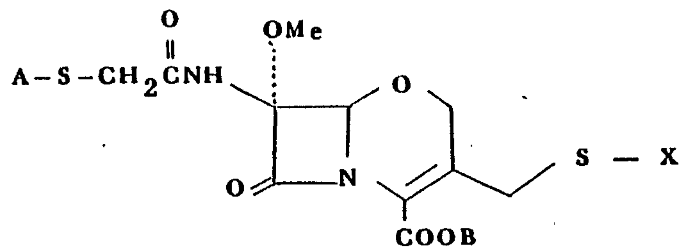

Table 4-1-1

| No. | A | B | X | IR($\nu$max $cm^{-1}$) | NMR($\delta$) |
|---|---|---|---|---|---|
| 4-1-1 | $CF_2H$ | $CHPh_2$ | 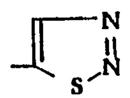 | 1795 | 3.53(s,3H), 4.05(s,2H), 4.42(s,2H), 5.03(s,1H), 6.83(s,1H), 6.87(t,55Hz,1H), 8.33(s,1H) |
| 4-1-2 | $CF_2H$ | $CHPh_2$ | 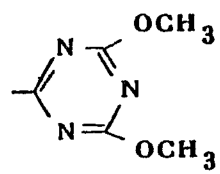 | 3390,1790,1725, 1706,1635,1540 | 3.57(s,5H), 3.97(s,6H), 4.25(ABq,2H), 4.58(s,2H), 5.10(s,1H), 6.93(t,56Hz,1H), 6.97(s,1H), 7.2~7.7(m,11H) |
| 4-1-3 | $CF_2H$ | $CHPh_2$ | 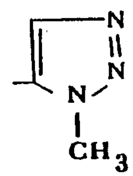 | 3370,1785,1720, 1700 | 3.57(s,5H), 3.77(bs,2H), 3.96(s,3H), 4.47(s,2H), 5.10(s,1H), 6.70(s,1H), 6.97(t,56Hz,1H), 7.17~7.67(m,11H), 7.62(s,1H) |
| 4-1-4 | $CF_2H$ | $CHPh_2$ | 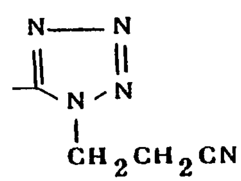 | 3370,2230,1783, 1717,1700 | 2.88(t,7Hz,2H), 3.55(s,3H), 3.40~4.67(m,8H), 5.10(s,1H), 6.67(s,1H), 6.98(t,56Hz,1H), 7.17~7.57(m,10H), 7.62(s,1H), 7.73(bs,1H) |

Table 4-1-2

| No. | A | B | X | IR(νmax cm⁻¹) | NMR (δ) |
|---|---|---|---|---|---|
| 4-1-5 | $CF_2H$ | $CHPh_2$ | 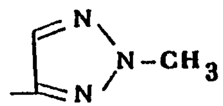 | 3380, 1782, 1720, 1700 | 3.52 (s, 3H), 3.65+4.02 (ABq, 14Hz, 2H), 3.88 (s, 3H), 4.47 (s, 2H), 5.02 (s, 1H), 6.68 (s, 1H), 6.83 (t, 55Hz, 1H), 7.13~7.47 (m, 11H) |
| 4-1-6 | $CF_2H$ | $CHPh_2$ | 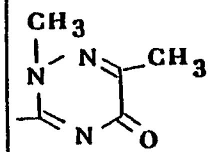 | 3375, 1790, 1710, 1650 | 2.27 (s, 3H), [3.55 (s, 3H)+3.60 (s, 3H)+2H], 4.08, 4.38 (q, 13Hz, 2H), 4.58 (bs, 2H) 5.07 (s, 1H), 6.92 (s, 1H), 6.95 (t, 56Hz, 1H), 7.07~7.67 (m, 10H) |
| 4-1-7 | $CF_2H$ | $CHPh_2$ | 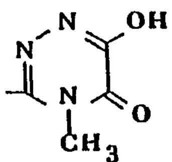 | 3375, 1785, 1705, 1585 | 3.27 (s, 3H), 3.55 (bs, 5H), 4.00 (bs, 2H), 4.52 (bs, 2H), 5.10 (s, 1H), 6.92 (s, 1H), 6.95 (t, 56Hz, 1H), 7.05~7.67 (m, 10H) |
| 4-1-8 | $CF_2H$ | $CHPh_2$ | 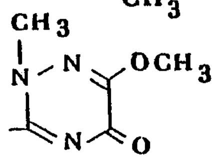 | 3375, 1785, 1705, 1665, 1580 | [3.58 (s, 3H)+3.62 (s, 3H)+(bs, 2H)], 3.88 (s, 3H), 4.20, 4.33 (q, 16Hz, 2H), 4.62 (bs, 2H), 5.07 (s, 1H), 6.97 (s, 1H), 7.02 (t, 56Hz, t), 7.08~7.71 (m, 10H) |
| 4-1-9 | $CF_2H$ | $CHPh_2$ | 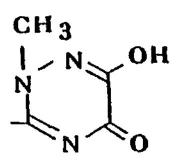 | 3475, 3400, 1790, 1745, 1720, 1650, 1600 | [3.58 (s, 3H)+3.62 (s, 3H)+2H], 5.95 (bs, 2H), 4.63 (s, 2H), 5.10 (s, 1H), 6.97 (s, 1H+t, 1H, 56Hz), 7.12~7.62 (m, 10H) |

Table 4-1-3

| No. | A | B | X | IR(νmax cm$^{-1}$) | NMR(δ) |
|---|---|---|---|---|---|
| 4-1-10 | $CF_2H$ | $CHPh_2$ | 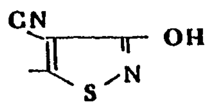 | 3375,2220,1790, 1725,1700 | [3.57(s,3H)+2H], 4.10(bs,2H), 4.50(bs,2H), 5.13(s,1H), 6.87(s,1H), 6.92(t,56Hz,1H), 7.05~7.73(m,10H) |
| 4-1-11 | $CF_2H$ | $CHPh_2$ | 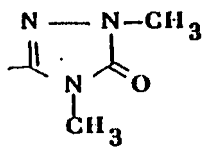 | 3380,1790,1700 | 3.12(s,3H), 3.23(s,3H), 3.53(s,3H+2H), 3.83, 4.00(q,15Hz,2H), 4.52(s,2H), 5.08(s,1H), 6.77(s,1H), 6.88(t,55Hz,1H) |
| 4-1-12 | $CF_2H$ | $CHPh_2$ | 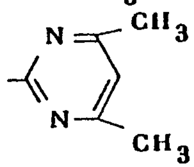 | 3390,1785,1720, 1700 | 2.33(s,6H), 3.55(s,5H,OMe·$SCH_2$−), 4.33(ABq,J=14Hz,2H), 4.60(s,2H), 5.08(s,1H), 6.67(s,1H), 6.90(t,J=56Hz,1H), 6.97(s,1H), 7.2~7.6(m,10H) |
| 4-1-13 | $CF_2H$ | $CHPh_2$ | 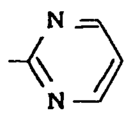 | 3380,1785,1725, 1700 | 3.55(s,5H), 4.28(ABq,J=14Hz,2H), 4.58(s,2H), 5.07(s,1H), 6.93(t,J=56Hz,1H), 7.00(s,1H), 7.2−7.7(m,11H), 8.40(d,J=6Hz,2H) |
| 4-1-14 | $CF_2H$ | $CHPh_2$ | 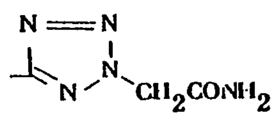 | 3375,1785,1720, 1695,1390,1075 | 3.50(bs,5H), 4.07(bs,2H), 4.45(bs,2H), 5.03(s,1H), 5.18(bs,2H), 6.58(bs,2H), 6.85(t,1H,56Hz), 6.85(s,1H), 7.2−7.6(m,10H) |

Table 4-1-4

| No. | A | B | X | IR(νmax cm⁻¹) | NMR(δ) |
|---|---|---|---|---|---|
| 4-1-15 | $CF_2H$ | $CHPh_2$ | 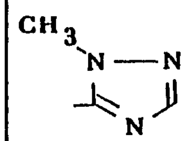 | 3380,1790,1720, 1700 | 3.52(s,3H), 3.60(s,5H), 4.03, 4.17(ABq,2H,14Hz), 4.52(s,2H), 5.03(s,1H), 6.87(s,1H), 6.87(t,1H,56Hz), 7.2-7.7(m,10H), 7.63(s,1H) |
| 4-1-16 | $CF_2H$ | $CHPh_2$ | 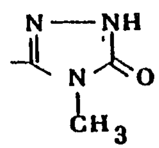 | 3350,3280,1780, 1750 | 3.07(s,3H), 3.50(s,5H), 3.92(s,2H), 4.50(bs,2H), 5.07(s,1H), 6.77(s,1H), 6.88(t,1H,56Hz), 6.98-7.63(m,10H) |
| 4-1-17 | $CF_2H$ | $CHPh_2$ | 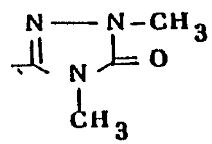 | 3380,1790,1700 | 3.12(s,3H), 3.23(s,3H), 3.53(s,5H), 3.83, 4.00(ABq,2H,15Hz), 4.52(s,2H), 5.08(s,1H), 6.77(s,1H), 6.88(t,1H,55Hz) |
| 4-1-18 | $CF_2H$ | $CHPh_2$ | 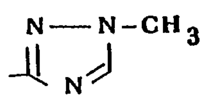 | 3370,1780,1720, 1700 | 3.55(s,5H), 3.73(s,3H) 4.10(s,2H), 4.53(s,2H), 5.05(s,1H), 6.88(s,1H), 6.97(t,1H,56Hz), 7.2-7.6(m,10H), 7.87(s,1H) |
| 4-1-19 | $CF_2H$ | $CHPh_2$ | 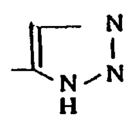 | 3380,1785,1725, 1700 | 3.52(s,5H), 3.85(s,2H), 4.45(s,2H), 5.07(s,1H), 6.85(s,1H), 6.90(t,1H,56Hz), 7.2-7.6(m,10H), 7.83(s,1H) |

Table 4-1-5

| No. | A | B | X | IR(νmax $cm^{-1}$) | NMR (δ) |
|---|---|---|---|---|---|
| 4-1-20 | $CF_2H$ | $CHPh_2$ | 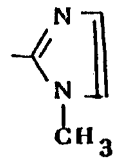 | 3385, 1790, 1726 1703, 1250, 1073 | 3.47 (s, 3H), 3.50 (s, 2H), 3.53 (s, 3H), 3.93 (s, 2H), 4.48 (s, 2H), 5.02 (s, 1H), 6.77 (s, 1H), 6.68, 6.87 (ABq, 2H, 1.0Hz), 6.92 (t, 1H, 56Hz), 7.2-7.6 (m, 10H) |
| 4-1-21 | $CF_2H$ | $CHPh_2$ | 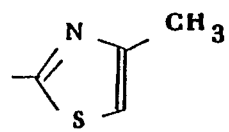 | 3370, 1782, 1710, 1693, 1388, 1257, 1067 mp: 157~158℃ | 2.33 (d, 3H, 1.0Hz), 3.56 (s, 5H), 4.08, 4.40 (ABq, 2H, 15Hz), 4.53 (s, 2H), 5.07 (s, 1H), 6.70 (q, 1H, 1.0Hz), 6.92 (s, 1H), 6.92 (t, 1H, 56Hz), 7.25-7.53 (m, 10H) |
| 4-1-22 | $CF_2H$ | $CHPh_2$ | 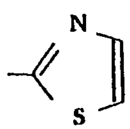 | 3380, 1785, 1720, 1702, 1388, 1259, 1075 mp: 160~161℃ | 3.57 (s, 5H), 4.15, 4.42 (ABq, 2H, 13Hz), 4.57 (s, 2H), 5.06 (s, 1H), 6.95 (s, 1H), 6.95 (t, 1H, 56Hz), 7.2-7.6 (m, 10H) |
| 4-1-23 | $CF_2H$ | $CHPh_2$ | 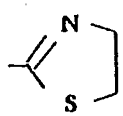 | 3195, 1772, 1721, 1661, 1248, 1081 | 3.52 (s, 3H), 3.70 (s, 2H), 4.15 (s, 2H), 3.36~4.30 (m, 4H), 4.58 (s, 2H), 5.15 (s, 1H), 6.75 (s, 1H), 7.10 (t, 1H, 56Hz), 7.09-7.73 (m, 10H) |
| 4-1-24 | $CF_2H$ | $CHPh_2$ | 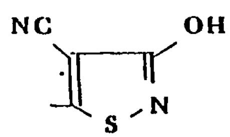 | 3375, 2220, 1790, 1725, 1700 | 3.57 (s, 5H), 4.10 (bs, 2H), 4.50 (bs, 2H), 5.13 (s, 1H), 6.87 (s, 1H), 6.92 (t, 1H, 56Hz), 7.05-7.73 (m, 10H) |

Table 4-1-6

| No. | A | B | X | IR(νmax $cm^{-1}$) | NMR (δ) |
|---|---|---|---|---|---|
| 4-1-25 | $CF_2H$ | $CHPh_2$ | 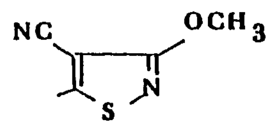 | 3375,2220,1790,<br>1720,1700 | 3.57 (s, 2H), 3.58 (s, 3H), 3.98 (s, 3H),<br>4.12 (s, 2H), 4.52 (s, 2H), 5.15 (s, 1H),<br>6.90 (s, 1H), 6.97 (t, 1H, 56Hz),<br>7.05−7.73 (m, 10H) |
| 4-1-26 | $CF_2H$ | $CHPh_2$ | 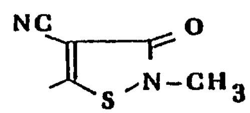 | 3375,2220,1790,<br>1720,1665 | 3.13 (s, 3H), 3.57 (s, 5H),<br>4.00, 4.23 (ABq, 2H, 13Hz), 4.52 (s, 2H),<br>5.10 (s, 1H), 6.87 (s, 1H),<br>6.93 (t, 1H, 56Hz), 7.07−7.67 (m, 10H) |
| 4-1-27 | $CF_2H$ | $CHPh_2$ | 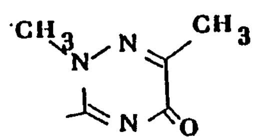 | 3375,1790,1710,<br>1650 | 2.27 (s, 3H), 3.55 (s, 3H), 3.60 (s, 5H),<br>4.08, 4.38 (ABq, 2H, 13Hz), 4.58 (s, 2H),<br>5.07 (s, 1H), 6.92 (s, 1H), 6.95 (t, 1H, 56Hz),<br>7.07−7.67 (m, 10H) |
| 4-1-28 | $CF_2H$ | $CHPh_2$ | 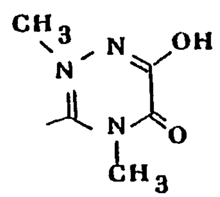 | 3375,1785,1705,<br>1585 | 3.27 (s, 3H), 3.55 (bs, 5H), 4.00 (bs, 2H),<br>4.52 (bs, 2H), 5.10 (s, 1H), 6.92 (s, 1H),<br>6.95 (t, 1H, 56Hz),<br>7.05−7.67 (m, 10H) |
| 4-1-29 | $CF_2H$ | $CHPh_2$ | 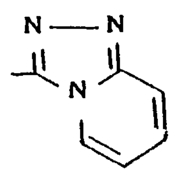 | 3380,1790,1728,<br>1700,1255,1078,<br>mp:118~120℃ | 3.52 (s, 3H), 3.62 (s, 2H), 4.00 (s, 2H),<br>4.59 (s, 2H), 5.05 (s, 1H), 6.67 (s, 1H),<br>6.97 (t, 1H, 55Hz),<br>6.70−8.0 (m, 14H) |

Table 4-1-7

| No. | A | B | X | IR(νmax cm⁻¹) | NMR(δ) |
|---|---|---|---|---|---|
| 4-1-30 | $CF_2H$ | $CHPh_2$ | 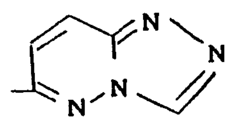 | 3390,1794,1727, 1703,1260,1075 mp:120~125℃ | 3.53(s,3H),3.62(s,2H), 4.17,4.33(ABq,2H,14.5Hz),4.55(s,2H), 5.08(s,1H),6.75,7.83(ABq,2H,10Hz), 6.95(s,1H),6.95(t,1H,56Hz), 7.13-7.57(m,10H),8.05(b,1H), 8.80(s,1H) |
| 4-1-31 | $CF_2H$ | $CHPh_2$ | 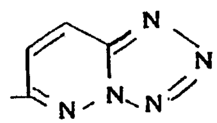 | 3385,1792,1720, 1702,1263,1074 | 3.53(s,5H),4.20,4.45(ABq,2H,13Hz), 4.61(s,2H),5.07(s,1H),6.88(t,1H,56Hz), 6.93(s,1H),7.10,7.66(ABq,2H,10Hz), 7.2-7.6(m,10H) |
| 4-1-32 | $CF_2H$ | $CHPh_2$ | 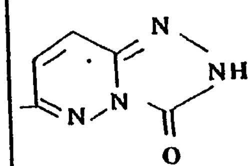 | 3385,3220,1795, 1723,1262,1075 mp:140~145℃ | 3.53(s,5H),4.20(s,2H),4.70(s,2H), 5.10(s,1H),6.92(s,1H),6.92(t,1H,56Hz), 6.42-7.66(m,12H),11.6(bs,1H) |
| 4-1-33 | $CF_2H$ | $CHPh_2$ | 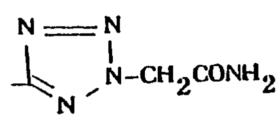 | 3375,1785,1720, 1695,1390,1075 | 3.50(bs,5H),4.07(bs,2H),4.45(bs,2H), 5.03(s,1H),5.18(bs,2H),6.58(bs,2H), 6.85(t,56Hz,1H),7.2~7.6(m), 6.85(s,1H), |
| 4-1-34 | $CF_2H$ | $CHPh_2$ | 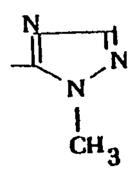 | 3380,1790,1720, 1700 | 3.52(s,3H),3.60(s,5H), 4.03,4.17(ABq,14Hz,2H),4.52(s,2H), 5.03(s,1H),6.87(s,1H), 6.87(t,56Hz,1H),7.2~7.7(m,10H), 7.63(s,1H) |

Table 4-1-8

| No. | A | B | X | IR($\nu$max $cm^{-1}$) | NMR ($\delta$) |
|---|---|---|---|---|---|
| 4-1-35 | $CF_2H$ | $CHPh_2$ | 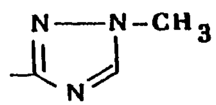 | 3370, 1780, 1720, 1700 | ($CDCl_3-CD_3OD$) 3.55 (s, 5H), 3.73 (s, 3H), 4.10 (s, 2H), 4.53 (s, 2H), 5.05 (s, 1H), 6.88 (s, 1H), 6.97 (t, 56Hz, 1H), 7.2~7.6 (m, 10H), 7.87 (s, 1H) |

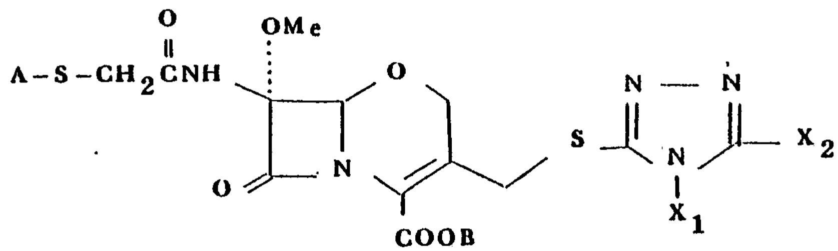

Table 4-2-1

| No. | A | B | $X_1$ | $X_2$ | IR($\nu$max $cm^{-1}$) | NMR ($\delta$) |
|---|---|---|---|---|---|---|
| 4-2-1 | $CF_2H$ | $CHPh_2$ | $CH_3$ | $CF_3$ | 3375, 1787, 1720, 1702 | 3.55 (s, 3H+2H), 3.58 (s, 3H); 4.22 (s, 2H), 4.65 (s, 2H), 5.06 (s, 1H), 6.83 (s, 1H), 6.88 (t, 56Hz, 1H), 7.2~7.7 (m, 11H) |
| 4-2-2 | $CF_2H$ | $CHPh_2$ | $CH_3$ | OH | 3350, 3280, 1780, 1705 | 3.07 (s, 3H), [3.50 (s, 3H)+2H (s)], 3.92 (bs, 2H), 4.50 (bs, 2H), 5.07 (s, 1H), 6.77 (s, 1H), 6.88 (t, 56Hz, 1H), 6.98~7.63 (m, 10H) |
| 4-2-3 | $CF_2H$ | $CHPh_2$ | $NH_2$ | $CH_3$ | (Nujol), 1790, 1780, 1715, 1660 | ($d_6$-DMSO), 2.28 (s, 3H), 3.50 (s, 3H), 3.68 (s, 2H), 4.08 (brs, 2H), 4.60 (brs, 2H), 5.15 (s, 1H), 5.80 (brs, 2H, $D_2O$で消える), 6.90 (s, 1H), 7.37 (t, J=56Hz, 1H), 7.2~7.8 (m, 10H) |
| 4-2-4 | $CF_2H$ | $CHPh_2$ | $NH_2$ | H | (Nujol), 1780, 1720, 1650, | ($d_4$-DMSO), 3.47 (s, 3H), 3.67 (s, 2H), 4.10 (brs, 2H), 4.60 (brs, 2H), 5.17 (s, 1H), 6.03 (brs, 2H), 6.90 (s, 1H), 7.35 (t, J=56Hz, 1H), 7.2~7.8 (m, 10H) 8.47 (s, 1H) |

Table 4-2-2

| No. | A | B | $X_1'$ | $X_2$ | IR($\nu$max $cm^{-1}$) | NMR ($\delta$) |
|---|---|---|---|---|---|---|
| 4-2-5 | $CF_2H$ | $CHPh_2$ | NHCHO | H | 1790, 1720, 1690(sh) | ($d_4$MeOH-$CDCl_3$) 3.53(s, 3H), 3.83(brs, 2H), 4.10(ABq, J=14Hz), 4.50(brs, 2H), 5.07(s, 1H), 6.80(s, 1H), 6.93(t, 1H, J=56Hz), 7.2~7.6(m, 10H), 8.07(s, 1H), 8.17(s, 1H) |
| 4-2-6 | $CF_2H$ | $CHPh_2$ | $NH_2$ | $CF_3$ | (Nujol) 3330, 3250, 3100, 1785, 1700, 1665 | ($d_4$-MeOH) 3.55(s, 3H), 3.43(brs, 2H), 4.13(s, 2H), 4.60(s, 2H), 5.13(s, 1H), 6.80(s, 1H), 7.10(t, J=56Hz, 1H), 7.2-7.6(m, 10H) |
| 4-2-7 | $CF_2H$ | $CHPh_2$ | H | H | 3180, 1785, 1720, 1665 | ($d_6$-acetone-$CDCl_3$) 3.53(s, 3H), 3.73(s, 2H), 4.13, 4.30(ABq, 2H, 14Hz), 4.63(s, 2H), 5.10(s, 1H), 6.93(s, 1H), 7.23(t, 1H, 56Hz), 7.2-7.8(m, 10H), 8.25(s, 2H) |
| 4-2-8 | $CF_2H$ | $CHPh_2$ | $CH_3$ | H | 3380, 1785, 1720, 1705 | 3.38(s, 3H), 3.58(s, 3H), 3.67(s, 2H), 4.08(s, 2H), 4.60(s, 2H), 5.10(s, 1H), 6.87(s, 1H), 7.05(t, 1H, 56Hz), 7.2-7.7(m, 10H), 8.0(s, 1H), 8.53(s, 1H) |
| 4-2-9 | $CF_2H$ | $CHPh_2$ | $C_2H_5$ | H | 3380, 1785, 1700 | 1.32(t, 3H, 7Hz), 3.55(s, 5H), 3.82(q, 2H, 7Hz), 4.18(s, 2H), 4.63(s, 2H), 5.08(s, 1H), 6.87(s, 1H), 6.97(t, 1H, 56Hz), 7.2-7.6(m, 10H), 7.80(s, 1H), 8.02(s, 1H) |

Table 4-2-3

| No. | A | B | $X_1$ | $X_2$ | IR($\nu$max $cm^{-1}$) | NMR($\delta$) |
|---|---|---|---|---|---|---|
| 4-2-10 | $CF_2H$ | $CHPh_2$ | $CH_2CH_2OH$ | H | 3390, 3150, 1785, 1705 | 3.53 (s, 5H), 3.5~4.3 (m, 6H), 4.52 (s, 2H), 5.05 (s, 1H), 6.73 (s, 1H), 6.93 (t, 1H, 56Hz), 7.2-7.6 (m, 10H), 7.95 (s, 2H) |
| 4-2-11 | $CF_2H$ | $CHPh_2$ | $CH_2CH_2CN$ | H | 3390, 2250, 1790, 1720, 1705, 1500, 1395, 1075 | 2.68 (t, 2H, 6Hz), 3.57 (s, 3H), 3.62 (s, 2H), 3.88, 4.25 (ABq, 2H, 13Hz), 4.07 (t, 2H, 6Hz), 4.60 (bs, 2H), 5.08 (s, 1H), 6.80 (s, 1H), 6.97 (t, 1H, 56Hz), 7.2-7.6 (m, 10H), 7.98 (bs, 1H), 8.17 (s, 1H) |
| 4-2-12 | $CF_2H$ | $CHPh_2$ | $CH_2CH_2N(CH_3)_2$ | H | 3370, 1785, 1720, 1700 | 2.20 (s, 6H), 2.50 (t, 2H, 6Hz), 3.57 (s, 3H), 3.63 (s, 2H), 3.87 (t, 2H, 6Hz), 4.17 (s, 2H), 4.63 (s, 2H), 5.10 (s, 1H), 6.88 (s, H), 7.03 (t, 1H, 56Hz), 7.2~7.7 (m, 10H), 8.08 (s, 1H), 8.22 (s, 1H) |
| 4-2-13 | $CF_2H$ | $CHPh_2$ | $CH_2CO_2C_2H_5$ | H | 3380, 1785, 1750, 1710 | 1.27 (t, 3H, 7Hz), 3.57 (s, 5H) 3.8-5.0 (m, 8H), 5.10 (s, 1H), 6.77 (s, 1H), 7.02 (t, 1H, 56Hz), 7.2-7.7 (m, 10H), 8.00 (s, 1H) |
| 4-2-14 | $CF_2H$ | $CHPh_2$ | $CH_2CH_2SO_2NH_2$ | H | | 3.42 (t, 2H, 7Hz), 3.57 (s, 5H), 4.13~4.50 (m, 4H), 4.57 (s, 2H), 5.10 (s, 1H), 6.77 (s, 1H), 7.03 (t, 1H, 56Hz), 7.2-7.6 (m), 8.17 (s, 1H) |

Table 4-2-4

| No. | A | B | $X_1$ | $X_2$ | IR($\nu$max $cm^{-1}$) | NMR($\delta$) |
|---|---|---|---|---|---|---|
| 4-2-15 | $CF_2H$ | $CHPh_2$ | 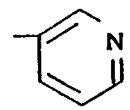 | H | 2380,1785,1720, 1700 | 3.55(s,3H), 3.62(s,2H), 4.00, 4.17(ABq,2H,14Hz), 4.58(s,2H), 5.07(s,1H), 6.80(s,1H), 6.98(t,1H,56Hz), 7.1-7.7(m,12H), 8.07(s,1H), 8.20(s,1H), 8.55(d,1H,3Hz), 8.68(dd,1H,4Hz,2Hz) |
| 4-2-16 | $CF_2H$ | $CHPh_2$ | $CH_3$ | $CH_3$ | 3380,1785,1725, 1705 | 2.20(s,3H), 3.27(s,3H), 3.55(s,3H), 3.65(s,2H), 4.00(s,2H), 4.53(s,2H), 5.05(s,1H), 6.82(s,1H), 7.02(t,1H,56Hz), 7.2-7.7(m,10H), 8.50(s,1H) |
| 4-2-17 | $CF_2H$ | $CHPh_2$ | $CH_3$ | $NH_2$ | 3380,1785,1725, 1700,1620 | ($CDCl_3-CD_3OD$) 3.25(s,3H), 3.55(s,3H), 3.63(s,2H), 3.63, 4.03(ABq,2H,13Hz), 4.50(s,2H), 5.10(s,1H), 6.78(s,1H), 7.03(t,1H,56Hz), 7.2~7.7(m,10H) |

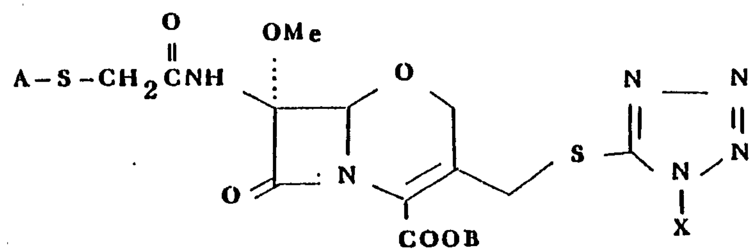

Table 5-1

| №. | A | B | X | IR($\nu$max $cm^{-1}$) | NMR ($\delta$) |
|---|---|---|---|---|---|
| 5-1 | $CF_2H$ | H | $CH_2CH_2N(CH_3)_2$ | 3430,3260,1775,<br>1665,1628<br>mp:162.0~3.5℃ | ($D_2O$) 3.01(s,6H),3.50(s,3H),3.68(s,2H),<br>3.78(t,6Hz,2H),4.07(d,13Hz,1H),<br>4.23(d,13Hz,1H),4.61(s,2H),4.89(t,6Hz,2H),<br>5.20(s,1H),7.11(t,55Hz,1H) |
| 5-2 | $CF_2H$ | H | $CH_2CH_3$ | 3500,3270,2600,<br>1783,1700,1634 | ($d_6$-acetone) 1.50(t,7Hz,3H),3.47(s,3H),<br>3.70(s,2H),4.33(q,7Hz,2H),4.33(s,2H),<br>4.52(s,2H),5.07(s,1H),5.07(bs,1H),<br>7.25(t,57Hz,1H),8.30(bs,1H) |
| 5-3 | $CF_2H$ | H | 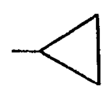 | (Nujol)<br>3366,3600,1783,<br>1704,1635,1600 | ($d_6$-acetone) 1.1~1.4(m,4H),3.3~3.7(m,1H),<br>3.47(s,3H),3.70(s,2H),4.37(s,2H),<br>4.65(s,2H),5.08(s,1H),5.92(bs,1H),<br>7.25(t,58Hz,1H),8.30(bs,1H) |
| 5-4 | $CF_2H$ | H | $CH_2CH_2OH$ | (Nujol)<br>3270,1782,1690,<br>1637<br>mp:53~56℃ | ($d_6$-acetone) 3.47(s,3H),3.70(s,2H),<br>3.93(t,5Hz,2H),4.30(s,2H),<br>4.42(t,5Hz,2H),4.63(s,2H),5.07(s,1H),<br>6.90(br,1H),7.23(t,56Hz,1H),8.30(bs,1H) |

Table 5-2

| No. | A | B | X | IR($\nu$max $cm^{-1}$) | NMR($\delta$) |
|---|---|---|---|---|---|
| 5-5 | $CF_2H$ | H | $CH_2CH_2SCH_3$ | (Nujol) 3270, 2590, 1782, 1700, 1635 | ($d_6$-acetone) 2.10(s, 3H), 3.03(t, 6Hz, 2H), 3.50(s, 3H), 3.73(m, 2H), 4.37(s, 2H), 4.57(t, 6Hz, 2H), 4.68(s, 2H), 5.12(s, 1H), 7.23(t, 56Hz, 1H), 7.63(bs, 1H), 8.25(bs, 1H), |
| 5-6 | $CF_2H$ | H | H | (Nujol) 1790, 1770 | ($d_6$-acetone) 3.47(s, 3H), 3.77(s, 2H), 3.97(s, 3H), 4.30(s, 2H), 4.63(s, 2H), 5.07(s, 1H), 7.23(t, 58Hz, 1H) |
| 5-7 | $CF_2H$ | H | $CH_2OCH_3$ | (KBr) 3280(br), 1785, 1705(br) | ($d_6$-acetone) 3.40(s, 3H), 3.53(s, 3H), 3.77(s, 2H), 4.43(s, 2H), 4.70(s, 2H), 5.15(s, 1H), 5.70(s, 2H), 7.25(t, 57Hz, 1H), 8.27(bs, 1H) |
| 5-8 | $CF_2H$ | H | $CH_2CH=CH_2$ | (KBr) 3275(br), 1785, 1700(br) | ($d_6$-acetone) 3.52(s, 3H), 3.73(s, 2H), 4.38(s, 2H), 4.68(s, 2H), 4.9~6.3(m, 6H), 7.23(t, 57Hz, 1H) |
| 5-9 | $CF_2H$ | H | $CH_2CH2OCH_3$ | (KBr) 3280(br), 1785, 1720~1690 | ($d_6$-acetone) 3.28(s, 3H), 3.52(s, 3H), 3.80(s, 2H), 3.83(t, 4Hz, 2H), 4.37(s, 2H), 4.57(t, 4Hz, 2H), 4.70(s, 2H), 5.17(s, 1H), 7.27(t, 57Hz, 1H) |
| 5-10 | $CF_2H$ | H | $CH_2CH(CH_3)_2$ | (KBr) 1780, 1705 | ($d_6$-acetone) 0.95(d, 7Hz, 6H), 2.23(q, 7Hz, 1H), 3.50(s, 3H), 3.77(s, 2H), 4.17(d, 2H), 4.40(s, 2H), 4.70(s, 2H), 5.17(s, 1H), 7.27(t, 56Hz, 1H) |

Table 5-3

| No. | A | B | X | IR($\nu$max $cm^{-1}$) | NMR ($\delta$) |
|---|---|---|---|---|---|
| 5-11 | $CF_2H$ | H | $CH_2CN$ | (KBr) 3310, 2980, 2940, 1785, 1700, 1068 | ($CD_3OD$) 3.53 (s, 3H), 3.60 (s, 2H), 4.30 (s, 2H), 4.60 (s, 2H), 5.05 (s, 1H), 5.60 (s, 2H), 7.07 (t, 1H, 57Hz) |
| 5-12 | $CF_2H$ | H | $CH_2CH_2CN$ | (Nujol) 3250, br, 2700br, 2248, 1783, 1708, 1688, 1068 | (DMSO) 3.15 (t, 2H, 6.7Hz), 3.40 (s, 3H), 3.63 (s, 2H), 4.25 (s, 2H), 4.52 (s, 2H), 4.58 (t, 2H, 6.7Hz), 5.08 (s, 1H), 7.28 (t, 1H, 56Hz) |
| 5-13 | $CF_2H$ | H | $CH_2CO_2CH_3$ | (KBr) 3300, 3000, 1785, 1755, 1705, 1070 | ($CD_3OD$) 3.53 (s, 3H), 3.62 (s, 2H), 3.82 (s, 3H), 4.30 (s, 2H), 4.57 (s, 2H), 5.07 (s, 1H), 5.30 (s, 2H), 7.10 (t, 1H, 56Hz), |
| 5-14 | $CF_2H$ | H | $CH_2CO_2C_2H_5$ | (Nujol) 1762, 1688, 1605, 1228, 1066 | (DMSO) 1.22 (t, 3H, 7.5Hz), 3.40 (s, 3H), 3.65 (s, 2H), 4.20 (q, 2H, 7.5Hz), 4.15, 4.41 (ABq, 2H, 13Hz), 4.38 (s, 2H), 4.95 (s, 1H), 5.38 (s, 2H), 7.32 (t, 1H, 56Hz) |
| 5-15 | $CF_2H$ | H | $CH_2CONH_2$ | (KBr) 3420, 3340, 1785, 1690, 1070 | ($CD_3OD$) 3.55 (s, 3H), 3.62 (s, 2H), 4.27 (s, 2H), 4.58 (s, 2H), 5.07 (s, 1H), 5.13 (s, 2H), 7.08 (t, 1H, 57Hz) |
| 5-16 | $CF_2H$ | H | $CH_2CONHCH_3$ | (KBr) 3340, 1785, 1680, 1068 | ($CD_3OD$) 2.80 (s, 3H), 3.53 (s, 3H), 3.62 (s, 2H), 4.25 (s, 2H), 4.57 (s, 2H), 5.10 (bs, 3H), 7.10 (t, 1H, 56Hz) |

Table 5-4

| No. | A | B | X | IR($\nu$max $cm^{-1}$) | NMR($\delta$) |
|---|---|---|---|---|---|
| 5-17 | $CF_2H$ | H | $CH_2CON(CH_3)_2$ | (KBr) 3270, 3000, 1785, 1715, 1660, 1070 | ($CD_3OD$) 2.98(s, 3H), 3.17(s, 3H), 3.53(s, 3H), 3.60(s, 2H), 4.27(s, 2H), 4.57(s, 2H), 5.10(s, 1H), 5.43(s, 2H), 7.13(t, 1H, 56Hz) |
| 5-18 | $CF_2H$ | H | $CH_2COCH_3$ | (Nujol) 1766, 1730, 1683, 1126, 1062 | (DMSO) 2.27(s, 3H), 3.57(s, 3H), 3.66(s, 2H), 3.93, 4.32(ABq, 2H, 13Hz), 4.33(s, 2H), 4.92(s, 1H), 5.52(s, 2H), 7.33(t, 1H, 56Hz) |
| 5-20 | $CF_2H$ | H | $CH_2CH_2CO_2H$ | (Nujol) 1780, 1720, 1700 | ($CD_3COCD_3$) 3.03(t, 2H, 7Hz), 3.46(s, 3H), 3.70(s, 2H), 4.33(s, 2H), 4.53(t, 2H, 7Hz), 4.63(s, 2H), 5.08(s, 1H), 7.21(t, 1H, 56Hz), 8.20(bs, 1H) |
| 5-21 | $CF_2H$ | H | $CH_2CO_2H$ | (Nujol) 1770, 1710, 1680 | (DMSO) 3.50(s, 3H), 3.73(s, 2H), 4.30, 4.43(ABq, 2H, 14Hz), 4.62(s, 2H), 5.13(s, 1H), 5.26(s, 2H), 7.00(t, 1H, 56Hz) |

Table 5-5

| No. | A | B | X | IR($\nu$max $cm^{-1}$) | NMR($\delta$) |
|---|---|---|---|---|---|
| 5-22 | $CF_2H$ | H | $CH_2CH_2CO_2CH_3$ | (KBr) 3400, 1775,1740,1690, 1615,1205,1065 | ($CD_3OD$) 3.05(t,2H,7Hz), 3.53(s,3H), 3.63(s,2H), 3.68(s,3H), 4.35(s,2H), 4.53(s,2H), 4.57(t,2H,7Hz), 5.07(s,1H), 7.13(t,1H,57Hz) |
| 5-23 | $CF_2H$ | H | $CH_2CH_2CO_2C_2H_5$ | (KBr) 3400, 1775,1733,1690, 1615,1205,1067 | ($CD_3OD$) 1.20(t,3H,7Hz), 3.02(t,2H,7Hz), 3.52(s,3H), 3.62(s,2H), 4.10(q,2H,7Hz), 4.32(s,2H), 4.52(s,2H), 4.53(t,2H,7Hz), 5.03(s,1H), 7.08(t,1H,56Hz) |
| 5-24 | $CF_2H$ | H | $CH_2CH_2CONH_2$ | (Nujol) 1790, 1708,1680 | (DMSO) 2.73(t,2H,7Hz), 3.40(s,3H), 3.65(s,2H), 4.25(s,2H), 4.43(t,2H,7Hz), 4.57(s,2H), 5.01(s,1H), 6.97(b,2H) 7.33(t,1H,56Hz), 7.40(b,1H) |
| 5-25 | $CF_2H$ | H | $CH_2CH_2SO_2NH_2$ | (KBr) 3310, 3270,1785,1710, 1695,1520,1338, 1150,1067 | ($CD_3OD$) 3.53(s,3H), 3.62(s,2H), 3.72(t,2H,7Hz), 4.27(s,2H), 4.62(s,2H), 4.78(t,2H,7Hz), 5.08(s,1H), 7.10(t,1H,57Hz) |
| 5-26 | $CF_2H$ | H | $-CH_2CONH-$ 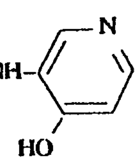 | (Nujol) 1670, 1780 | ($d_6$-acetone+$d_6$-DMSO) 3.47(3H,s), 3.72(s,2H), 4.32(s,2H), 4.62(s,2H), 5.08(s,1H), 5.57(s,2H), 6.80(d,1H,7Hz), 7.20(t,1H,56Hz), 7.92(d,1H,7Hz), 8.98(s,1H) |

Table 5-6

| No. | A | B | X | IR($\nu$max $cm^{-1}$) | NMR($\delta$) |
|---|---|---|---|---|---|
| 5 - 27 | $CF_2H$ | H | 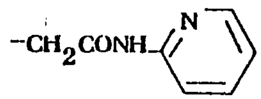 | (Nujol) 1670, 1775 | ($d_6$-acetone) 3.48(s, 3H), 3.75(s, 2H), 4.32(s, 2H), 4.63(s, 2H), 5.10(s, 1H), 5.75(s, 2H), 7.23(t, 1H, 56Hz), 6.6~8.10(m, 3H) |
| 5 - 28 | $CF_2H$ | H | $-CH_2CONHCONH_2$ | (Nujol) 1710, 1780 | ($d_6$-acetone) 3.48(s, 3H), 3.75(s, 2H), 4.27(s, 2H), 4.63(s, 2H), 5.08(s, 1H), 5.42(s, 2H), 7.17(t, 1H, 56Hz) |
| 5 - 29 | $CF_2H$ | H | $-CH_2SO_3H$ | (KBr) 3440, 2600, 1778, 1684, 1660, 1511 | ($d_6$-acetone - $CD_3OD$) 3.50(s, 3H), 3.70(s, 2H), 4.31(s, 2H), 4.64(s, 2H), 5.10(s, 1H), 5.25(s, 2H), 7.23(t, 58Hz, 1H) |
| 5 - 30 | $CF_2H$ | H | 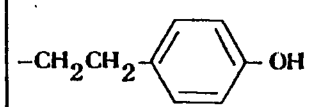 | | ($d_6$-acetone) 3.10(t, 2H, 7Hz), 3.47(s, 3H), 3.71(s, 2H), 4.23(s, 2H), 4.47(t, 2H, 7Hz), 4.53(s, 2H), 5.03(s, 1H), 6.66, 6.86(ABq, 4H, 8Hz), 7.17(t, 1H, 55Hz), |
| 5 - 31 | $CF_2H$ | H | $-C(CH_3)_2CO_2H$ | | ($d_6$-acetone) 1.97(s, 6H), 3.50(s, 2H), 3.70(s, 3H), 4.42(s, 2H), 4.63(s, 2H), 5.12(s, 1H), 7.27(t, 1H, 58Hz) |
| 5 - 32 | $CF_2H$ | H | $-CH_2CH_2OCONH_2$ | | ($d_6$-acetone) 3.50(s, 3H), 3.73(s, 2H), 4.36(s, 2H), 4.67(s, 2H), 4.3~4.7(m, 4H), 5.10(s, 1H), 7.25(t, 1H, 56Hz) |

Table 5-7

| No. | A | B | X | IR($\nu max$ $cm^{-1}$) | NMR ($\delta$) |
|---|---|---|---|---|---|
| 5-33 | $CF_2H$ | H | $-CH=CH_2$ | | ($d_6$-acetone) 3.52 (s, 3H), 3.75 (s, 2H), 4.45 (s, 2H), 4.72 (s, 2H), 5.13 (s, 1H), 5.52 (dd, 1H, 2Hz, 9Hz), 5.97 (dd, 1H, 2Hz, 16Hz), 7.23 (t, 1H, 57Hz), 7.27 (dd, 1H, 9Hz, 16Hz) |
| 5.-34 | $CF_2H$ | H | 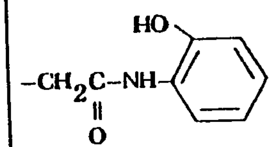 | | ($d_6$-acetone $-CD_3OD$) 3.50 (s, 3H), 3.29 (s, 2H), 4.33 (s, 2H), 4.64 (s, 2H), 5.11 (s, 1H), 5.50 (s, 2H), 6.70~7.15 (m, 4H), 7.22 (t, 1H, 56Hz) |
| 5-35 | $CF_2H$ | H | 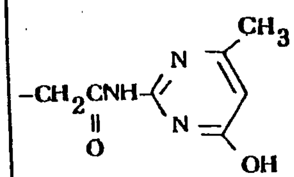 | (Nujol) 1690, 1780, 3230 | |

Table 6-1

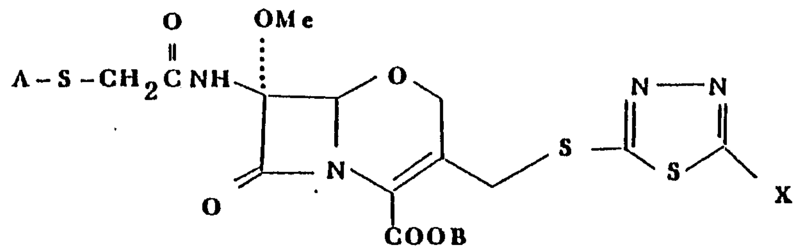

| No. | A | B | X | IR ($\nu$max $cm^{-1}$) | NMR ($\delta$) |
|---|---|---|---|---|---|
| 6-1 | $CF_2H$ | H | $NH_2$ | (nujol)3170,1780, 1675 | ($d_6$-DMSO) 7.31 (t, 1H, 56Hz), 5.01 (S, 1H), 4.50 (S, 2H), 4.10 (S, 2H), 3.67 (S, 2H), 3.40 (S, 3H) |
| 6-2 | $CF_2H$ | H | H | (KBr),1783,1692 | ($d_6$-acetone) : 3.52 (S, 3H, OMe) ; 3.73 (s, 2H, $SCH_2$) ; 4.50 (ABq, 14Hz, 2H, $CH_2S$-) ; 4.68 (S, 2H, ) ; 5.15 (S, 1H, $C_6$-H) ; 7.27 (t, 56HZ, 1H, $CF_2H$) |
| 6-3 | $CF_2H$ | H | $-CH_2$-NHBoc | (Nujol)1780,1670 | ($d_4$-MeOH) : 3.50 (S, 3H, OMe), 3.60 (S, 2H, $CH_2$), 4.40 (ABq, 2H), 4.63 (S, 4H, $CH_2 \times 2$), 5.03 (S, 1H), 7.13 (t, 1H, 56HZ) |
| 6-4 | $CF_2H$ | H | $CH_2OH$ | (Nujol),1780,1690 | ($d_4$-MeOH) : 3.53 (S, 3H, OMe), 3.60 (S, 2H, $CH_2$), 4.40 (ABq, 2H), 4.63 (s, 2H, $CH_2$), 4.93 (S, 2H, $CH_2$), 5.07 (S, 1H, $C_6$-H), 7.13 (t, 1H, 56Hz) |
| 6-5 | $CF_2H$ | H | $CH_2OCH_2COOBH$ | (Nujol),1785,1710, 1690 | ($d_4$-MeOH) : 3.50 (s, 3H, OMe), 3.62 (S, 2H, $CH_2$), 4.22 (s, 2H, $CH_2$), 4.37 (ABq, 2H), 4.60 (s, 2H, $CH_2$), 4.93 (s, 2H, $CH_2$), 5.07 (s, 1H) 7.10 (t, 1H, J=56HZ) |

Table 6-2

| No. | A | B | X | IR ( $\nu$ max $cm^{-1}$ ) | NMR ( $\delta$ ) |
|---|---|---|---|---|---|
| 6-6 | $CF_2H$ | H | $CH_2CN$ | (Nujol) 1780, 1700(sh), 1680 | ($CDCl_3$-$d_4$·MeOH) : 3.60 (s, 3H), 3.62 (s, 2H), 4.43 (br. S, 2H), 4.63 (s, 2H), 4.77 (S, 2H), 5.08 (s, 1H), 7.07 (t, 1H, 56HZ) |
| 6-7 | $CF_2H$ | H | $CH_2$-COOMe | (KBr), 1783, 1735, 1703 | ($d_4$-MeOH) : 3.55 (s, 3H), 3.63 (br. s, 2H), 3.77 (s, 3H), 4.42 (ABq, 14Hz, 2H), 4.62 (br. s, 2H), 4.83 (brs, 2H), 5.08 (s, 1H), 6.93 (t, 56Hz, 1H) |
| 6-8 | $CF_2H$ | H | $CH_2$-COOBH | (Nujol) 1775, 1700(broad) | ($d_4$-MeOH) : 3.52 (s, 3H), 3.60 (s, 2H), 4.37 (ABq, 14Hz, 2H), 4.60 (brs, 2H), 4.83 (br. s, 2H), 5.05 (s, 1H), 7.10 (t, 56HZ, 1H) |
| 6-9 | $CF_2H$ | H | $NH_2$ | (KBr), 1780, 1673(br), 1628 | ($d_4$-MeOH) : 3.57 (s, 3H), 3.63 (s, 2H), 4.17 (ABq, 14HZ, 2H), 4.60 (brs, 2H), 5.07 (s, 1H), 7.10 (t, 56Hz, 1H) |
| 6-10 | $CF_2H$ | H | SMe | (Nujol), 1780, 1700(sh), 1690 | ($d_4$-MeOH) : 2.77 (s, 3H), 3.50 (s, 3H), 3.63 (s, 2H), 4.33 (brs, 2H), 4.62 (s, 2H), 5.08 (s, 1H), 7.08 (t, 56HZ, 1H) |
| 6-11 | $CF_2H$ | H | COOEt | (KBr), 1785, 1740(sh), 1722 | ($d_4$-MeOH) : 1.43 (t, 7Hz, 3H), 3.55 (s, 3H), 3.62 (s, 2H), 4.50 (q, 7Hz, 2H), 4.57 (ABq, 2H), 4.67 (s, 2H), 5.10 (s, 1H), 7.10 (t, 56Hz, 1H) |

Table 6-3

| No. | A | B | X | IR ( $\nu max\ cm^{-1}$ ) | NMR ( $\delta$ ) |
|---|---|---|---|---|---|
| 6-12 | $CF_2H$ | H | $CH_3$ | (Nujol) 1780, 1720(sh), 1690 | ($d_4$-MeOH) : 2.70(s, 3H), 3.50(S, 3H), 3.60(brs, 2H), 4.33(ABq, 14Hz, 2H), 4.60(s, 2H), 5.03(s, 1H), 7.07(t, 56Hz, 1H) |

Table 7-1

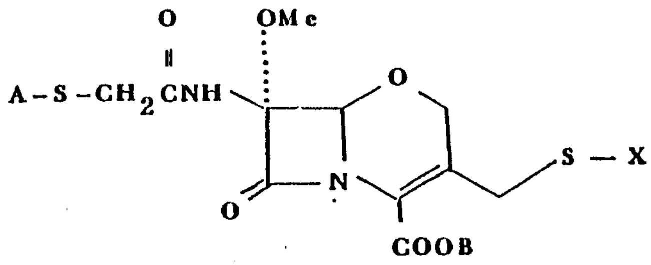

| № | A | B | X | IR ($\nu$max $cm^{-1}$) | NMR ($\delta$) |
|---|---|---|---|---|---|
| 7-1 | $CF_2H$ | H | 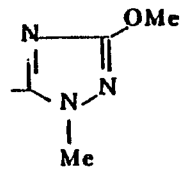 | 1782~1752(br), 1697, 1542 | ($CD_3COCD_3$) : 3.52 (S, 3H), 3.63 (S, 3H), 3.74 (S, 2H), 4.25 (brS, 2H), 4.65 (brS, 2H), 5.12 (S, 1H), 7.25 (t, 57Hz, t), 8.33(brS, 1H) |
| 7-2 | $CF_2H$ | H | 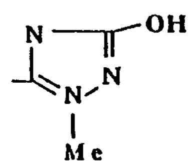 | (Nujol) 1783, 1685, 1630, 1530 | ($CD_3COCD_3$) : 3.50~4.05 (m, 10H), 4.62 (brS, 2H), 5.10 (S, 1H), 7.22(t, 56Hz, 1H) |
| 7-3 | $CF_2H$ | H | 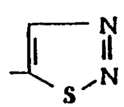 | | ($CD_3COCD_3$) : 3.52 (S, 3H), 4.33 (S, 2H), 4.67 (S, 2H), 5.17 (1H, S), 7.27(t, 57Hz, 1H), 8.67 (S, 1H) |
| 7-4 | $CF_2H$ | H | 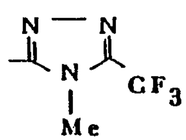 | (KBr) 3340, 1788, 1700, 1640 | ($d_6$-acetone) : 3.53 (3H, S), 3.75 (2H, S), 3.83 (3H, S), 4.28 (2H, S), 4.70 (2H, S), 5.12 (1H, S), 7.25 (1H, t, 57Hz) |

Table 7-2

| № | A | B | X | IR ($\nu$ max $cm^{-1}$) | NMR ($\delta$) |
|---|---|---|---|---|---|
| 7-5 | $CF_2H$ | H | 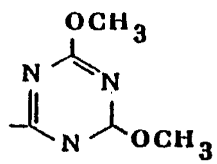 | 1787, 1749, 1704, 1632 | 3.50 (S, 3H), 3.57 (S, 2H), 3.97 (S, 6H), 4.23 (S, 2H), 4.58 (S, 2H), 5.08 (S, 1H), 6.95 (t, 57Hz), 7.20 (S, 1H), 7.68 (S, 1H) |
| 7-6 | $CF_2H$ | H | 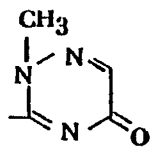 | (Nujol) 3320, 1785, 1705, 1635 Rf=0.382 (EtOAc : HOAc : $H_2O$=8 : 1 : 1) | ($CDCl_3$-$CD_3OD$) 2.27 (3H, S), [3.53 (3H, S) + 3.56 (2H, brS)], 3.77 (3H, S), 4.25, 4.41 (2H, q, 14Hz), 4.58 (2H, S), 5.05 (1H, S), 7.02 (1H, t, 56Hz) |
| 7-7 | $CF_2H$ | H | 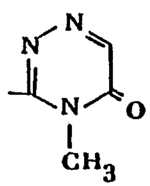 | (Nujol) 3200, 1785, 1710, 1585 Rf=0.276 (EtOAc : HOAc : $H_2O$=8:1:1 | ($CDCl_3$-$CD_3OD$) 3.47 (3H, S), [3.55 (3H, S) + 3.58 (2H, S)], 4.10, 4.27 (2H, q, 15Hz), 4.57 (2H, S), 5.08 (1H, S), 7.07 (1H, t, 56Hz) |
| 7-8 | $CF_2H$ | H | 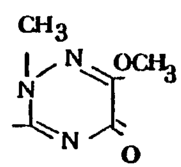 | (Nujol) 3200, 1780, 1700, 1650 Rf=0.311 (EtOAc : HOAc : $H_2O$= 8 : 1 : 1) | ($CDCl_3$-$CD_3OD$) [3.53 (3H, S) +3.57 (2H, brS)], 3.57 (3H, S), 3.93 (3H, S) 4.28, 4.42 (2H, q, 14Hz) 4.58 (2H, brS), 5.07 (1H, S) 7.02 (1H, t, 56Hz) |

Table 7-3

| № | A | B | X | IR ( ν max $cm^{-1}$ ) | NMR ( δ ) |
|---|---|---|---|---|---|
| 7-9 | $CF_2H$ | H | 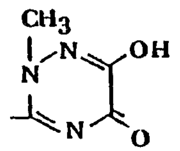 | (Nujol) 3200, 1760, 1745, 1725, 1680, 1630<br>Rf = 0.281 ( EtOAc : HOAc : $H_2O$ = 5:1:1) | ($CDCl_3$-$CD_3OD$) [3.55 (3H, S) + 3.70 (3H, S) + 2H], 4.35 (2H, brS), 4.60 (2H, S), 5.07 (1H, S), 7.00 (1H, t, 56Hz) |
| 7-10 | $CF_2H$ | H | 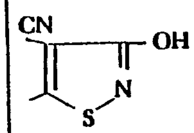 | (Nujol) 3150, 2200, 1780, 1650<br>Rf = 0.554 ( EtOAc : HOAc : $H_2O$ = 8 : 1 : 1 ) | ($CDCl_3$-$CD_3OD$) [3.55 (3H, S) + 2H], 4.18 (2H, S), 4.55 (2H, S), 5.05 (1H, S), 6.98 (1H, t, 56Hz) |
| 7-11 | $CF_2H$ | H | 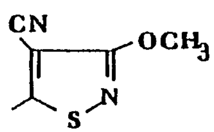 | (Nujol) 3260, 2220, 1780, 1700<br>Rf = 0.417 ( EtOAc : HOAc : $H_2O$ = 18 : 1 : 1) | ($CDCl_3$-$CD_3OD$) [3.55 (3H, S) + (2H, S)], 4.08 (3H, S), 4.25 (2H, S), 4.57 (2H, S), 5.12 (1H, S), 7.05 (1H, t, 56Hz) |
| 7-12 | $CF_2H$ | H | 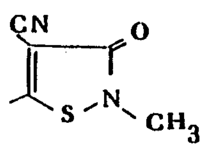 | (Nujol) 3275, 2220, 1795, 1720, 1650<br>Rf = 0.364 ( EtOAc : HOAc : $H_2O$ = 8:1:1) | ($CDCl_3$-$CD_3OD$) 3.37 (3H, S), [3.57 (3H, S) + (2H, S)], 4.13, 4.33 (2H, q, 14Hz), 4.57 (2H, S), 5.13 (1H, S), 7.03 (1H, t, 56Hz) |

Table 7-4

| No. | A | B | X | IR ($\nu$max $cm^{-1}$) | NMR ($\delta$) |
|---|---|---|---|---|---|
| 7-13 | $CF_2H$ | H | 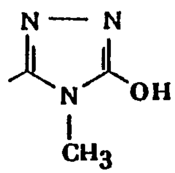 | (Nujol) 3250, 1790, 1700 Rf=0.20 (EtOAc: HOAc: $H_2O$=8:1:1) | ($CDCl_3$-$CD_3Cl$) 3.27 (3H, S), [3.53 (3H, S) + 3.57 (2H, S)], 4.00 (2H, S), Ca 4.50 (2H, S), 5.12 (1H, S), 7.07 (1H, t, 56Hz) |
| 7-14 | $CF_2H$ | H | 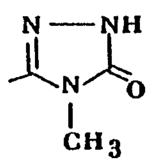 | (Nujol) 3250, 1790, 1690 Rf=0.40 (EtOAc: HOAc: $H_2O$=8:1:1) | ($CDCl_3$-$CD_3OD$) 3.25 (3H, S), 3.45 (3H, S), 3.55 (3H, S+2H), 3.98 (2H, S), 4.55 (2H, S), 5.08 (1H, S), 7.02 (1H, t, 56Hz) |
| 7-15 | $CF_2H$ | H | 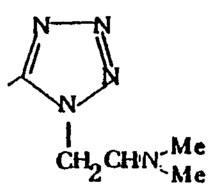 | (KBr) 1780, 1705 | ($d_6$-acetone): 0.95 (d, 7Hz, 6H), 2.23 (quint, 7Hz, 1H), 3.50 (S, 3H), 3.77 (S, 2H), 4.17 (d, 2H), 4.40 (S, 2H), 4.70 (S, 2H), 5.17 (S, 1H), 7.27 (t, 56Hz, 1H) |
| 7-16 | $CF_2H$ | H | 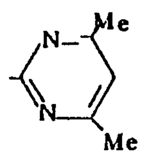 | (Nujol) 1780, 1710 1690 | ($d_4$MeOH): 2.07 (S, 6H), 3.52 (S, 3H), 3.60 (S, 2H), 4.30 (ABq, J=14Hz·2H), 4.90 (S, 2H), 5.02 (S, 1H), 6.90 (S, 1H), 7.10 (t, J=56Hz, 1H) |

Table 7-5

| No. | A | B | X | IR ($\nu$ max $cm^{-1}$) | NMR ($\delta$) |
|---|---|---|---|---|---|
| 7-17 | $CF_2H$ | H | 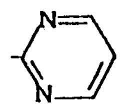 | (Nujol) 1780, 1690 | ($d_4$MeOH): 3.50 (S, 3H), 3.57 (S, 2H) 4.27 (ABq J=14Hz, 2H), 4.57 (S, 2H), 5.00 (S, 1H), 7.10 (t, J=6Hz, 1H), 8.53 (d, J=6Hz, 2H) |
| 7-18 | $CF_2H$ | H | 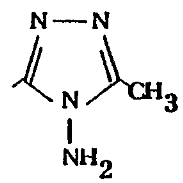 | (KBr) 3230, 1785, 1690, 1630 | ($d_4$-MeOH): 2.50 (S, 3H), 3.50 (S, 3H), 3.62 (S, 2H), 4.13 (S, 2H), 4.60 (S, 2H), 5.05 (S, 1H), 7.07 (t, 56Hz, 1H) |
| 7-19 | $CF_2H$ | H | 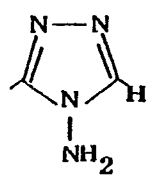 | (Nujol) 1770, 1660 | ($d_4$-MeOH): 3.50 (S, 3H), 3.63 (S, 2H), 4.13 (S, 2H), 4.60 (S, 2H), 5.05 (S, 1H), 7.08 (t, 56Hz, 1H) |
| 7-20 | $CF_2H$ | H | 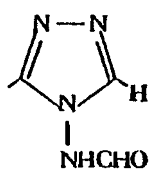 | (Nujol) 1775, 1700 1850(sh) | ($d_4$MeOH): 3.53 (S, 3H), 3.63 (S, 2H), 4.13 (S, 2H), 4.53 (S, 2H), 5.07 (S, 1H), 7.07 (t, 56Hz, 1H), 8.33 (S, 1H) |

Table 7-6

| № | A | B | X | IR ( ν max $cm^{-1}$ ) | NMR ( δ ) |
|---|---|---|---|---|---|
| 7-21 | $CF_2H$ | H | 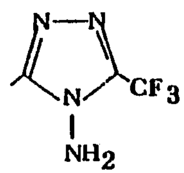 | (KBr) 3320, 3280, 3210, 1785, 1700 | ($d_4$MeOH) 3.53 (S, 3H), 3.63 (S, 2H), 4.23 (S, 2H), 4.67 (S, 2H), 5.12 (S, 1H), 7.15 (t, 56Hz, 1H) |
| 7-22 | $CF_2H$ | H | 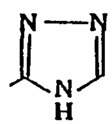 | (KBr) 3260, 1775, 1690 | [$CDCl_3$-$CD_3OD$] 3.55 (s, 5H), 4.17 (s, 2H), 4.57 (s, 2H), 5.08 (s, 1H), 7.03 (t, 1H, 56Hz), 8.22 (s, 1H) |
| 7-23 | $CF_2H$ | H | 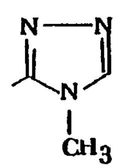 | (KBr) 1780, 1695 | [$CDCl_3$-$CD_3OD$] 3.53 (s, 3H), 3.58 (s, 2H), 3.68 (s, 3H), 3.95, 4.22 (ABq, 2H, 13Hz), 4.60 (s, 2H), 5.10 (s, 1H), 7.03 (t, 1H, 56Hz), 8.35 (s, 1H) |
| 7-24 | $CF_2H$ | H | 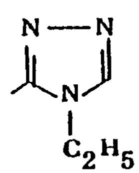 | (KBr) 1785, 1695 | [$CDCl_3$-$CD_3OD$] 1.45 (t, 3H, 7Hz), 3.52 (s, 5H), 4.03 (q, 2H, 7Hz), 4.08 (s, 2H), 4.57 (s, 2H), 5.07 (s, 1H), 6.97 (t, 1H, 56Hz), 8.33 (s, 1H) |

Table 7-7

| No. | A | B | X | IR ( ν max $cm^{-1}$ ) | NMR ( δ ) |
|---|---|---|---|---|---|
| 7 - 25 | $CF_2H$ | H | 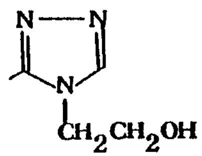 | (KBr) 3400, 1789, 1693 | [$CDCl_3$-$CD_3OD$] 3.53 (s, 3H), 3.58 (s, 2H), 3.6 - 4.3 (m, 6H), 4.53 (s, 2H), 5.07 (s, 1H), 7.03 (t, 1H, 56Hz), 8.50 (s, 1H) |
| 7 - 26 | $CF_2H$ | H | 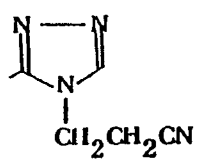 | (KBr) 3360, 2262, 1785, 1695, 1510, 1065 | 3.05 (t, 2H, 7Hz), 3.53 (s, 3H), 3.63 (s, 2H), 4.10 (s, 2H), 4.43 (t, 2H, 7Hz), 4.60 (s, 2H), 5.10 (s, 1H), 7.12 (t, 1H, 56Hz), 8.70 (s, 1H) |
| 7 - 27 | $CF_2H$ | H | 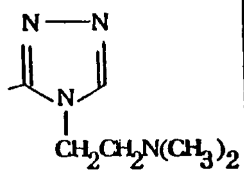 | (KBr) 1783, 1680 | [$CDCl_3$-$CD_3OD$] 2.97 (s, 6H), 3.53 (s, 3H), 3.63 (bs, 4H), 4.56 (bs, 4H), 5.07 (s, 1H), 7.05 (t, 1H, 56Hz), 8.67 (s, 1H) |
| 7 - 28 | $CF_2H$ | H | 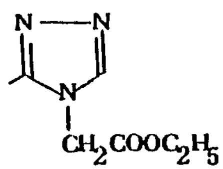 | (KBr) 3300, 1786, 1750, 1700 | [$CDCl_3$-$CD_3OD$] 1.32 (t, 3H, 7Hz), 3.52 (s, 5H), 3.92, 4.18 (ABq, 2H, 12Hz), 4.27 (q, 2H, 7Hz), 4.55 (s, 2H), 4.83 (s, 2H), 5.08 (s, 1H), 7.00 (t, 1H, 56Hz), 8.38 (s, 1H) |

Table 7-8

| № | A | B | X | IR ($\nu$ max $cm^{-1}$) | NMR ($\delta$) |
|---|---|---|---|---|---|
| 7-29 | $CF_2H$ | H | 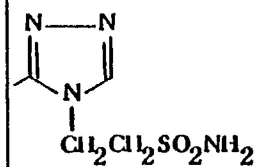 | (KBr)3390, 1780, 1690, 1510, 1335, 1150, 1065 | (DMSO) 3.38 (s, 3H), 3.45 (t, 2H, 7Hz), 3.63 (s, 2H), 4.07 (bs, 2H), 4.35 (t, 2H, 7Hz), 4.53 (bs, 2H), 5.05 (s, 1H), 7.08 (s, 1H), 7.28 (t, 1H, 56Hz), 8.62 (s, 1H) |
| 7-30 | $CF_2H$ | H | 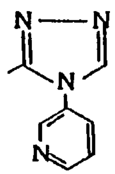 | (KBr)3420, 1785, 1695 | ($CD_3Cl-CD_3OD$) 3.52 (s, 3H), 3.58 (s, 2H), 4.00, 4.17 (ABq, 2H, 12Hz), 4.50 (s, 2H), 5.03 (s, 1H), 7.02 (t, 1H, 56Hz), 7.5, 7.9 (m, 2H), 8.52 (s, 1H), 8.7 (m, 2H) |
| 7-31 | $CF_2H$ | H | 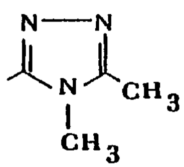 | (KBr)3420, 1782, 1690 | ($CDCl_3-CD_3OD$) 2.48 (s, 3H), 3.53 (s, 3H), 3.60 (s, 5H), 3.92, 4.15 (ABq, 2H, 12Hz), 4.58 (s, 2H), 5.08 (s, 1H), 7.02 (t, 1H, 56Hz) |
| 7-32 | $CF_2H$ | H | 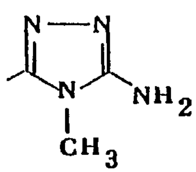 | (KBr)3360, 1782, 1685 | ($CDCl_3$-DMSO) 3.40 (s, 6H), 3.63 (s, 2H), 3.98 (s, 2H), 4.48 (s, 2H), 5.05 (s, 1H), 7.25 (t, 1H, 56Hz) |

Table 7-9

| No. | A | B | X | IR ($\nu$ max $cm^{-1}$) | NMR ($\delta$) |
|---|---|---|---|---|---|
| 7-33 | $CF_2H$ | H | 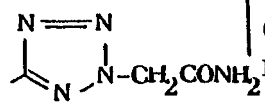 | (KBr)3420, 3340, 1780, 1690, 1065 | [$CD_3OD$] 3.53 (s, 3H), 3.62 (s, 2H), 4.12, 4.35 (ABq, 2H, 14Hz), 4.58 (s, 2H), 5.05 (s, 1H), 5.42 (s, 2H), 7.12 (t, 1H, 56Hz) |
| 7-34 | $CF_2H$ | H | 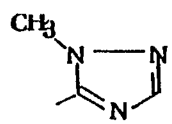 | (KBr)3280, 1783, 1700 | [$CDCl_3$-$CD_3OD$] 3.63 (s, 5H), 3.82 (s, 3H), 4.18 (s, 2H), 4.55 (s, 2H), 5.08 (s, 1H), 6.98 (t, 1H, 56Hz), 7.83 (s, 1H) |
| 7-35 | $CF_2H$ | H | 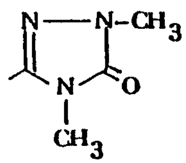 | (Nujol) 3250, 1790, 1690 | [$CDCl_3$-$CD_3OD$] 3.25 (s, 3H), 3.45 (s, 3H), 3.55 (s, 5H), 3.98 (s, 2H), 4.55 (s, 2H), 5.08 (s, 1H), 7.02 (t, 1H, 56Hz) |
| 7-36 | $CF_2H$ | H | 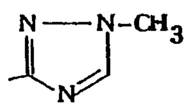 | (KBr)1785, 1700 | [$CDCl_3$-$CD_3OD$] 3.50 (s, 5H), 3.85 (s, 3H), 4.00, 4.20 (ABq, 2H, 13Hz), 4.53 (s, 2H), 5.03 (s, 1H), 6.72 (t, 1H, 56Hz), 7.98 (s, 1H) |
| 7-37 | $CF_2H$ | H | 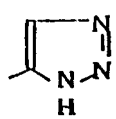 | | [$CDCl_3$-$CD_3OD$] 3.52 (s, 5H), 3.90 (s, 2H), 4.53 (s, 2H), 5.03 (s, 1H), 6.97 (t, 1H, 56Hz), 7.67 (s, 1H) |

Table 7-10

| No. | A | B | X | IR ($\nu$ max $cm^{-1}$) | NMR ($\delta$) |
|---|---|---|---|---|---|
| 7-38 | $CF_2H$ | H | 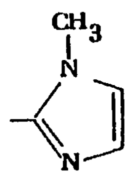 | (Nujol) 1785, 1668 | [$CD_3COCD_3$] 3.50 (s, 3H), 3.80 (s, 2H), 3.92 (s, 3H), 4.10, 4.40 (ABq, 2H, 13Hz), 4.63 (s, 2H), 5.17 (s, 1H), 6.93 (t, 1H, 56Hz), 7.47, 7.62 (ABq, 2H, 1.5Hz) |
| 7-39 | $CF_2H$ | H | 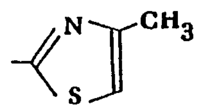 | (Nujol) 3180, 1780, 1704, 1660, 1061 | [DMSO] 2.30 (d, 3H, 1.0Hz), 3.38 (s, 3H), 3.63 (s, 2H), 4.10, 4.30 (ABq, 2H; 14Hz), 4.48 (s, 2H), 5.05 (s, 1H), 7.18 (q, 1H, 1.0Hz), 7.30 (t, 1H, 56Hz) |
| 7-40 | $CF_2H$ | H | 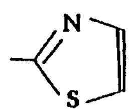 | (Nujol) 1778, 1680, 1060 | [DMSO] 3.40 (s, 3H), 3.65 (s, 2H), 4.28 (bs, 2H), 4.50 (s, 2H), 5.07 (s, 1H), 7.30 (t, 1H, 57Hz), 7.70 (s, 1H) |
| 7-41 | $CF_2H$ | H | 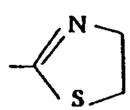 | (Nujol) 1780, 1686 | [DMSO] 3.40 (s, 3H), 3.63 (s, 2H), 4.13 (s, 2H), 4.41 (s, 2H), 7.30 (t, 1H, 56Hz), 9.21 (s, 1H) |
| 7-42 | $CF_2H$ | H | 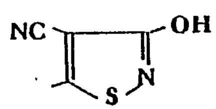 | (Nujol) 3150, 2220, 1780, 1650 | [$CDCl_3$-$CD_3OD$] 3.55 (s, 5H), 4.18 (s, 2H), 4.55 (s, 2H), 5.05 (s, 1H), 6.98 (t, 1H, 56Hz) |

Table 7-11

| No. | A | B | X | IR (ν max $cm^{-1}$) | NMR (δ) |
|---|---|---|---|---|---|
| 7-43 | $CF_2H$ | H | 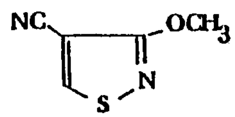 | (Nujol) 3260, 2220, 1780, 1700 | ($CDCl_3-CD_3OD$) 3.55 (s, 5H), 4.08 (s, 3H), 4.25 (s, 2H), 4.57 (s, 2H), 5.12 (s, 1H), 7.05 (t, 1H, 56Hz) |
| 7-44 | $CF_2H$ | H | 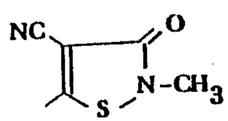 | (Nujol) 3275, 2220, 1795, 1720, 1650 | ($CDCl_3-CD_3OD$) 3.37 (s, 3H), 3.57 (s, 5H), 4.13, 4.33 (ABq, 2H, 14Hz), 4.57 (s, 2H), 5.13 (s, 1H), 7.03 (t, 1H, 56Hz) |
| 7-45 | $CF_2H$ | H | 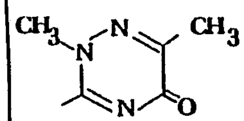 | (Nujol) 3320, 1785, 1705, 1635 | ($CDCl_3-CD_3OD$) 2.27 (s, 3H), 3.53 (s, 3H), 3.56 (s, 2H), 3.77 (s, 3H), 4.25, 4.41 (ABq, 2H, 14Hz), 4.58 (s, 2H), 5.05 (s, 1H), 7.02 (t, 1H, 56Hz) |
| 7-46 | $CF_2H$ | H | 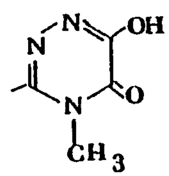 | (Nojol) 3200, 1785, 1710, 1585 | ($CDCl_3-CD_3OD$) 3.47 (s, 3H), 3.55 (s, 3H), 3.58 (s, 2H), 4.10, 4.27 (ABq, 2H, 15Hz), 4.57 (s, 2H), 5.08 (s, 1H), 7.07 (t, 1H, 56Hz) |
| 7-47 | $CF_2H$ | H | 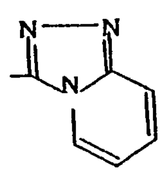 | (Nujol) 3180, 1785, 1683 | (DMSO) 3.52 (s, 3H), 3.67 (s, 2H), 4.00 (s, 2H), 4.57 (s, 2H), 5.00 (s, 1H), 7.32 (t, 1H, 55Hz), 6.63 - 8.63 (m, 4H), 9.33 (b, 1H) |

Table 7-12

| No. | A | B | X | IR ( ν max $cm^{-1}$ ) | NMR ( δ ) |
|---|---|---|---|---|---|
| 7-48 | $CF_2H$ | H | 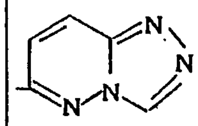 | (Nujol) 1782, 1693 | (DMSO) 3.42 (s, 3H), 3.67 (s, 2H), 4.07, 4.66 (ABq, 2H, 14Hz), 4.60 (s, 2H), 5.13 (s, 1H), 7.28 (t, 1H, 56Hz), 7.40, 8.17 (ABq, 2H, 10Hz), 9.25 (b, 1H), 9.40 (s, 1H) |
| 7-49 | $CF_2H$ | H | 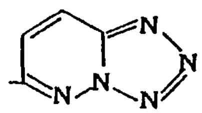 | (Nujol) 3270, 1785, 1700, 1068 | (DMSO) 3.43 (s, 3H), 3.66 (s, 2H), 4.20, 4.53 (ABq, 2H, 14Hz), 4.63 (s, 2H), 5.15 (s, 1H), 7.00 (t, 1H, 55Hz), 7.73, 8.60 (ABq, 2H, 10Hz) |
| 7-50 | $CF_2H$ | H | 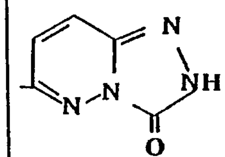 | (Nujol) 1780, 1715, 1685 | (DMSO) 3.37 (s, 3H), 3.65 (s, 2H), 4.30 (bs, 2H), 4.33 (s, 2H), 4.92 (s, 1H), 6.70, 7.46 (ABq, 2H, 9.0Hz), 7.28 (t, 1H, 56Hz) |
| 7-51 | $CF_2H$ | H | 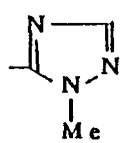 | (KBr) 3280, 1783, 1700<br>Rf=0.14 ( EtOAc : HOAc : 水 = 18 : 1 : 1 | ($CDCl_3$-$CD_3OD$) 3.63 (S, 5H), 3.82 (S, 3H), 4.18 (S, 2H) 4.55 (S, 2H), 5.08 (S, 1H), 6.98 (t, 1H, 56Hz), 7.83 (S, 1H) |

Table 8-1

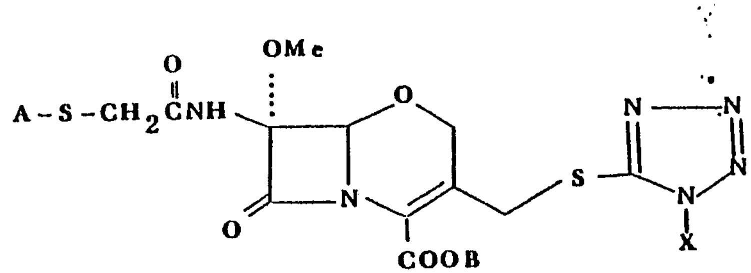

| № | A | B | X | IR ( ν max cm⁻¹ ) | NMR ( δ ) |
|---|---|---|---|---|---|
| 8 - 1 | $CF_2H$ | POM | $-CH_2CH_2N(CH_3)_2$ | | 1.23 (S, 9H), 2.28 (S, 6H), 2.76 (t, 6Hz, 2H), 3.53 (S, 3H), 3.53 (S, 2H), 4.27 (S, 2H), 4.30 (t, 6Hz, 2H), 4.63 (S, 2H), 5.08 (S, 1H), 5.88 (d, 5Hz, 1H), 6.08 (d, 5Hz, 1H), 6.96 (t, 56Hz, 1H), 7.43 (S, 1H) |
| 8 - 2 | $CF_2H$ | POM | $-C_2H_5$ | 3370, 1791, 1754, 1699, 1632 | 1.23 (S, 9H), 1.51 (t, 7Hz), 3.51 (S, 3H), 3.55 (S, 2H), 4.22 (q, 7Hz), 4.30 (S, 2H), 4.63 (brs, 2H), 5.06 (S, 1H), 5.93 (q, 10Hz, 6Hz, 2H), 6.95 (t, 57Hz, 1H), 7.33 (brs, 1H) |
| 8 - 3 | $CF_2H$ | POM | 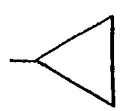 | | 1.2~1.3 (m, 4H), 1.23 (S, 9H), 3.2~3.6 (m, 1H), 3.52 (S, 5H), 4.33 (S, 2H), 4.67 (S, 2H), 5.07 (S, 1H), 5.93 (ABq, 9Hz, 6Hz, 2H), 6.93 (t, 56Hz, 1H), 7.27 (S, 1H) |

Table 8-2

| No. | A | B | X | IR($\nu$max $cm^{-1}$) | NMR ($\delta$) |
|---|---|---|---|---|---|
| 8 - 4 | $CF_2H$ | POM | $-CH_2CH_2OH$ | 3390, 1791, 1753, 1702, 1633 | 1.23 (S, 9H), 3.50 (S, 3H), 3.57 (S, 2H), 4.20 (S, 2H), 3.80～4.50 (m, 4H), 4.58 (S, 2H), 5.03 (S, 1H), 5.83 (ABq, 6Hz, 1H), 6.00 (ABq, 6Hz, 1H(, 6.95 (t, 56Hz, 1H), 7.48 (S, 1H) |
| 8 - 5 | $CF_2H$ | POM | $-CH_2CH_2SCH_3$ | 3390, 1790, 1753, 1704 | 1.25 (S, 9H), 2.12 (S, 3H), 2.98 (t, 6Hz, 2H), 3.55 (S, 3H), 3.60 (S, 2H), 4.32 (S, 2H), 4.45 (t, 6Hz, 2H), 4.65 (S, 2H), 5.08 (S, 1H), 5.86 (ABq, 5Hz, 1H), 6.01 (ABq, 5Hz, 1H), 6.95 (t, 56Hz, 1H), 7.3 (brs, 1H) |
| 8 - 6 | $CF_2H$ | POM | $-CHF_2$ | 3370, 1787, 1745, 1700 | 1.25 (9H, S), 3.53 (3H, S), 3.60 (2H, S), 4.25, 4.57 (2H, ABq, 14Hz), 4.68 (2H, S), 5.10 (1H, S), 5.88, 6.03 (2H, ABq, 6Hz), 6.97 (1H, t, 56Hz), 7.45 (1H, S), 7.58 (1H, t, 58Hz) |
| 8 - 7 | $CF_2H$ | POM | H | 3393, 1792, 1754, 1701, 1632 | 1.23 (S, 9H), 3.52 (bs, 5H), 3.90 (S, 3H), 4.27 (bs, 2H), 4.63 (S, 2H), 5.05 (S, 1H), 5.93 (ABq, 6.0Hz, 2H), 6.93 (t, 56Hz) |

Table 8-3

| No. | A | B | X | IR ($\nu_{max}$ $cm^{-1}$) | NMR ($\delta$) |
|---|---|---|---|---|---|
| 8 - 8 | $CF_2H$ | POM | $-CH_2OCH_3$ | 3360, 1785, 1745, 1690 | 1.23 (S, 9H), 3.40 (S, 3H), 3.57 (S, 3H), 3.63 (S, 2H), 4.33 (S, 2H), 4.68 (S, 2H), 5.12 (S, 1H), 5.60 (S, 2H), 5.92 + 6.07 (ABq, 5Hz), 7.02 (t, 57Hz), 7.58 (bs, 1H) |
| 8 - 9 | $CF_2H$ | POM | $-CH_2CH=CH_2$ | 3370, 1790, 1750, 1695 | 1.23 (S, 9H), 3.57 (S, 3H), 3.65 (S, 2H), 4.35 (S, 2H), 4.70 (S, 2H), 4.8～6.2 (m, 5H), 5.13 (S, 1H), 5.92 + 6.07 (ABq, 5Hz), 6.89 (t, 57Hz), 7.58 (bs, 1H) |
| 8-10 | $CF_2H$ | POM | $-CH_2CH_2OCH_3$ | 3370, 1790, 1757, 1700 | 1.23 (S, 9H), 3.28 (S, 3H), 3.50 (S, 3H), 3.62 (S, 2H), 3.80 (t, 2H, 4Hz), 4.32 (S, 2H), 4.47 (t, 2H, 4Hz), 4.67 (S, 2H), 5.13 (S, 1H), 5.88 + 6.07 (ABq, 5Hz), 7.00 (t, 1H, 54Hz), 7.65 (bs, 1H) |
| 8-11 | $CF_2H$ | POM | $CH_2CN$ | 3380, 2960, 1795, 1755, 1700, 1390, 1115, 1070 | 1.23 (s, 9H), 3.53 (s, 3H), 3.57 (s, 2H), 4.30 (s, 2H), 4.62 (s, 2H), 5.07 (s, 1H), 5.30 (s, 2H), 5.85, 5.98 (ABq, 2H, 6Hz), 6.92 (t, 1H, 56Hz) |

Table 8-4

| No. | A | B | X | IR ($\nu$ max $cm^{-1}$) | NMR ($\delta$) |
|---|---|---|---|---|---|
| 8-12 | $CF_2H$ | POM | $CH_2CH_2CN$ | 3380, 2950, 2240, 1790, 1750, 1700, 1385, 1115, 1070 | 1.25 (s, 9H), 3.08 (t, 2H, 6Hz), 3.55 (s, 3H), 3.60 (s, 2H), 4.30 (s, 2H), 4.57 (t, 2H, 6Hz), 5.88, 6.02 (ABq, 2H, 6Hz), 6.97 (t, 1H, 57Hz), 7.37 (bs, 1H), 4.65 (s, 2H), 5.08 (s, 1H) |
| 8-13 | $CF_2H$ | POM | $CH_2COOCH_3$ | 3375, 2950, 1790, 1755, 1700, 1385, 1115, 1070 | 1.25 (s, 9H), 3.52 (s, 3H), 3.57 (s, 2H), 3.82 (s, 3H), 4.28 (s, 2H), 4.62 (s, 2H), 5.08 (s, 3H), 5.85, 5.98 (ABq, 2H, 6Hz), 6.92 (t, 1H, 56Hz) |
| 8-14 | $CF_2H$ | POM | $CH_2COOC_2H_5$ | 3380, 1790, 1755, 1702, 1386, 1255, 1115, 1068 | 1.42 (s, 9H), 1.47 (t, 3H, 7Hz), 3.55 (s, 5H), 4.28 (s, 2H), 4.28 (q, 2H, 7Hz), 4.53 (s, 2H), 5.07 (s, 2H), 5.10 (s, 1H), 5.87, 6.00 (ABq, 2H, 6Hz), 6.93 (t, 1H, 56Hz), 7.23 (bs, 1H) |
| 8-15 | $CF_2H$ | POM | $CH_2CONH_2$ | 3360, 2960, 1790, 1750, 1730, 1700, 1390, 1115, 1070 | 1.23 (s, 9H), 3.52 (s, 3H), 3.57 (s, 2H), 4.23 (s, 2H), 4.58 (s, 2H), 5.05 (s, 1H), 5.07 (s, 2H), 5.83, 5.97 (ABq, 2H, 6Hz), 6.95 (t, 1H, 56Hz) |

Table 8-5

| No | A | B | X | IR ($\nu_{max}$ $cm^{-1}$) | NMR (δ) |
|---|---|---|---|---|---|
| 3-16 | $CF_2H$ | POM | $CH_2CONHCH_3$ | 3360, 2955, 1790, 1750, 1690, 1390, 1115, 1065 | 1.23 (s, 9H), 2.85 (d, 3H, 5Hz), 3.53 (s, 3H), 3.62 (s, 2H), 4.25 (bs, 2H), 4.63 (bs, 2H), 4.98 (bs, 2H), 5.08 (s, 1H), 5.83, 6.00 (ABq, 2H, 6Hz), 6.93 (t, 1H, 56Hz), 7.48 (bs, 1H) |
| 8-17 | $CF_2H$ | POM | $CH_2CON(CH_3)_2$ | 3375, 2950, 1785, 1750, 1700, 1675, 1390, 1115, 1070 | 1.22 (s, 9H), 2.98 (s, 3H), 3.12 (s, 3H), 3.52 (s, 3H), 3.55 (s, 2H), 4.22 (bs, 2H), 4.62 (bs, 2H), 5.08 (s, 1H), 5.17 (s, 2H), 5.85, 5.98 (ABq, 2H, 6Hz), 6.93 (t, 1H, 56Hz), 7.30 (bs, 1H) |
| 8-18 | $CF_2H$ | POM | $CH_2COCH_3$ | 3375, 1786, 1750, 1700, 1255, 1112, 1066 | 1.23 (s, 9H), 2.30 (s, 3H), 3.50 (s, 3H), 3.53 (s, 2H), 4.22 (s, 2H), 4.59 (s, 2H), 5.05 (s, 1H), 5.17 (s, 2H), 5.87, 5.93 (ABq, 2H, 6Hz), 6.93 (t, 1H, 56Hz), 7.30 (bs, 1H) |

Table 8-6

| No. | A | B | X | IR ($\nu$ max $cm^{-1}$) | NMR ($\delta$) |
|---|---|---|---|---|---|
| 8-20 | $CF_2H$ | POM | $CH_2CH_2CO_2CH_3$ | 3380, 2960, 1790, 1740, 1700, 1385, 1115, 1070 | 1.22 (s, 9H), 3.00 (t, 2H, 7Hz), 3.53 (s, 3H), 3.58 (bs, 2H), 3.70 (s, 3H), 4.30 (bs, 2H), 4.48 (t, 2H, 7Hz), 4.65 (bs, 2H), 5.10 (s, 1H), 5.87, 6.00 (ABq, 2H, 6Hz), 6.97 (t, 1H, 56Hz), 7.35 (bs, 1H) |
| 8-21 | $CF_2H$ | POM | $CH_2CH_2CO_2C_2H_5$ | | 1.22 (s, 9H), 1.23 (t, 3H, 7Hz), 2.98 (t, 2H, 7Hz), 3.52 (s, 3H), 3.57 (bs, 2H), 4.15 (q, 2H, 7Hz), 4.30 (s, 2H), 4.45 (t, 2H, 7Hz), 4.63 (s, 2H), 5.07 (s, 1H), 5.85, 5.98 (ABq, 2H, 5Hz), 6.92 (t, 1H, 56Hz) |
| 8-22 | $CF_2H$ | POM | $CH_2CH_2CONH_2$ | 3480, 3400, 1795, 1758, 1697, 1260, 1120, 1075 | 1.22 (s, 9H), 2.93 (t, 2H, 7.3Hz), 3.50 (s, 3H), 3.59 (s, 2H), 4.23 (s, 2H), 4.47 (t, 2H, 7.3Hz), 4.60 (s, 2H), 5.09 (s, 1H), 5.90, 5.97 (ABq, 2H, 6.5Hz), 6.0 (b, 2H), 7.0 (t, 1H, 56Hz), 7.8 (b, 1H) |
| 8-23 | $CF_2H$ | POM | $CH_2CH_2SO_2NH_2$ | 3350, 3270, 2970, 1790, 1755, 1700, 1395, 1060, 1037 | 1.21 (s, 9H), 3.50 (s, 3H), 3.53 (s, 2H), 3.67 (t, 2H, 6Hz), 4.23 (s, 2H), 4.57 (s, 2H), 4.70 (t, 2H, 6Hz), 5.03 (s, 1H), 5.80, 5.93 (ABq, 2H, 6Hz), 6.93 (t, 1H, 56Hz), 8.30 (s, 1H) |

Table 8-7

| No. | A | B | X | IR ($\nu_{max}$ $cm^{-1}$) | NMR (δ) |
|---|---|---|---|---|---|
| 8-24 | $CF_2H$ | POM | $CH_2CH_2N(CH_3)_2$ HCl | 3400, 3150, 2400 ~ 2600, 1790, 1750, 1695, 1649 | ($d_6$-acetone): 1.20 (s, 9H), 3.03 (s, 6H), 3.45 (s, 3H), 3.77 (s, 2H), 3.85 (t, J=7Hz, 2H), (4.28 (d, J=14Hz, 1H); 4.45 (d, J=14Hz, 1H)), 4.73 (s, 2H), 4.95 (t, J=7Hz, 2H), 5.20 (s, 1H), (5.83 (d, J=6Hz, 1H); 6.03 (d, J=6Hz, 1H), 7.30 (t, J=58Hz, 1H) |

Table 9-1

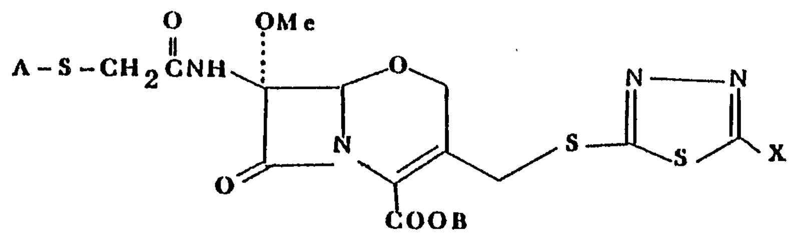

| No. | A | B | X | IR ($\nu$ max cm$^{-1}$) | NMR ($\delta$) |
|---|---|---|---|---|---|
| 9 - 1 | $CF_2H$ | POM | H | 1785, 1740, 1700 | 1.23 (s, 9H), 3.53 (s, 3H), 3.60 (s, 2H), 4.47 (ABq, J=14Hz, 2H), 4.67 (s, 2H), 5.10 (s, 1H), 5.95 (ABq, J=6Hz), 6.95 (t, J=56Hz, 1H) |
| 9 - 2 | $CF_2H$ | POM | $CH_2OCH_2COOH$ | 1785, 1750, 1710 | 1.23 (s, 18H), 3.50 (s, 3H), 3.57 (brs, 2H, $CH_2$), 4.23 (s, 2H), 4.37 (ABq, 2H), 4.60 (brS, 2H, $CH_2$), 4.97 (s, 2H), 5.07 (s, 1H), 5.80 (s, 2H), 5.93 (ABq, J=5Hz, 2H), 6.97 (t, 1H, J=56Hz) |
| 9 - 3 | $CF_2H$ | POM | $CH_3$ | 1785, 1745, 1695 | ($CDCl_3$-$d_4$MeOH) 1.23 (s, 9H), 2.73 (s, 3H), 3.53 (s, 5H), 4.33 (ABq, J=14Hz, 2H), 4.60 (br. s, 2H), 5.03 (s, 1H), 5.93 (ABq, J=6Hz), 6.98 (t, 1H, J=56Hz) |

Table 9-2

| No | A | B | X | IR ($\nu$ max $cm^{-1}$) | NMR ($\delta$) |
|---|---|---|---|---|---|
| 9 - 4 | $CF_2H$ | POM | $CH_2OH$ | 1780, 1740, 1695 | 1.23 (s, 9H), 3.57 (s, 3H), 3.60 (brS, 2H), 4.33 (ABq, J=14Hz, 2H), 4.62 (brS, 2H), 5.00 (brS, 2H), 5.10 (s, 1H), 5.95 (ABq, J=6Hz, 2H), 6.97 (t, 1H; J=56Hz) |
| 9 - 5 | $CF_2H$ | POM | $CH_2CN$ | 1790, 1750, 1700 | 1.23 (s, 9H), 3.53 (s, 3H), 3.58 (s, 2H), 4.22 (s, 2H), 4.38 (ABq, 14Hz; 2H), 5.10 (s, 1H), 5.93 (ABq, 6Hz, 2H), 6.93 (t, 1H, 56Hz) |
| 9 - 6 | $CF_2H$ | POM | $CH_2COOMe$ | 1790, 1740, 1700 | 1.23 (s, 9H), 3.52 (s, 3H), 3.53 (brS, 2H), 3.78 (s, 3H), 4.13 (s, 2H), 4.38 (ABq, 14Hz, 2H), 4.63 (brS, 2H), 5.07 (s, 1H), 5.95 (ABq, 6Hz, 2H), 6.90 (t, 56Hz, 1H) |
| 9 - 7 | $CF_2H$ | POM | $NH_2$ | 1785, 1750, 1695 | 1.23 (s, 9H), 3.57 (s, 3H), 3.67 (br S, 2H), 4.17 (br, 2H), 4.62 (br 2H), 5.08 (s, 1H), 5.92 (ABq, 6Hz, 2H), 7.00 (t, 1H, 56Hz) |
| 9 - 8 | $CF_2H$ | POM | SMe | 1785, 1750, 1700 | ($CDCl_3$-$d_4$MeOH (5 : 1)) 1.23 (s, 9H), 2.77 (s, 3H), 3.50 (s, 5H), 4.33 (ABq, 14Hz, 2H), 4.62 (s, 2H), 5.07 (s, 1H), 5.93 (ABq, 6Hz, 2H), 6.83 (t, 1H, 56Hz) |

Table 9-3

| No. | A | B | X | IR ($\nu$ max $cm^{-1}$) | NMR ($\delta$) |
|---|---|---|---|---|---|
| 9 - 9 | $CF_2H$ | POM | COOEt | 1790, 1745, 1720, 1700 (sh) | 1.25 (s, 9H), 1.43 (t, 7Hz, 3H), 3.57 (s, 3H), 3.63 (br. S, 2H), 4.50 (ABq, 14Hz, 2H), 4.53 (q, 7Hz, 2H), 4.70 (brS, 2H), 5.08 (s, 1H), 5.97 (ABq, 6Hz, 2H), 6.93 (t, 56Hz, 1H) |
| 9 - 10 | $CF_2H$ | POM | $CH_2NHBOC$ | 3450, 3380, 1790, 1755, 1710 | 1.23 (S, 9H), 1.47 (S, 9H), 3.53 (S, 3H), 3.62 (S, 2H), 4.37 (ABq, 2H), 4.63 (brs, 4H), 5.07 (S, 1H), 5.93 (ABq, 6Hz), 6.93 (t, 1H, 56Hz) |
| 9 - 11 | $CF_2H$ | POM | $CH_2NH_2$ | 1790, 1750, 1690 | 1.23 (s, 9H), 3.55 (s, 3H), 3.60 (s, 2H), 4.30 (brs, 2H), 4.43 (brs, 2H), 4.63 (brs, 2H), 5.13 (s, 1H), 5.90 (ABq, J=6Hz, 2H), 6.93 (t, J=56Hz, 1H) |

Table 10-1

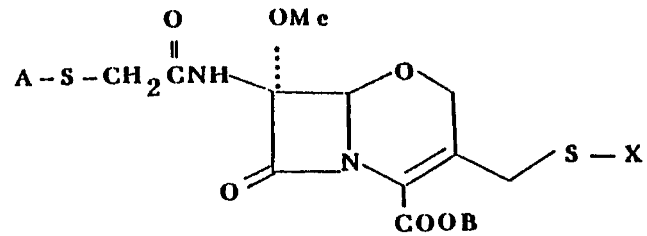

| № | A | B | X | IR ( ν max cm$^{-1}$ ) | NMR ( δ ) |
|---|---|---|---|---|---|
| 10-1 | $CF_2H$ | POM | 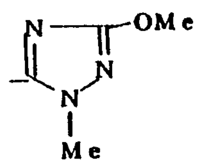 | 3370, 1780, 1745, 1685, 1670, 1630, 1530 | 1.23 ( S , 9H ) , 3.49 ( S , 3H ) , 3.53 ~ 3.59 (brs , 5H) , 3.86 ( S , 3H ) , 4.10 ( brs , 2H ) , 4.57 ( S , 2H ) , 5.05 ( S , 1H ) , 5.90 ( ABq , 5.5Hz , 2H ) , 6.97 ( t , 57Hz , 1H ) |
| 10-2 | $CF_2H$ | POM | 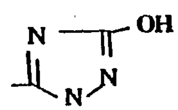 | 1784, 1750, 1700, 1630, 1566 | 3.55 ( S , 3H ) , 3.58 ~ 4.25 ( m , 7H ) , 4.62 ( bs , 2H ) , 5.16 , 5.82 ( ABq , 6Hz , 2H ) , 7.07 ( t , 57Hz , 1H ) , 10.87 ( bs , 1H ) |
| 10-3 | $CF_2H$ | POM | 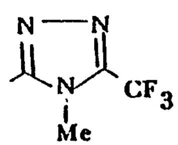 | 3370, 1790, 1756, 1703 | 1.23 ( 9H , S ) , 3.55 ( 3H + 2H , S ) , 3.70 ( 3H , S ) , 4.25 ( 2H , S ) , 4.67 ( 2H , S ) , 5.08 ( 1H , S ) , 5.83 , 5.98 ( 2H , ABq , 6Hz ) , 6.95 ( 1H , t , 55Hz ) |
| 10-4 | $CF_2H$ | POM | 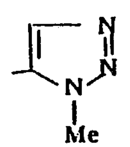 | 3370, 1785, 1745, 1695 | 1.23 ( 9H , S ) , 3.58 ( 3H , S ) , 3.45 ~ 4.00 ( 4H , m ) , 4.15 ( 3H , S ) , 4.58 ( 2H , S ) , 5.17 ( 1H , S ) , 5.70 , 6.03 ( 2H , ABq , 5Hz ) , 4.27 ( 1H , t , 57Hz ) , 7.75 ( 1H , S ) , 8.05 ( 1H , S ) |

Table 10-2

| № | A | B | X | IR ( $\nu_{max}$ $cm^{-1}$ ) | NMR ( $\delta$ ) |
|---|---|---|---|---|---|
| 10-5 | $CF_2H$ | POM | 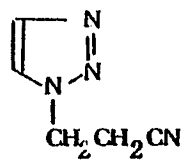 | 3370, 2240, 1790, 1740, 1695 | 1.20 (9H, S), 3.15 (2H, t, 7Hz), 3.55 (3H, S), 3.67 (2H, bs), 3.67~4.90 (6H, m), 5.13 (1H, S), 5.67, 5.82 (2H, ABq, 6Hz), 7.05 (1H, t, 57Hz), 7.77 (1H, S), 7.92 (1H, bs) |
| 10-6 | $CF_2H$ | POM | 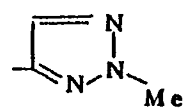 | 3380, 1782, 1745, 1700 | 1.22 (9H, S), 3.53 (3H, S), 3.62 (2H, bs), 3.72, 4.07 (2H, ABq, 12Hz), 4.17 (3H, S), 4.57 (2H, S), 5.08 (1H, S), 5.75, 5.95 (2H, ABq, 6Hz), 7.02 (1H, t, 57Hz), 7.55 (2H, S) |
| 10-7 | $CF_2H$ | POM | 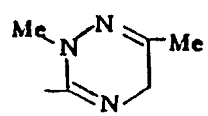 | 3375, 1790, 1750, 1700, 1650<br>Rf=0.423 ( EtOAc ) | 1.23 (9H, S), 2.25 (3H, S), 3.52 (3H, S), 3.60 (2H, S), 3.72 (3H, S), 4.13, 4.47 (2H, q, 14Hz), 4.57 (2H, bs), 5.07 (1H, S), 5.90, 6.03 (2H, q, 6Hz), 6.97 (1H, t, 56Hz) |
| 10-8 | $CF_2H$ | POM | 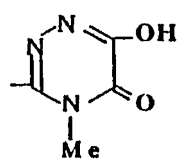 | 3375, 1790, 1750, 1710, 1590<br>Rf=0.364 ( EtOAc ) | 1.23 (9H, S), [3.50 (3H, S) + 3.53 (3H, S)], 3.67 (2H, bs), 4.08 (2H, bs), 4.62 (2H, bs), 5.15 (1H, S), 5.92 (2H, bs), 7.03 (1H, t, 56Hz) |

Table 10-3

| No. | A | B | X | IR ( ν max $cm^{-1}$ ) | NMR ( δ ) |
|---|---|---|---|---|---|
| 10-9 | $CF_2H$ | POM | 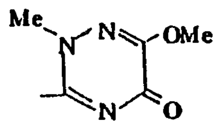 | 3375, 1785, 1695, 1580<br>Rf=0.296 ( EtOAc ) | 1.23 (9H, S), [3.55 (3H, S) + 3.60 (2H, bs)], 3.73 (3H, S), 3.92 (3H, S), 4.17, 4.43 (2H, q, 16Hz), 4.62 (2H, bs), 5.08 (1H, S), 5.92, 6.05 (2H, q, 6Hz), 7.03 (1H, t, 56Hz) |
| 10-10 | $CF_2H$ | POM | 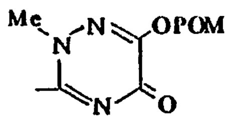 | 3350, 1775, 1735, 1680, 1655<br>Rf=0.322 ( Benzene : EtOAc = 1 : 2 | 1.23 (18H, S), 3.53 (3H, S), 3.58 (2H, S), 3.70 (3H, S), 4.10, 4.43 (2H, q, 11Hz), 4.60 (2H, S), 5.07 (1H, S), 5.87 (2H, S), 5.88, 6.02 (2H, q, J=6Hz), 6.95 (1H, t, 56Hz) |
| 10-11 | $CF_2H$ | POM | 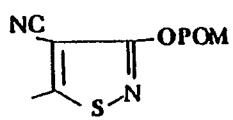 | 3375, 2220, 1790, 1750, 1695<br>Rf=0.319 ( Benzene : EtOAc = 2 : 1 ) | 1.23 (18H, S), [3.55 (3H, S) + 3.60 (2H, S)] 4.17 (2H, S), 4.57 (2H, S), 5.13 (1H, S) 5.83, 5.97 (2H, q, 6Hz), 6.03 (2H, S), 6.97. (1H, t, 56Hz) |
| 10-12 | $CF_2H$ | POM | 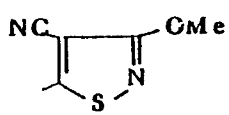 | 3400, 2230, 1800, 1760, 1710<br>Rf=0.293 ( Benzene : EtOAc = 2 : 1 ) | 1.23 (9H, S), [3.55 (3H, S) + 3.58 (2H, S)], 4.07 (3H, S), 4.15 (2H, S), 4.57 (2H, S), 5.13 (1H, S), 5.82, 5.95 (2H, q, 6Hz), 6.95 (1H, t, 56Hz) |
| 10-13 | $CF_2H$ | POM | 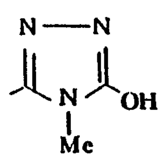 | 3380, 3200, 1790, 1710<br>Rf=0.329 ( EtOAc ) | 1.23 (9H, S), 3.22 (3H, S), 3.50 (3H, S), 3.63 (2H, S), 4.05 (2H, bs), 4.60 (2H, bs), 5.12 (1H, S), 5.83, 5.93 (2H, q, J=6Hz) 6.85 (1H, t, 56Hz) |

Table 10-4

| № | A | B | X | IR ($\nu$ max $cm^{-1}$) | NMR ($\delta$) |
|---|---|---|---|---|---|
| 10-14 | $CF_2H$ | POM | 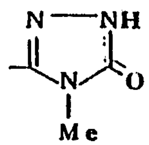 | 3380, 1790, 1750, 1705<br>Rf=0.333 ( EtOAc ) | 1.23 (9H, S), 3.20 (3H, S), 3.43 (3H, S), 3.52 (3H, S), 3.62 (2H, S), 3.98 (2H, S), 4.57 (2H, S), 5.10 (1H, S), 5.82, 5.95 (2H, q, 6Hz), 6.97 (1H, t, 56Hz) |
| 10-15 | $CF_2H$ | POM | 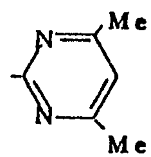 | 1785, 1720 | 1.23 (s, 9H), 2.40 (S, 6H), 3.50 (s, 5H), 4.30 (ABq, 14Hz, 2H), 4.60 (S, 2H), 5.08 (S, 1H), 6.30 (ABq, 6Hz, 2H), 6.73 (S, 1H), 6.97 (t, 56Hz, 1H) |
| 10-16 | $CF_2H$ | POM | 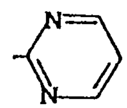 | 1790, 1755, 1705 | ($CDCl_3$-$d_4$MeOH=1 : 1) 1.23 (s, 9H), 3.50 (s, 5H), 4.25 (ABq, 14Hz, 2H), 4.60 (S, 2H), 5.03 (S, 1H), 5.97 (ABq, 6Hz, 2H), 7.05 (t, 6Hz, 1H), 8.50 (d, 6Hz, 2H) |
| 10-17 | $CF_2H$ | POM | 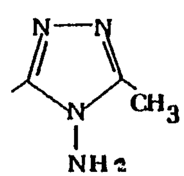 | 3360, 1785, 1750, 1730, 1700 | 1.27 (S, 9H), 2.45 (s, 3H), 3.53 (S, 3H), 3.70 (S, 2H), 4.00 (br, S, 2H), 4.60 (br, S), 5.03 (brS, 2H), 5.07 (S, 1H), 5.87 (ABq, 6Hz, 2H), 7.03 (t, 56Hz, 1H) |

Table 10-5

| № | A | B | X | IR ($\nu$ max $cm^{-1}$) | NMR ($\delta$) |
|---|---|---|---|---|---|
| 10-18 | $CF_2H$ | POM | 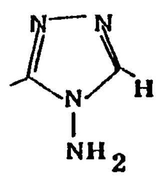 | 1790, 1740, 1700 | ($CDCl_3$-$d_4$MeOH) 1.23 (S, 9H), 3.50 (S, 3H), 3.57 (S, 2H), 4.07 (ABq, 14Hz, 2H), 6.30 (br S, 2H), 5.07 (S, 1H), 5.83 (ABq, 6Hz, 2H), 7.00 (t, 56Hz, 1H), 8.33 (S, 1H) |
| 10-19 | $CF_2H$ | POM | 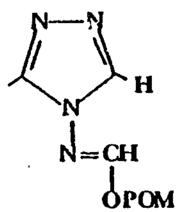 | 3380, 1790, 1740, 1700 | ($CDCl_3$-$d_4$MeOH) 1.23 (s, 18H), 3.47 (S, 5H), 4.13 (brS, 2H), 4.70 (brS, 2H), 5.05 (S, 1H), 5.60 (S, 2H), 5.87 (ABq, 6Hz, 2H), 6.80 (t, 1H, 56Hz), 8.27 (brS, 1H), 8.60 (S, 1H), |
| 10-20 | $CF_2H$ | POM | 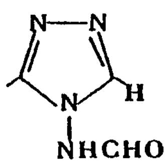 | 1785, 1745, 1700 | 1.27 (S, 9H), 3.50 (S, 3H), 3.60 (brS, 2H), 4.17 (ABq, 2H), 4.60 (brS, 2H), 5.03 (S, 1H), 5.87 (ABq, 2H, 6Hz), 6.93 (t, 56Hz, 1H), 7.83 (brS, 1H), 8.27 (S, 1H) |
| 10-21 | $CF_2H$ | POM | 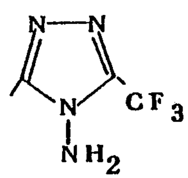 | 3370, 1790, 1750, 1730, 1695 | 1.27 (S, 9H), 3.52 (S, 3H), 3.58 (S, 2H), 4.15 (S, 2H), 4.60 (brS, 2H), 5.17 (brS, 2H), 5.87 (ABq, 6Hz, 2H), 6.92 (t, 1H, 56Hz) |

Table 10-6

| No. | A | B | X | IR ($\nu_{max}\ cm^{-1}$) | NMR ($\delta$) |
|---|---|---|---|---|---|
| 10-22 | $CF_2H$ | POM | 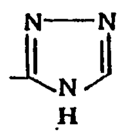 | 3380, 3130, 1790, 1750, 1700<br>Rf=0.16 (Benzene : EtOAc = 1 : 2) | 1.23 (s, 9H), 3.50 (s, 3H), 3.60 (s, 2H), 3.97, 4.27 (ABq, 2H, 14Hz), 4.55 (s, 2H), 5.03 (s, 1H), 5.85, 5.95 (ABq, 2H, 6Hz), 6.93 (t, 1H, 56Hz), 7.70 (s, 1H), 8.20 (s, 1H) |
| 10-23 | $CF_2H$ | POM | 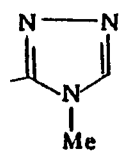 | 3380, 1790, 1700<br>Rf=0.09 (EtOAc) | 1.23 (s, 9H), 3.52 (s, 3H), 3.63 (s, 5H), 4.12 (s, 2H), 4.60 (s, 2H), 5.07 (s, 1H), 5.86, 5.93 (ABq, 2H, 5Hz), 6.97 (t, 1H, 56Hz), 7.98 (s, 1H), 8.20 (s, 1H) |
| 10-24 | $CF_2H$ | POM | 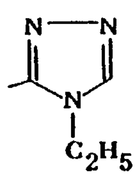 | 3380, 1785, 1750, 1700<br>Rf=0.13 (EtOAc) | 1.23 (s, 9H), 1.45 (t, 3H, 7Hz), 3.52 (s, 3H), 3.65 (s, 2H), 3.98 (q, 2H, 7Hz), 4.18 (s, 2H), 4.63 (s, 2H), 5.08 (s, 1H), 5.85, 5.95 (ABq, 2H, 5Hz), 7.02 (t, 1H, 56Hz), 8.28 (s, 1H) |
| 10-25 | $CF_2H$ | POM | 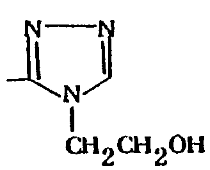 | 3360, 1785, 1750, 1695<br>Rf=0.06 (EtOAc) | ($CDCl_3-CD_3OD$) 1.22 (s, 9H), 3.53 (s, 3H), 3.60 (s, 2H), 3.7~4.3 (m, 6H), 4.57 (s, 2H), 5.10 (s, 1H), 5.80, 5.93 (ABq, 2H, 6Hz), 7.03 (t, 1H, 56Hz), 8.40 (s, 1H) |

Table 10-7

| No. | A | B | X | IR ($\nu$ max $cm^{-1}$) | NMR ($\delta$) |
|---|---|---|---|---|---|
| 10-26 | $CF_2H$ | POM | 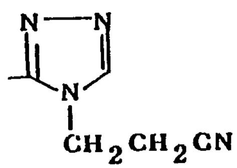 | 3380, 2960, 1790, 1750, 1695, 1495, 1385, 1058, 1035 | 1.22 (s, 9H), 2.95 (t, 2H, 6Hz), 3.53 (s, 3H), 3.62 (s, 2H), 4.03, 4.23 (ABq, 2H, 13Hz), 4.37 (t, 2H, 6Hz), 4.63 (bs, 2H), 5.10 (s, 1H), 5.83, 5.97 (ABq, 2H, 6Hz), 7.03 (t, 1H, 56Hz), 8.00 (bs, 1H), 8.53 (s, 1H) |
| 10-27 | $CF_2H$ | POM | 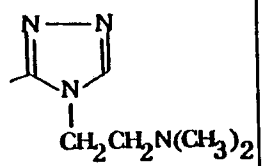 | 3380, 1785, 1750, 1700 Rf=0.15 (EtOAc : $CD_3OD$ = 4 : 1) | 1.22 (s, 9H), 2.25 (s, 6H), 2.58 (t, 2H, 6Hz), 3.52 (s, 3H), 3.62 (s, 2H), 3.97 (t, 2H, 6Hz), 4.13 (s, 2H), 4.60 (s, 2H), 5.05 (s, 1H), 5.80, 5.93 (ABq, 2H, 6Hz), 6.97 (t, 1H, 56Hz), 8.00 (bs, 1H), 8.30 (s, 1H) |
| 10-28 | $CF_2H$ | POM | 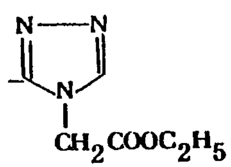 | 3370, 1785, 1750, 1700 Rf=0.22 (EtOAc) | 1.23 (s, 9H), 1.32 (t, 3H, 7Hz), 3.52 (s, 3H), 3.62 (s, 2H), 4.08 (s, 2H), 4.25 (q, 2H, 7Hz), 4.58 (s, 2H), 4.75 (s, 2H), 5.07 (s, 1H), 5.80, 5.93 (ABq, 2H, 6Hz), 6.97 (t, 1H, 56Hz), 7.78 (s, 1H), 8.28 (s, 1H) |
| 10-29 | $CF_2H$ | POM | 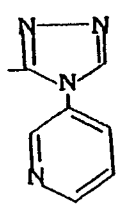 | 3380, 1790, 1755, 1700 Rf=0.11 (EtOAc) | 1.18 (s, 9H), 3.50 (s, 3H), 3.60 (s, 2H), 4.13 (s, 2H), 4.57 (s, 2H), 5.02 (s, 1H), 5.75, 5.88 (ABq, 2H, 5Hz), 6.92 (t, 1H, 56Hz), 7.4~7.9 (m, 3H), 8.32 (s, 1H), 8.5~8.7 (m, 2H) |

Table 10-8

| No. | A | B | X | IR ($\nu$ max $cm^{-1}$) | NMR ($\delta$) |
|---|---|---|---|---|---|
| 10-30 | $CF_2H$ | POM | 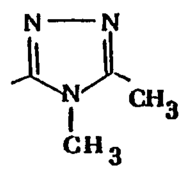 | 3370, 1785, 1750, 1700 | 1.23 (s, 9H), 2.42 (s, 3H), 3.50 (s, 3H), 3.52 (s, 3H), 3.65 (s, 2H), 4.05 (s, 2H), 4.58 (s, 2H), 5.07 (s, 1H), 5.77, 5.90 (ABq, 2H, 6Hz), 7.0 (t, 1H, 56Hz) |
| 10-31 | $CF_2H$ | POM | 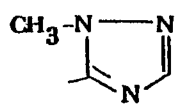 | 3380, 1790, 1755, 1705<br>Rf=0.18 (Benzene : EtOAc = 1 : 1) | 1.25 (s, 9H), 3.55 (s, 5H), 3.78 (s, 3H), 4.13, 4.27 (ABq, 2H, 13Hz), 4.62 (s, 2H), 5.10 (s, 1H), 5.90, 6.00 (ABq, 2H, 5Hz), 6.97 (t, 1H, 56Hz), 7.30 (s, 1H), 7.83 (s, 1H) |
| 10-32 | $CF_2H$ | POM | 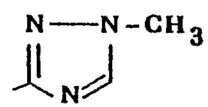 | 3380, 1785, 1750, 1695<br>Rf=0.15 (Benzene : EtOAc = 1 : 2) | 1.23 (s, 9H), 3.53 (s, 5H), 3.87 (s, 3H), 3.92, 4.32 (ABq, 2H, 13Hz), 4.57 (s, 2H), 5.05 (s, 1H), 5.68, 5.78 (ABq, 2H, 5Hz), 6.90 (t, 1H, 56Hz), 7.92 (s, 1H) |
| 10-33 | $CF_2H$ | POM | 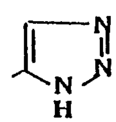 | 3380, 1790, 1750, 1700<br>Rf=0.18 (Benzene : EtOAc = 1 : 1) | 1.23 (s, 9H), 3.53 (s, 3H), 3.60 (s, 2H), 3.88 (s, 2H), 4.53 (s, 2H), 5.06 (s, 1H), 5.75, 5.88 (ABq, 2H, 6Hz), 7.00 (t, 1H, 56Hz), 7.72 (s, 1H) |

Table 10-9

| № | A | B | X | IR ($\nu$ max $cm^{-1}$) | NMR ($\delta$) |
|---|---|---|---|---|---|
| 10-35 | $CF_2H$ | POM | 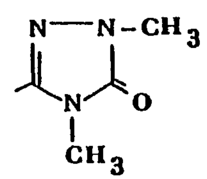 | 3380, 1790, 1750, 1705 | 1.23 (s, 9H), 3.20 (s, 3H), 3.43 (s, 3H), 3.52 (s, 3H), 3.62 (s, 2H), 3.98 (s, 2H), 4.57 (s, 2H), 5.10 (s, 1H), 5.82, 5.95 (ABq, 2H, 6Hz), 6.97 (t, 1H, 56Hz), |
| 10-36 | $CF_2H$ | POM | 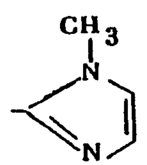 | 3480, 1790, 1752, 1700, 1258, 1113, 1070 Rf=0.30 ( EtOAc ) | 1.23 (s, 9H), 3.50 (s, 3H), 3.59 (s, 2H), 3.63 (s, 3H), 3.90, 4.07 (ABq, 2H, 13Hz), 4.50 (s, 2H), 5.10 (s, 1H), 5.80, 5.83 (ABq, 2H, 7Hz), 6.97 (t, 1H, 56Hz), 6.93 (split, 1H, 1.0Hz), 6.98 (split, 1H, 1.0Hz), 7.4 (bs, 1H) |
| 10-37 | $CF_2H$ | POM | 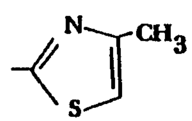 | 3385, 1786, 1754, 1703, 1388, 1257, 1118, 1070 Rf=0.46 ( EtOAc : $CHCl_3$=1 : 1) | 1.23 (s, 9H), 2.36 (d, 3H, 1.0Hz), 3.52 (s, 5H), 4.02, 4.38 (ABq, 2H, 14Hz), 4.53 (s, 2H), 5.03 (s, 1H), 5.85, 5.97 (ABq, 2H, 6.5Hz), 6.75 (q, 1H, 1.0Hz), 6.92 (t, 1H, 56Hz), 7.20 (bs, 1H) |
| 10-38 | $CF_2H$ | POM | 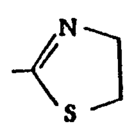 | 3375, 1785, 1750, 1693, 1385, 1255, 1114, 1066 Rf=0.52 ( EtOAc : Benzene = 1 : 1) | 1.23 (s, 9H), 3.40 (t, 2H, 7.5Hz), 3.52 (s, 5H), 3.95, 4.27 (ABq, 2H, 14Hz), 4.15 (t, 2H, 7.5Hz), 4.52 (s, 2H), 5.05 (s, 1H), 5.85, 5.94 (ABq, 2H, 6.0Hz), 6.92 (t, 1H, 56Hz), 7.28 (bs, 1H) |

Table 10-10

| № | A | B | X | IR (ν max $cm^{-1}$) | NMR (δ) |
|---|---|---|---|---|---|
| 10-39 | $CF_2H$ | POM | 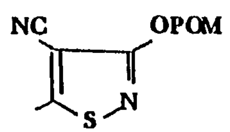 | 3375, 2220, 1790, 1750, 1695 | 1.23 (s, 18H), 3.55 (s, 3H), 3.60 (s, 2H), 4.17 (s, 2H), 4.57 (s, 2H), 5.13 (s, 1H), 5.83, 5.97 (ABq, 2H, 6Hz), 6.03 (s, 2H), 6.97 (t, 1H, 56Hz) |
| 10-40 | $CF_2H$ | POM | 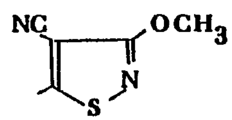 | | 1.23 (s, 9H), 3.55 (s, 3H), 3.58 (s, 2H), 4.07 (s, 3H), 4.15 (s, 2H), 4.57 (s, 2H), 5.13 (s, 1H), 5.82, 5.95 (ABq, 2H, 6Hz), 6.95 (t, 1H, 56Hz) |
| 10-41 | $CF_2H$ | POM | 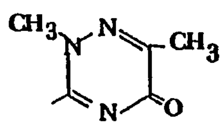 | 3375, 1790, 1750, 1700, 1650 | 1.23 (s, 9H), 2.25 (s, 3H), 3.52 (s, 3H), 3.60 (s, 2H), 3.72 (s, 3H), 4.13, 4.47 (ABq, 2H, 14Hz), 4.57 (s, 2H), 5.07 (s, 1H), 5.90, 6.03 (ABq, 2H, 6Hz), 6.97 (t, 1H, 56Hz) |
| 10-42 | $CF_2H$ | POM | 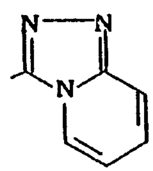 | 3380, 1792, 1755, 1699, 1260, 1115, 1072<br>Rf = 0.28 ( EtOAc ) | 1.20 (s, 9H), 3.53 (s, 3H), 3.65 (s, 2H), 4.07 (s, 2H), 4.63 (s, 2H), 5.10 (s, 1H), 5.65, 5.80 (ABq, 2H, 6Hz), 6.98 (t, 1H, 56Hz), 6.90-8.29 (m, 4H) |

Table 10-11

| No. | A | B | X | IR ($\nu$ max $cm^{-1}$) | NMR ($\delta$) |
|---|---|---|---|---|---|
| 10-43 | $CF_2H$ | POM | 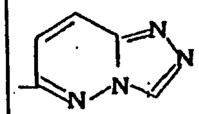 | 3380, 1790, 1752, 1699, 1257, 1111, 1076<br>Rf=0.28 ( EtOAc ) | 1.20 (s, 9H), 3.52 (s, 3H), 3.63 (s, 2H), 4.16, 4.41 (ABq, 2H, 13Hz), 4.60 (s, 2H), 5.10 (s, 1H), 5.90, 6.05 (ABq, 2H, 6Hz), 6.88, 7.97 (ABq, 2H, 9Hz), 6.97 (t, 1H, 56Hz), 7.72 (b, 1H), 8.97 (s, 1H) |
| 10-44 | $CF_2H$ | POM | 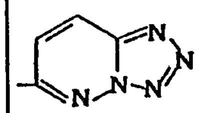 | 3380, 1792, 1756, 1700, 1258, 1115, 1070<br>Rf=0.48 ( EtOAc ) | 1.20 (s, 9H), 3.47 (s, 3H), 3.55 (s, 2H), 4.14, 4.59 (ABq, 2H, 15Hz), 4.63 (s, 2H), 5.06 (s, 1H), 5.89, 6.07 (ABq, 2H, 5.5Hz), 6.90 (t, 1H, 55Hz), 7.25, 8.15 (ABq, 2H, 10Hz), 7.35 (b, 1H) |
| 10-45 | $CF_2H$ | POM | 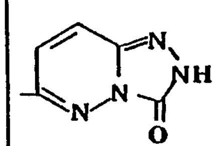 | 3380, 3200, 1788, 1725, 1258, 1115, 1065<br>Rf=0.40 ( EtOAc ) | 1.25 (s, 9H), 3.57 (s, 3H), 3.62 (s, 2H), 4.09, 4.37 (ABq, 2H, 14Hz), 4.69 (s, 2H), 5.10 (s, 1H), 5.93, 6.00 (ABq, 2H, 6.5Hz), 6.75, 7.40 (ABq, 2H, 10Hz), 6.97 (t, 1H, 56Hz), 11.7 (b, 1H) |

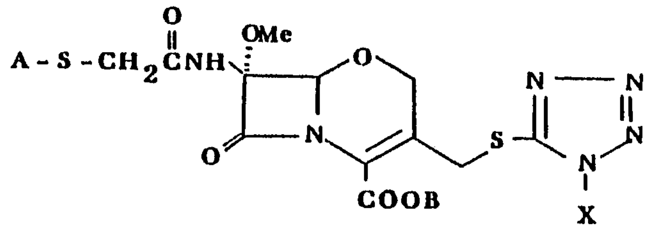

Table 11-1

| No. | A | B | X | IR ($\nu max$ $cm^{-1}$) | NMR ($\delta$) |
|---|---|---|---|---|---|
| 11-1 | $CF_2H$ | $CH_2OCOCH_3$ | Et | 3390、1789、1771、1736、1703、1632 | 1.52 (t, 3H, 7Hz)、2.13 (S, 3H)、2.11 (S, 5H)、4.23 (q, 7Hz)、4.03 (S, 2H)、4.63 (S, 2H)、5.05 (S, 1H)、5.92 (ABq, 9Hz, 6Hz, 2H)、6.95 (t, 57Hz, 1H)、7.37 (S, 1H) |
| 11-2 | $CF_2H$ | $-CH(CH_3)OCOOEt$ | Et | 3395、1795、1766、1704、1636 | |

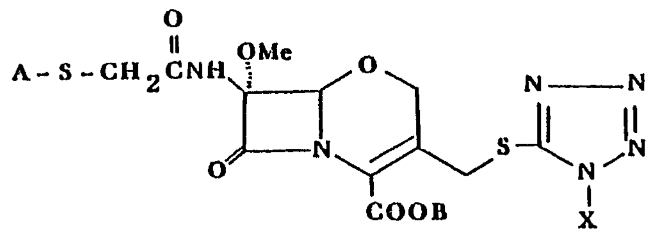

Table 12-1

| № | A | B | X | IR ($\nu$ max $cm^{-1}$) | NMR($\delta$) |
|---|---|---|---|---|---|
| 12-1 | $CF_2H$ | K | $CH_2CH_2COOCH_3$ | (KBr) 3400、1775、1740、1690、1615、1205、1065 Rf= 0.21 ( EtOAc : HOAc ; $H_2O$ = 18:1:1) | ($CD_3OD$) : 3.05 ( t , J=7Hz , 2H)、3.53 (S , 3H)、3.63 (S , 2H)、3.68 (S , 3H)、4.35 (S , 2H)、4.53 (S , 2H)、4.57 ( t , J=7Hz , 2H)、5.07 (S , 1H)、7.13 ( t , J=57Hz , 1H) |
| 12-2 | $CF_2H$ | K | $CH_2CH_2COOC_2H_5$ | (KBr) 3400、1775、1733、1690、1615、1205、1067 Rf =0.60 ( EtOAc : HOAc-$H_2O$=8:1:1 ) | ($CD_3OD$) : 1.20 ( t , J=7Hz , 3H)、3.02 ( t , J=7Hz , 2H)、3.52 (S , 3H)、3.62 (S , 2H)、4.10 (q , J=7Hz , 2H)、4.32 (S , 2H)、4.52 (S , 2H)、4.53 ( t , J=7Hz , 2H)、5.03 (S , 1H)、7.08 ( t , J=56Hz , 1H) |
| 12-3 | $CF_2H$ | Na | $CH_2CH_2SCH_3$ | (Nujol) 3265、1769、1687、1613 | |
| 12-4 | $CF_2H$ | Na | 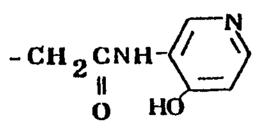 | (KBr) 1690、1770. | ($D_2O$) 3.50 (S , 3H)、3.67 (S , 2H)、4.03 d+4.33d (ABq , 14Hz , 2H)、5.08 (S , 1H)、5.48 (S , 2H)、6.53 (d , 1H , 7Hz)、6.83 ( t , 1H , 56Hz)、7.73 (d, 1H, 7Hz)、8.60 (S , 1H) |

Table 12-2

| No. | A | B | X | IR ($\nu$ max $cm^{-1}$) | NMR ($\delta$) |
|---|---|---|---|---|---|
| 12-5 | $CF_2H$ | Na | 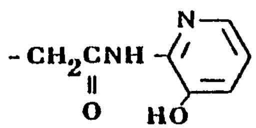 | (KBr) 1680, 1765 | ($D_2O$) 3.47 (S, 3H), 3.67 (S, 2H), 4.07 (d, 1H, 14Hz), 4.20 (d, 1H, 14Hz), 4.47 (S, 2H), 5.07 (S, 1H), 5.42 (S, 2H), 6.98 (t, 1H, 56Hz), 4.10~4.84 (m, 3H) |
| 12-6 | $CF_2H$ | Na | $-CH_2CONHCONH_2$ | | ($D_2O$) 3.50 (S, 3H), 3.67 (S, 2H), 4.03d + 4.30d (ABq, 14Hz, 2H), 4.47 (S, 2H), 5.10 (S, 1H), 5.42 (S, 2H), 7.00 (t, 1H, 56Hz) |
| 12-7 | $CF_2H$ | Na | $-(CH_2)_2NHCO-$ 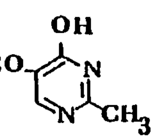 | | ($D_2O$) 2.97 (S, 3H), 4.00 (S, 3H), 4.17 (S, 2H), 5.57 (S, 1H), 7.57 (t, 56.7Hz, 1H), 8.97 (S, 1H) |
| 12-8 | $CF_2H$ | Na | $-CH_2CONHOCH_3$ | | (d6-DMSO) 3.81 (S, 3H), 4.07 (S, 5H), 4.72 (q, 2H, 12Hz), 4.80 (S, 2H), 5.39 (S, 1H), 5.46 (S, 2H), 7.76 (t, 1H, 56Hz) |
| 12-9 | $CF_2H$ | Na | $CH_2NHCONH_2$ | | |
| 12-10 | $CF_2H$ | Na | $-CH_2-$ 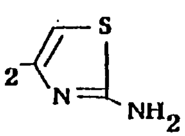 | | ($d_6$-acetone-DMSO) 3.50 (S, 5H), 4.45 (q, 2H, 13.5Hz), 4.50 (S, 2H), 5.02 (S, 1H), 5.33 (S, 2H), 6.05 (S, 1H), 6.85 (S, 2H), 7.30 (t, 1H, 56Hz) |

Table 12-3

| No. | A | B | X | IR (ν max $cm^{-1}$) | NMR (δ) |
|---|---|---|---|---|---|
| 12-11 | $CF_2H$ | Na | $-CH_2-\underset{NOH}{\underset{\|}{C}}-CH_3$ | | ($D_2O$) 1.89 (S, 3H), 3.56 (S, 3H), 3.73 (S, 2H), 4.23 (q, 2H, 12Hz), 4.55 (S, 2H), 5.18 (S, 2H), 5.32 (S, 1H), 7.12 (t, 1H, 56Hz) |
| 12-12 | $CF_2H$ | Na | $-CH_2\underset{O}{\underset{\|}{C}}NH_2$ | (KBr) 1608, 1690, 1769, 3400 | ($D_2O$) 3.55 (S, 3H), 3.73 (S, 2H), 4.23 (q, 2H, J=13.5Hz), 4.55 (S, 2H), 5.18 (S, 1H), 5.32 (S, 2H), 7.12 (t, 1H, J=56Hz) |
| 12-13 | $CF_2H$ | Na | $-CH_2CH_2\underset{O}{\underset{\|}{C}}NH_2$ | | ($D_2O$) 2.98 (t, 2H, 7Hz), 3.55 (S, 3H), 3.73 (S, 2H), 4.5~4.7 (m, 4H), 5.19 (S, 1H), 7.13 (t, 1H, 57Hz) |
| 12-14 | $CF_2H$ | Na | $-CH_2CH_2NHSO_2CH_3$ | | |
| 12-15 | $CF_2H$ | Na | $CH_2CH_2OH$ | | ($D_2O$) 4.00 (S, 3H), 4.16 (S, 2H), 4.47 (t, 2H, 6Hz), 4.56 (q, 2H, 8Hz), 5.01 (S, 2H), 5.01 (t, 2H, 6Hz), 5.63 (S, 1H), 7.58 (t, 1H, 56Hz) |
| 12-16 | $CF_2H$ | Na | 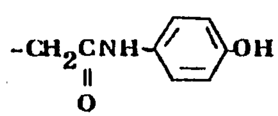 | | ($d_6$-DMSO) 3.39 (S, 3H), 3.67 (S, 2H), 4.31 (q, 2H, 12Hz), 4.42 (S, 2H), 4.98 (S, 1H), 5.29 (S, 2H), 7.34 (t, 1H, 56Hz) 6.68, 6.78, 7.34, 7.44, 各S, 各1H |

Table 13-1

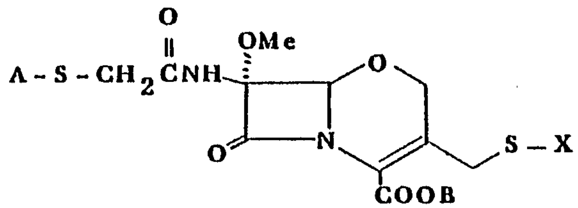

(-S-Tet : -S- 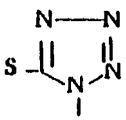 )

| № | A | B | X | IR (ν max cm⁻¹) | NMR (δ) |
|---|---|---|---|---|---|
| 13-1 | $CF_2H$ | $CHPh_2$ | 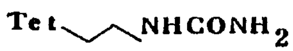 | 3500、3380、1786、1722、1690、1672、1252、1075 | ($CDCl_3$ + $CD_3OD$): 3.51 (b, 2H)、3.56 (S, 2H)、3.58 (S, 3H)、4.15-4.39 (m, 4H)、4.44 ; 4.70 (ABq, 2H, 17Hz)、5.04 (S, 1H)、5.99 (t, 1H, 56Hz)、7.30-7.61 (m, 10H) |
| 13-2 | $CF_2H$ | H | 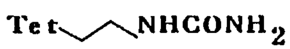 | (Nujol): 3440、3330、1781、1680、1070 | ($d_6$-DMSO+$CD_3OD$): 3.35 (2H, t, 6Hz)、3.38 (S, 3H)、3.63 (S, 2H)、4.22 (S, 2H)、4.39 (t, 2H, 6Hz)、4.54 (S, 2H)、5.06 (S, 1H)、7.28 (t, 1H, 56Hz)、9.23 (S, 1H) |
| 13-3 | $CF_2H$ | $CHPh_2$ | 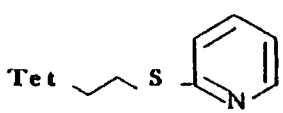 | 3375、1785、1715、1705、1255、1073 | 3.55 (t, 2H, 7Hz)、3.55 (S, 5H)、4.23 (S, 2H)、4.50 (t, 2H, 7Hz)、4.61 (S, 2H)、5.04 (S, 1H)、6.89 (S, 1H)、6.90 (t, 1H, 56Hz)、6.83-7.62 (m, 13H)、8.35 (dd, 1H, 2Hz, 6Hz) |
| 13-4 | $CF_2H$ | H | 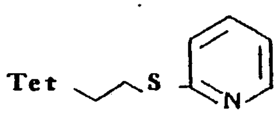 | (Nujol): 3260、1772、1680、1065 | ($CDCl_3$ + $CD_3OD$): 3.54 (S, 5H)、3.63 (t, 2H, 7Hz)、4.27 (S, 2H)、4.55 (S, 2H)、4.61 (t, 2H, 7Hz)、5.05 (S, 1H)、7.01 (t, 1H, 57Hz)、7.0-7.64 (m, 3H)、8.43 (dd, 1H, 2Hz, 6Hz) |

Table 13-2

| No. | A | B | X | IR ($\nu$ max $cm^{-1}$) | NMR ($\delta$) |
|---|---|---|---|---|---|
| 13-5 | $CF_2H$ | $CHPh_2$ | 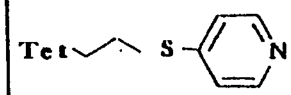 | 3375、1787、1718、1706、1255、1076 | 3.39(t, 2H, 6.5Hz)、3.58(S, 5H)、4.20(S, 2H)、4.28(t, 2H, 6.5Hz)、4.52; 4.73(ABq, 2H, 17Hz)、5.07(S, 1H)、6.89(S, 1H)、6.94(t, 1H, 56Hz)、7.01-7.54(m, 10H)、7.61($A_2B_2$/$A_2$ part, d, 2H, ~6Hz)、8.40($B_2$ part of ($A_2B_2$), d, 2H, ~6Hz) |
| 13-6 | $CF_2H$ | H | 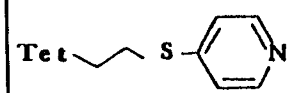 | (Nujol): 3200、1778、1670、1190、1065 | ($CDCl_3$ + $CD_3OD$): 3.45(S, 2H)、3.56(S, 3H)、3.71(t, 2H, 6.5Hz)、3.97; 4.30(ABq, 2H, 13Hz)、4.47(S, 2H)、4.62(t, 2H, 6.5Hz)、5.00(S, 1H)、6.98(t, 1H, 56Hz)、7.70($A_2$ part ($A_2B_2$), d, 2H, ~6.6Hz)、8.50($A_2B_2$/$B_2$ part, d, 2H, ~6.5Hz) |
| 13-7 | $CF_2H$ | $CHPh_2$ | 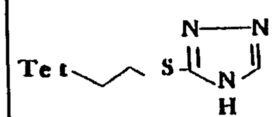 | 3370、1785、1712、1698、1250、1075 | 3.54(t, 2H, 7Hz)、3.57(S, 5H)、4.17(S, 2H)、4.94(S, 2H)、4.51(t, 2H, 7Hz)、5.06(S, 1H)、6.90(S, 1H)、6.91(t, 1H, 56Hz)、7.20-7.62(m, 10H)、7.77(S, 1H)、7.86(S, 1H) |
| 13-8 | $CF_2H$ | H | 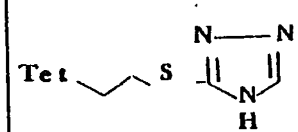 | (Nujol): 3250、1777、1680、1070 | ($CDCl_3$ + $CD_3OD$): 3.58(S, 5H)、3.63(t, 2H, 6.5Hz)、4.13; 4.25(ABq, 2H, 14Hz)、4.54(S, 2H)、4.68(t, 2H, 6.5Hz)、5.07(S, 1H)、7.01(t, 1H, 57Hz)、8.21(S, 1H) |

Table 13-3

| No. | A | B | X | IR (ν max $cm^{-1}$) | NMR(δ) |
|---|---|---|---|---|---|
| 13-9 | $CF_2H$ | $CHPh_2$ | 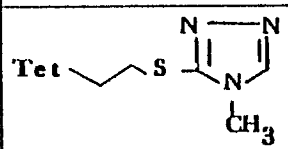 | 3380、1788、1720、1706、1252、1076 | 3.36(S,3H)、3.58(S,3H)、3.63(S,2H)、3.66(t,2H,7Hz)、4.06;4.28(ABq,2H,13Hz)、4.59(t,2H,7Hz)、4.61(S,2H)、5.08(S,1H)、6.86(S,1H)、6.97(t,1H,57Hz)、7.25-7.67(m,10H)、7.86(S,1H)、7.97(S,1H) |
| 13-10 | $CF_2H$ | H | 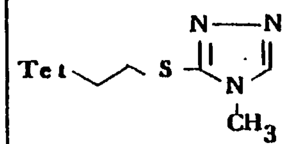 | (Nujol): 1778、1688、1067 | $(CDCl_3+CD_3OD)$: 3.54(S,5H)、3.59(S,3H)、3.73(t,2H,6Hz)、4.15;4.34(ABq,2H,12Hz)、4.57(S,2H)、4.71(t,2H,6Hz)、5.04(S,1H)、7.01(t,1H,56Hz)、8.34(S,1H) |
| 13-11 | $CF_2H$ | $CHPh_2$ | 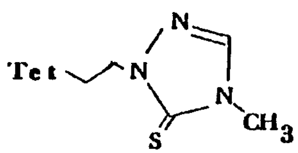 | 3375、1787、1710、1256、1068 | 3.23(S,3H)、3.56(S,2H)、3.59(S,3H)、4.08;4.36(ABq,2H,14Hz)、4.55(S,2H)、4.60(bs,4H)、5.01(S,1H)、6.90(S,1H)、6.90(t,1H,56Hz)、7.25-7.66(m,10H)、7.71(S,1H) |
| 13-12 | $CF_2H$ | H | 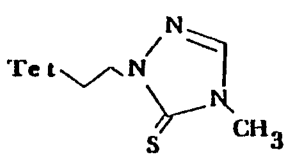 | (Nujol): 3260、1782、1700、1688、1067 | $(CDCl_3+CD_3OD)$: 3.52(S,3H)、3.58(S,5H)、4.15;4.34(ABq,2H,14Hz)、4.55-4.78(m,6H)、5.02(S,1H)、7.00(t,1H,57Hz)、8.08(S,1H) |

Table 13-4

| No. | A | B | X | IR (ν max $cm^{-1}$) | NMR (δ) |
|---|---|---|---|---|---|
| 13-13 | $CF_2H$ | $CHPh_2$ | 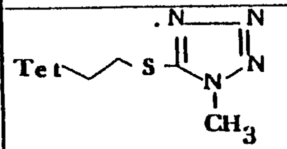 | 3370, 1786, 1715, 1704, 1255, 1078 | 3.56 (S, 5H), 3.73 (t, 2H, 6.5Hz), 3.78 (S, 3H), 4.22 (S, 2H), 4.63 (S, 2H), 4.63 (t, 2H, 6.5Hz), 5.08 (S, 1H), 6.88 (S, 1H), 6.94 (t, 1H, 57Hz), 7.26 - 7.62 (m, 10H) |
| 13-14 | $CF_2H$ | Na | 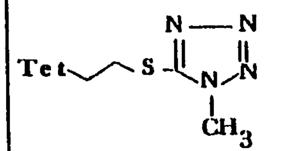 | (Nujol): 3250, 1760, 1680, 1602, 1062 | ($D_2O$ (TMS external standard)): 3.97 (S, 3H), 4.14 (S, 2H), 4.29 (t, 2H, 6.5Hz), 4.38 (S, 3H), 4.57 (S, 2H), 4.94 (S, 2H), 5.20 (t, 2H, 6.5Hz), 5.61 (S, 1H), 7.53 (t, 1H, 56Hz) |
| 13-15 | $CF_2H$ | $CHPh_2$ | Tet-$CH_2$S-$CH_2CONH_2$ | 3470, 3370, 1781, 1710, 1680, 1250, 1071 | ($CDCl_3$ + $CD_3OD$): 3.24 (S, 2H), 3.56 (S, 3H), 3.61 (S, 2H), 4.27 (S, 2H), 4.61 (S, 2H), 5.08 (S, 1H), 5.33 (S, 2H), 6.90 (S, 1H), 6.99 (t, 1H, 57Hz), 7.26 - 7.62 (m, 10H) |
| 13-16 | $CF_2H$ | Na | Tet-$CH_2SCH_2CONH_2$ | (Nujol): 3400, 3160, 1756, 1665, 1592, 1060 | ($D_2O$ (TMS external standard)): 3.91 (S, 2H), 3.97 (S, 3H), 4.15 (S, 2H), 4.57 ; 4.76 (ABq, 2H, 13Hz), 5.00 (S, 2H), 5.61 (S, 1H), 6.02 (S, 2H), 7.54 (t, 1H, 5.7Hz) |
| 13-17 | $CF_2H$ | $CHPh_2$ | 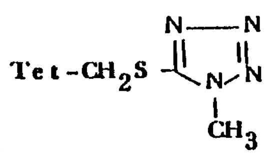 | 3375, 1781, 1710, 1695, 1252, 1070 | 3.53 (S, 5H), 3.77 (S, 3H), 4.18 ; 4.32 (ABq, 2H, 13Hz), 4.58 (S, 2H), 5.07 (S, 1H), 6.48 (S, 2H), 6.87 (S, 1H), 6.90 (t, 1H, 57Hz), 7.25 - 7.60 (m, 10H) |

Table 13-5

| № | A | B | X | IR (ν max $cm^{-1}$) | NMR (δ) |
|---|---|---|---|---|---|
| 13-18 | $CF_2H$ | Na | Tet -$CH_2S$- 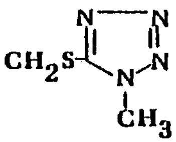 | (Nujol): 1760、1680、1603、1062 | ($D_2O$ (TMS·external standard)): 3.98(S,3H)、4.16(S,2H)、4.32(S,3H)、4.71(S,2H)、4.99(S,2H)、5.63(S,1H)、7.33(S,2H)、7.54(t,1H,57Hz) |
| 13-19 | $CF_2H$ | $CHPh_2$ | Tet-CH-$CONH_2$ 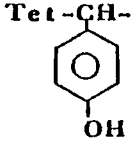 | (KBr): 1782、1690 | ($CDCl_3$+$(CD_3)_2CO$): 3.51(S,3H)、3.61(S,2H)、3.9-4.3(m,2H)、4.4(brs,2H)、5.01(S,1H)、6.24(S,1H)、6.7-7.7(m,15H)、7.07(t,1H,57Hz)、8.26(S,1H)、8.58(S,1H) |
| 13-20 | $CF_2H$ | H | Tet-CH-$CONH_2$ 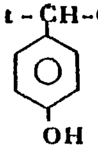 | (KBr): 1782、1690 | ($CDCl_3$+$(CD_3)_2CO$+$CD_3OD$): 3.52(S,3H)、3.64(S,2H)、4.28(S,2H)、4.48(S,2H)、5.03(S,1H)、6.31(S,1H)、6.85($A_2B_2/A_2$ part, d, 2H, ~9Hz)、7.13(t, 1H, 57Hz)、7.37($A_2B_2/B_2$ part, d, 2H, ~9Hz) |
| 13-21 | $CF_2H$ | $CHPh_2$ | Tet 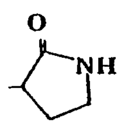 | 3440、1790、1730、1080 | 2.45-2.90(m,2H)、3.30-3.70(m,2H)、3.54(S,3H)、3.56(S,2H)、4.25(S,2H)、4.55(brs,2H)、4.80-5.15(m,1H)、5.07(S,1H)、6.70(S,1H)、6.90(S,1H)、6.90(t,1H,55Hz)、7.23-7.60(m,11H) |

Table 13-6

| No. | A | B | X | IR (ν max $cm^{-1}$) | NMR (δ) |
|---|---|---|---|---|---|
| 13-22 | $CF_2H$ | H | 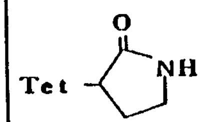 | (KBr): 3360, 1780, 1710, 1065 | ($CD_3OD$): 2.50-3.00(m, 2H), 3.45-3.70(m, 2H), 3.53(S, 3H), 3.60(S, 2H), 4.30(S, 2H), 4.56, 4.60(2xs, 2H), 5.08(S, 1H), 5.46; 5.49(2xt, 1H), 7.12(t, 1H, 57Hz) |
| 13-23 | $CF_2H$ | $CHPh_2$ | 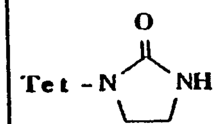 | | 3.54(S, 3H), 3.56(S, 2H), 3.56(t, 2H, 6Hz), 3.88(t, 2H, 6Hz), 4.26(S, 2H), 4.61(S, 2H), 5.06(S, 1H), 6.02(S, 1H), 6.90(t, 1H, 57Hz), 6.92(S, 1H), 7.23-7.60(m, 11H) |
| 13-24 | $CF_2H$ | H | 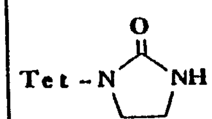 | (KBr): 3360, 1780, 1740, 1695, 1065 | ($CDCl_3$ + $CD_3OD$): 3.53(S, 3H), 3.57(S, 2H), 3.65(t, 2H, 7Hz), 4.00(t, 2H, 6Hz), 4.35(S, 2H), 4.63(S, 2H), 5.06(S, 1H), 7.05(t, 1H, 57Hz) |
| 13-25 | $CF_2H$ | $CHPh_2$ | 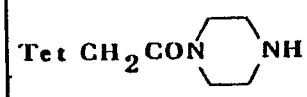 | 3370, 1785, 1695, 1665 | 2.74-3.06(m, 4H), 3.30-3.70(m, 4H), 3.41(S, 2H), 3.54(S, 3H), 4.20(brs, 2H), 4.50; 4.66(ABq, 2H, 21Hz), 5.04 (S, 1H), 5.07(brs, 2H), 6.91(S, 1H), 6.91(t, 1H, 57Hz), 7.2-7.6(m, 10H) |
| 13-26 | $CF_2H$ | H | 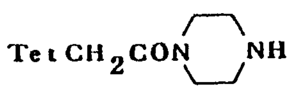 | (Nujol): 3270, 1770, 1660 | |

Table 13-7

| No. | A | B | X | IR ($\nu$ max $cm^{-1}$) | NMR ($\delta$) |
|---|---|---|---|---|---|
| 13-27 | $CF_2H$ | $CHPh_2$ | 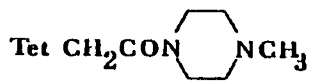 | 3360、1785、1700、1665 | 2.26 (S, 3H)、2.19-2.53 (m, 4H)、3.27-3.70 (m, 6H)、3.52 (S, 3H)、4.19 (brs, 2H)、4.50; 4.64 (ABq, 2H, 18Hz)、5.03 (S, 1H)、5.04 (S, 2H)、6.87 (S, 1H)、6.94 (t, 1H, 57Hz)、7.2-7.6 (m, 10H) |
| 13-28 | $CF_2H$ | H | 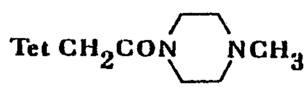 | (Nujol): 3200、1775、1665、1600 | ($D_2O$ (TMS external standard)): 3.46 (S, 3H)、3.60-4.80 (m, 8H)、3.98 (S, 3H)、4.16 (brs, 2H)、4.56; 4.74 (ABq, 2H, 15Hz)、4.93 (brs, 2H)、5.61 (S, 1H)、6.07 (brs, 2H)、7.57 (t, 1H, 57Hz) |
| 13-29 | $CF_2H$ | $CHPh_2$ | 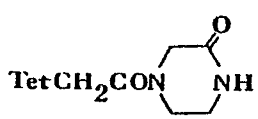 | 3390、1785、1705、1680 | 3.30 (brs, 2H)、3.53 (brs, 7H), 4.10 (brs, 4H)、4.54 (brs, 2H)、5.07 (brs, 3H)、6.86 (S, 1H)、6.94 (t, 1H, 57Hz)、7.2-7.6 (m, 10H) |
| 13-30 | $CF_2H$ | H | 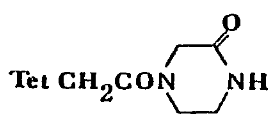 | (Nujol): 3260、1780、1660 | (d6-DMSO): 3.10-3.47 (m, 2H)、3.39 (S, 3H)、3.65 (brs, 2H)、3.53-3.85 (m, 2H)、3.94; 4.18 (2xs, 2H)、4.23 (brs, 2H)、4.53 (brs, 2H)、5.07 (S, 1H)、5.55; 5.58 (2xs, 2H)、7.32 (t, 1H, 57Hz) |

Table 13-8

| No. | A | B | X | IR (ν max $cm^{-1}$) | NMR (δ) |
|---|---|---|---|---|---|
| 13-31 | $CF_2H$ | $CHPh_2$ | 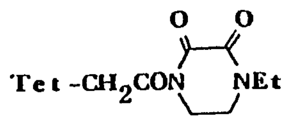 | 3370, 1785, 1715, 1690 | 1.16 (t, 3H, 7Hz), 3.47 (brs, 2H), 3.54 (S, 3H), 3.62 (q, 2H, 7Hz), 3.3 - 3.7 (m, 2H), 3.8 - 4.2 (m, 2H), 4.14 (S, 2H), 4.54 (brs, 2H), 5.07 (S, 1H), 5.60 (brs, 2H), 6.85 (S, 1H), 6.97 (t, 1H, 57Hz), 7.2 - 7.6 (m, 10H) |
| 13-32 | $CF_2H$ | H | 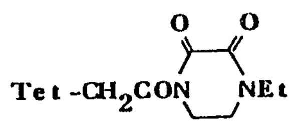 | (Nujol): 3260, 1780, 1710, 1675 | ($D_2O$+$NaHCO_3$ (TMS external standard)): 1.50 - 1.80 (m, 3H), 3.7 - 4.3 (m, 6H), 3.99 (S, 3H), 4.17 (brs, 2H), 4.50 - 4.9 (m, 2H), 4.99 (brs, 2H), 5.63 (S, 1H), 7.57 (t, 1H, 57Hz) |
| 13-33 | $CF_2H$ | $CHPh_2$ | 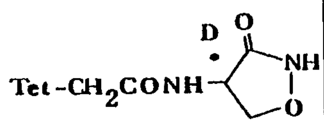 | 3270, 1785, 1710 | ($CDCl_3$ + d6-Acetone): 3.54 (S, 3H), 3.64 (brs, 2H), 3.93 - 4.10 (m, 1H), 4.21 (brs, 1H), 4.50 - 4.95 (m, 2H), 4.58 (brs, 2H), 5.08 (S, 1H), 5.11 (S, 2H), 6.87 (S, 1H), 7.05 (t, 1H, 57Hz), 7.2 - 7.6 (m, 10H) |
| 13-34 | $CF_2H$ | H | 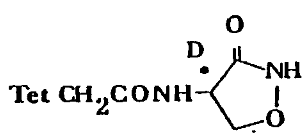 | (Nujol): 3260, 1780, 1685 | (d6-Acetone): 3.50 (S, 3H), 3.73 (brs, 2H), 4.02 - 4.20 (m, 1H), 4.30 (brs, 2H), 4.63 (brs, 2H), 4.60 - 5.00 (m, 2H), 5.12 (S, 1H), 5.30 (S, 2H), 7.25 (t, 1H, 57Hz) |

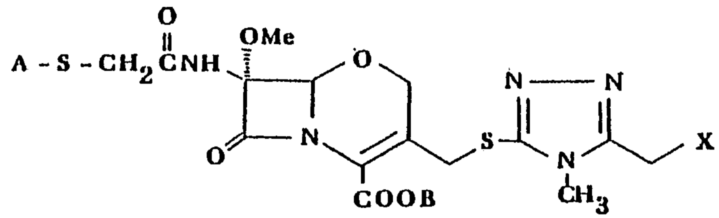

Table 14-1

| No. | A | B | X | IR ($\nu$ max $cm^{-1}$) | NMR ($\delta$) |
|---|---|---|---|---|---|
| 14-1 | $CF_2H$ | $CHPh_2$ | 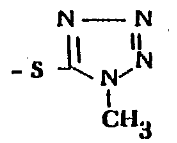 | 3380、1790、1710、1080 | 3.55(S, 6H)、3.60(S, 2H)、3.86(S, 3H)、3.99; 4.19(ABq, 2H, 14Hz)、4.53(S, 2H)、4.46; 4.66(ABq, 2H, 18Hz)、5.06(S, 1H)、6.85(S, 1H)、6.96(t, 1H, 57Hz)、7.25-7.60(m, 11H) |
| 14-2 | $CF_2H$ | H | 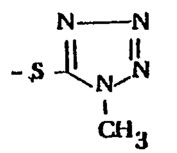 | (KBr): 3400、1780、1695、1065 | ($CD_3OD$): 3.52(S, 3H)、3.61(S, 2H)、3.75(S, 3H)、3.98(S, 3H)、4.05(S, 2H)、4.56(S, 2H)、4.69(S, 2H)、5.06(S, 1H)、7.12(t, 1H, 57Hz) |
| 14-3 | $CF_2H$ | $CHPh_2$ | 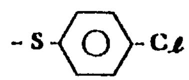 | 3380、1790、1715、1075 | 3.46(S, 3H)、3.55(S, 3H)、3.57(S, 2H)、4.05(S, 2H)、4.12(brs, 2H)、4.60(brs, 2H)、5.05(S, 1H)、6.85(S, 1H)、6.93(t, 1H, 57Hz)、7.20-7.60(m, 15H) |
| 14-4 | $CF_2H$ | H | 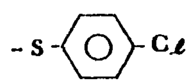 | (KBr): 3430、1780、1690、1067 | ($CD_3OD$): 3.52(S, 3H)、3.60(S, 2H)、3.70(S, 3H)、4.05(S, 2H)、4.28(S, 2H)、4.53(S, 2H)、5.05(S, 1H)、7.12(t, 1H, 57Hz)、7.33(S, 4H) |

Table 14-2

| No. | A | B | X | IR ($\nu$ max $cm^{-1}$) | NMR ($\delta$) |
|---|---|---|---|---|---|
| 14-5 | $CF_2H$ | $CHPh_2$ | -S- 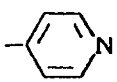 | 3380、1790、1710、1080 | 3.48(S, 3H)、3.55(S, 3H)、3.60(S, 2H)、4.03; 4.16(ABq, 2H, 13Hz)、4.18(S, 2H)、4.50; 4.66 (ABq, 2H, 19Hz)、5.04(S, 1H)、6.83(S, 1H)、6.95(t, 1H, 57Hz)、7.20-7.60(m, 13H)、8.42 (d, 2H, 5Hz) |
| 14-6 | $CF_2H$ | H | -S- 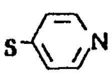 $\cdot CF_3COOH$ | (KBr): 3420、1780、1685、1625、1125、1065 | ($CD_3OD$): 3.50(S, 3H)、3.62(S, 2H)、3.75(S, 3H)、3.93; 4.10(ABq, 2H, 13Hz)、4.57(S, 2H)、4.66(S, 2H)、5.01(S, 1H)、7.12(t, 1H, 57Hz)、7.72 ($A_2B_2/A_2$ part, d, 2H, ~6Hz)、8.44($A_2B_2/B_2$ part, d, 2H, ~6Hz) |

**Claims**

1. A 7β-(fluorinated methylthioacetamido)-7α-methoxy-3-(nucleophilic group-substituted methyl)-1-dethia-1-oxa-3-cephem-4-carboxylic acid or its pharmaceutically acceptable salts and esters represented by the following formula

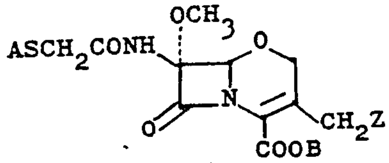

(wherein

A is monofluoromethyl or difluoromethyl and

B is hydrogen or a metal forming a physiologically acceptable ion or a pharmaceutically acceptable carboxy protecting ester forming group having 1 to 20 carbon atoms; and

Z is an halogen or an acyloxy or heterocyclyl thio group having up to 15 carbon atoms, wherein the heterocyclic part of the heterocyclyl thio group is a mono or di-cyclic group) provided Z is not 1-(optionally protected hydroxyalkyl)-1H-tetrazol-5-ylthio.

2. A compound as claimed in Claim 1 wherein A is difluoromethyl.

3. A compound as claimed in Claim 1 wherein B is hydrogen or a pharmaceutically acceptable salt or ester forming group.

4. A compound as claimed in Claim 1 wherein B is a carboxy protecting ester forming group.

5. A compound as claimed in Claim 1 wherein Z is alkanoyloxy, carbamoyloxy, or halogen.

6. A compound as claimed in Claim 1 wherein Z is heterocyclyl thio.

7. A compound as claimed in Claim 6 wherein the heterocyclic part has an optionally substituted or hydrogenated heterocyclic system selected from the member consisting of imidazole, isothiazole, pyrazolotetrazole, pyrazolotriazole, pyridotriazole, pyrimidine, tetrazole, thiadiazone, thiazole, triazine, and triazole.

8. A compound as claimed in Claim 7 wherein the optional substituent on the heterocyclic part is selected from the group consisting of acylamino, alkoxy, alkylthio, amino, carboxy, or protected carboxy, cyano, hydroxy, oxo, and pyridyl, and the heterocyclic part of the heterocyclyl thio group has up to 15 carbon atoms including the substituent.

9. A compound as claimed in Claim 7 wherein the optional substituent on the heterocycle is straight, branched, cyclic, or unsaturated 1 to 4C alkyl optionally substituted by one to three 1 to 12C groups selected from the member consisting of acylamino, acyloxy, alkoxy, alkoxycarbamoyl, alkylcarbamoyl, alkylthio, aroylamino, aryl, arylcarbamoyl, arylthio, carbamoyl, carbamoylalkylthio, carboxy, protected carboxy, carboxyalkoxy, protected carboxyalkoxy, cyano, dialkylamino, halo, N-heterocyclic carbamoyl, heterocyclic, heterocyclyl thio, hydroxy, hydroxyimino, oxo, sulfamoyl, sulfo, ureido, and ureidocarbonyl.

10. A compound as claimed in Claim 1 wherein B is hydrogen, acetoxymethyl, benzyl, t-butyl, calcium, methyldioxolenylmethyl, diphenylmethyl, ethoxycarbonyloxyethyl, indanyl, magnesium, p-methoxybenzyl, p-nitrobenzyl, phenyl, phthalidyl, pivaloyloxymethyl, potassium, sodium, 2,2,2-trichloroethyl, allyl, and xylyl.

11. A compound as claimed in Claim 2 wherein B is hydrogen, acetoxymethyl, alkali metal, diphenylmethyl, ethoxycarbonyloxyethyl, or pivaloyloxymethyl.

12. A compound as claimed in Claim 11 wherein Z is tetrazolylthio, thiadiazolylthio, triazinylthio, or triazolylthio each substituted by one or two of 1 to 3C alkyl.

13. A compound as claimed in Claim 11 wherein Z is 1-substituted 1H-tetrazol-5-yl.

14. A compound as claimed in Claim 13 wherein the substituent is methyl or vinyl.

15. A compound as claimed in Claim 13 wherein the substituent is carbamoylmethyl or 2-carbamoylethyl.

16. A compound as claimed in Claim 13 wherein the substituent is cyanomethyl or cyanoethyl.

17. A compound as claimed in Claim 13 wherein the substituent is 2-oxoimidazolidin-1-yl.

18. A compound as claimed in Claim 13 wherein the substituent is 2-dimethylaminoethyl.

19. A compound as claimed in Claim 13 wherein the substituent is chloromethyl or difluoromethyl.

20. A compound as claimed in Claim 13 wherein the substituent is 2-sulfamoylethyl.

21. A compound as claimed in Claim 13 wherein the substituent is 2-methanesulfonylaminoethyl.

22. A compound as claimed in Claim 11 wherein Z is acetoxy, carbamoyloxy, N-protected carbamoyloxy, or halo.

23. A process for preparing a compound as claimed in Claim 1 which comprises amidating 7β-amino-7α-methoxy-3-(Z-substituted methyl)-1-dethia-1-oxa-3-cephem-4-carboxylic acid derivative represented by the following formula:

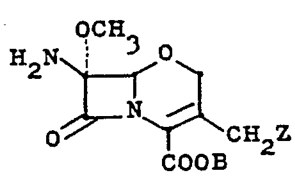

(wherein B and Z are as defined in Claim 1)
or its reactive derivative with (fluorinated methylthio)acetic acid represented by the formula: $ASCH_2COOH$ (wherein A is as defined in Claim 1) or its reactive derivative.

24. A process for preparing a compound as claimed in Claim 1 which comprises substituting 7β-(fluorinated methylthio)acetamido-7α-methoxy-3-halomethyl-1-dethia-1-oxa-3-cephem-4-carboxylic acid derivative represented by the following formula:

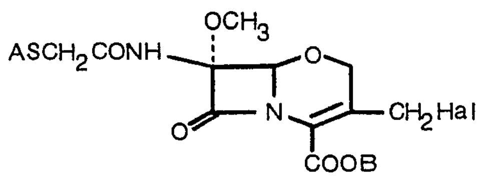

(wherein A and B are as defined in Claim 1 and Hal is halogen)
with a heterocyclic thiol represented by the following formula:

HZ

(wherein Z is a heterocyclic thio as defined in Claim 6)
or its reactive derivative.

25. A process for preparing a compound as claimed in Claim 1 which comprises methoxylating 7ξ-(fluorinated methyl)thioacetamido-3-(Z-substituted methyl)-1-dethia-1-oxa-3-cephem-4-carboxylic acid derivative represented by the following formula:

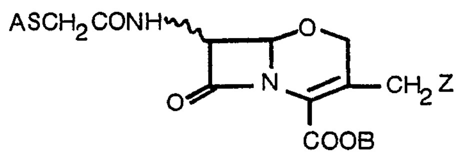

(wherein A, B, and Z are as defined in Claim 1)
with an N-halogenating reagent, hydrogen halide scavenger, and methanol, successively.

26. A process for preparing a salt as claimed in Claim 1 which comprises neutralizing a 7β-(fluorinated methylthio)acetamido-7α-methoxy-3-(Z-substituted methyl)-1-dethia-1-oxa-3-cephem-4-carboxylic acid derivative represented by the following formula:

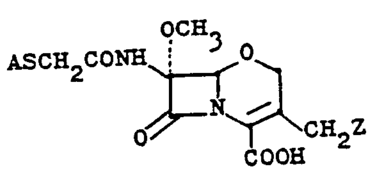

(wherein A and Z are as defined in Claim 1)
or its reactive derivative with a base.

27. A process for preparing a carboxylic acid as claimed in Claim 1 which comprises deesterifying a 7β-(fluorinated methylthio)acetamido-7α-methoxy-3-(Z-substituted methyl)-1-dethia-1-oxa-3-cephem-4-carboxylic acid ester represented by the following formula:

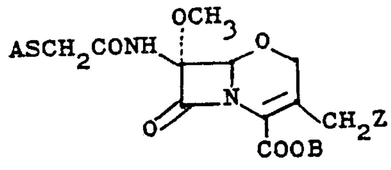

(wherein A and Z are as defined in Claim 1 and B is an ester forming group).

28. An antibacterial composition comprising an effective amount of the compound as claimed in Claim 1 and conventional carrier.

29. A method for combating bacteria in vitro which comprises bringing an effective amount of the compound as claimed in Claim 1 to contact with the bacteria.

**Patentansprüche**

1. 7β-(fluoriertes Methylthioacetamido)-7α-methoxy-3-(nucleophilgruppen-substituiertes Methyl)-1-dethia-1-oxa-3-cephem-4-carbonsäure oder deren pharmazeutisch annehmbaren Salze und Ester mit der folgenden Formel

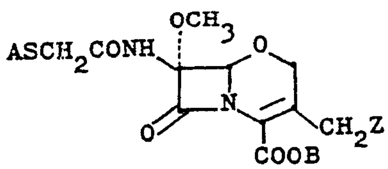

(worin

A Monofluormethyl oder Difluormethyl und

B Wasserstoff oder ein Metall, welches ein physiologisch annehmbares Ion bildet, oder eine pharmazeutisch annehmbare carboxyschützende esterbildende Gruppe mit 1 bis 20 Kohlenstoffatomen bildet, und

Z Wasserstoff oder Acyloxy oder einen heterocyclischen Thiorest mit bis zu 15 Kohlenstoffatomen bedeuten, worin der heterocyclische Teil des heterocyclischen Thiorestes ein mono- oder dicyclischer Rest ist) vorausgesetzt Z ist nicht 1-(gegebenenfalls geschütztes Hydroxyalkyl)-1H-tetrazol-5-ylthio.

2. Verbindung nach Anspruch 1, worin A Difluoromethyl ist.

3. Verbindung nach Anspruch 1, worin B Wasserstoff oder ein pharmazeutisch annehmbares Salz oder esterbildender Rest ist.

4. Verbindung nach Anspruch 1, worin B ein carboxyschützender esterbildender Rest ist.

5. Verbindung nach Anspruch 1, worin Z Alkanoyloxy, Carbamoyloxy oder Halogen ist.

6. Verbindung nach Anspruch 1, worin Z heterocyclisches Thio ist.

7. Verbindung nach Anspruch 6, worin der heterocyclische Teil ein gegebenenfalls substituiertes oder hydriertes heterocyclisches System ausgewählt aus den Bestandteilen bestehend aus Imidazol, Isothiazol, Pyrazolotetrazol, Pyrazolotriazol, Pyridotriazol, Pyrimidin, Tetrazol, Thiadiazol, Thiazol, Triazin and Triazol hat.

8. Verbindung nach Anspruch 7, worin der wahlweise Substituent an dem heterocyclischen Teil aus der Gruppe bestehend aus Acylamino, Alkoxy, Alkylthio, Amino, Carboxy oder geschütztes Carboxy, Cyano, Hydroxy, Oxo und Pyridyl ausgewählt ist, und der heterocyclische Teil des heterocyclischen Thiorestes bis zu 15 Kohlenstoffatome einschließlich des Substituenten hat.

9. Verbindung nach Anspruch 7, worin der wahlweise Substituent an dem Heterozyklus ein gerader, verzweigter, cyclischer oder ungesättigter 1 bis 4C Alkylrest ist, welcher wahlweise mit einem bis drei 1 bis 12C-Resten ausgewählt aus den Bestandteilen bestehend aus Acylamino, Acyloxy, Alkoxy, Alkoxycarbamoyl, Alkylcarbamoyl, Alkylthio, Aroylamino, Aryl, Arylcarbamoyl, Arylthio, Carbamoyl, Carbamoylalkylthio, Carboxy, geschütztes Carboxy, Carboxyalkoxy, geschütztes Carboxyalkoxy, Cyano, Dialkylamino, Halo, N-heterocyclisches Carbamoyl, Heterozyklus, Heterocyclylthio, Hydroxy, Hydroxyimino, Oxo, Sulfamoyl, Sulfo, Ureido und Ureidocarbonyl ist.

10. Verbindung nach Anspruch 1, worin B Wasserstoff, Acetoxymethyl, Benzyl, t-Butyl, Calcium, Methyldioxolenylmethyl, Diphenylmethyl, Ethoxycarbonyloxyethyl, Indanyl, Magnesium, p-Methoxybenzyl, p-Nitrobenzyl, Phenyl, Phthalidyl, Pivaloyloxymethyl, Kalium, Natrium, 2,2,2-Trichlorethyl, Allyl und Xylyl ist.

11. Verbindung nach Anspruch 2, worin B Wasserstoff, Acetoxymethyl, Alkalimetall, Diphenylmethyl, Ethoxycarbonyloxyethyl oder Pivaloyloxymethyl ist.

12. Verbindung nach Anspruch 11, worin Z ein durch ein oder zwei von 1 bis 3C-Alkylresten substituiertes Tetrazolylthio, Thiadiazolylthio, Triazinylthio oder Triazolylthio ist.

13. Verbindung nach Anspruch 11, worin Z 1-substituiertes 1H-Tetrazol-5-yl ist.

14. Verbindung nach Anspruch 13, worin der Substituent Methyl oder Vinyl ist.

15. Verbindung nach Anspruch 13, worin der Substituent Carbamoylmethyl oder 2-Carbamoylethyl ist.

16. Verbindung nach Anspruch 13, worin der Substituent Cyanomethyl oder Cyanoethyl ist.

17. Verbindung nach Anspruch 13, worin der Substituent 2-Oxoimidazolidin-1-yl ist.

18. Verbindung nach Anspruch 13, worin der Substituent 2-Dimethylaminoethyl ist.

19. Verbindung nach Anspruch 13, worin der Substituent Chlormethyl oder Difluormethyl ist.

20. Verbindung nach Anspruch 13, worin der Substituent 2-Sulfamoylethyl ist.

21. Verbindung nach Anspruch 13, worin der Substituent 2-Methansulfonylaminoethyl ist.

22. Verbindung nach Anspruch 11, worin Z Acetoxy, Carbamoyloxy, N-geschütztes Carbamoyloxy oder Halo ist.

23. Verfahren zum Herstellen einer Verbindung nach Anspruch 1, welches das Amidieren eines 7β-

Amino-7α-methoxy-3-(Z-substituiertes Methyl)-1-dethia-1-oxa-3-cephem-4-carbonsäurederivats der folgenden Formel

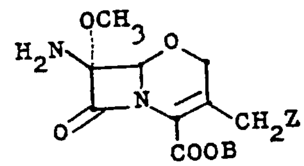

(worin B und Z wie in Anspruch 1 definiert sind) oder seinem reaktiven Derivat mit fluorierter Methylessigsäure mit der Formel $ASCH_2COOH$ (worin A wie in Anspruch 1 definiert ist) oder seinem reaktiven Derivat umfaßt.

24. Verfahren zum Herstellen einer Verbindung nach Anspruch 1, welches das Substituierten eines 7β-(fluorierten Methylthio)acetamido-7α-methoxy-3-halomethyl-1-dethia-1-oxa-3-cephem-4-carbonsäurederivats mit der folgenden Formel

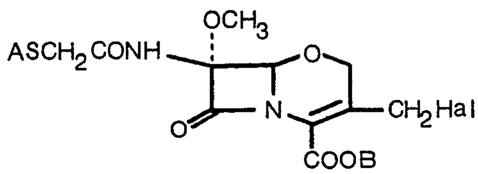

(worin A und B wie in Anspruch 1 definiert sind und Hal Halogen ist) mit einem heterocyclischen Thiol mit der folgenden Formel

HZ

(worin Z ein heterocyclisches Thio wie in Anspruch 6 definiert ist) oder seinem reaktiven Derivat umfaßt.

25. Verfahren zum Herstellen einer Verbindung nach Anspruch 1, welches das Methoxylieren eines 7ξ-(fluorierten Methyl)-thioacetamido-3-(Z-substituiertes Methyl)-1-dethia-1-oxa-3-cephem-4-carbonsaurederivats mit der folgenden Formel

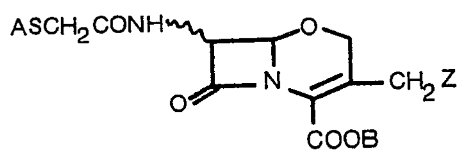

(worin A, B und Z wie in Anspruch 1 definiert sind) mit einem N-halogenierenden Reagenz, einem Halogenwasserstofffänger und Methanol aufeinanderfolgend umfaßt.

26. Verfahren zum Herstellen eines Salzes nach Anspruch 1, welches das Neutralisieren eines 7β-(fluorierten Methylthio)-acetamido-7α-methoxy-3-(Z-substituiertes Methyl)-1-dethia-1-oxa-3-cephem-4-carbonsäurederivats mit der folgenden Formel

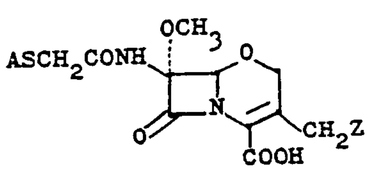

(worin A und Z wie in Anspruch 1 definiert sind) oder seinem reaktiven Derivat mit einer Base umfaßt.

27. Verfahren zum Herstellen einer Carbonsäure nach Anspruch 1, welches das Entestern eines 7β-(fluorierten Methylthio)-acetoamido-7α-methoxy-3-(Z-substituiertes Methyl)-1-dethia-1-oxa-3-cephem-4-carbonsäureesters mit der folgenden Formel

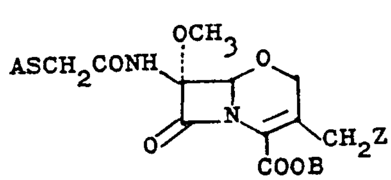

(worin A und Z wie in Anspruch 1 definiert sind und B ein esterbildender Rest ist) umfaßt.

28. Antibakterielle Zusammensetzung mit einer wirksamen Menge der Verbindung nach Anspruch 1 und einem herkömmlichen Träger.

29. Verfahren zur in vitro Bekämpfung von Bakterien, welche das Inkontaktbringen einer wirksamen Menge der Verbindung nach Anspruch 1 mit den Bakterien umfaßt.

**Revendications**

1. Acide 7β-(méthylthioacétamido fluoré)-7α-méthoxy-3-(méthyle substitué par un groupe nucléophile)-1-déthia-1-oxa-3-céphème-4-carboxylique ou ses sels et esters pharmaceutiquement acceptables représentés par la formule suivante:

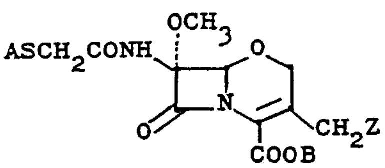

(dans laquelle)

A est un group monofluorométhyle ou difluorométhyle et

B est un atome d'hydrogène ou un métal formant un ion physiologiquement acceptable ou un groupe formant un ester protecteur du groupe carboxy pharmaceutiquement acceptable ayant 1 à 20 atomes de carbone; et

Z est un atome d'halogène ou un groupe acyloxy ou hétérocyclyle thio ayant jusqu'à 15 atomes de carbone, dans lequel la partie hétérocyclique du groupe hétérocyclyle thio est un groupe mono- ou dicyclique) sous réserve que Z n'est pas un groupe 1-(hydroxyalkyle éventuellement protégé) — 1H-tétrazol-5-yl-thio.

2. Composé selon la revendication 1, dans lequel A est un groupe difluorométhyle.

3. Composé selon la revendication 1, dans lequel B est un atome d'hydrogène ou un groupe formant un sel ou ester pharmaceutiquement acceptable.

4. Composé selon la revendication 1, dans lequel B est un groupe formant un ester protecteur du groupe carboxy.

5. Composé selon la revendication 1, dans lequel Z est un groupe alcanoyloxy, carbamoyloxy ou un atome d'halogène.

6. Composé selon la revendication 1, dans lequel Z est un groupe hétérocyclyle thio.

7. Composé selon la revendication 6, dans lequel la partie hétérocyclique a un système hétérocyclique éventuellement substitué ou hydrogéné choisi parmi l'imidazole, l'isothiazole, le pyrazolotétrazole, le pyrazolotriazole, le pyridotriazole, la pyrimidine, le tétrazole, le thiadiazole, le thiazole, la triazine et le triazole.

8. Composé selon la revendication 7, dans lequel le substituant facultatif sur la partie hétérocylique est choisi parmi les groupes acylamino, alcoxy, alkylthio, amino, carboxy ou carboxy protégé, cyano, hydroxy, oxo et pyridyle, et la partie hétérocyclique du groupe hétérocyclyle thio a jusqu'à 15 atomes de carbone y compris le substituant.

9. Composé selon la revendication 7, dans lequel le substituant facultatif sur l'hétérocyclique est un groupe alkyle en $C_1$ à $C_4$ linéaire, ramifié, cyclique ou insaturé, éventuellement substitué par un à trois groupes en $C_1$ à $C_{12}$ choisi parmi les groupes acylamino, acyloxy, alcoxy, alcoxycarbamoyle, alkylcarbamoyle, alkylthio, aroylamino, aryle, arylcarbamoyle, arylthio, carbamoyle, carbamoylalkylthio, carboxy, carboxy protégé, carboxyalcoxy, (carboxy protégé)alcoxy, cyano, dialkylamino, halo, carbamoyle N-hétérocyclique, hétérocyclique, hétérocyclyle thio, hydroxy, hydroxyimino, oxo, sulfamoyle, sulfo, uréido, et uréidocarbonyle.

10. Composé selon la revendication 1, dans lequel B est un atome d'hydrogène, un groupe acétoxyméthyle, benzyle, t-butyle, un atome de calcium, un groupe méthyldioxolènylméthyle, diphénylméthyle, éthoxycarbonyloxyéthyle, indanyle, un atome de magnésium, un groupe p-méthoxybenzyle, p-nitrobenzyle, phényle, phtalidyle, pivaloyloxyméthyle, un atome de potassium, un atome de sodium, un groupe 2,2,2-trichloréthyle, allyle et xylyle.

11. Composé selon la revendication 2, dans lequel B est un atome d'hydrogène, un groupe acétoxyméthyle, un atome de métal alcalin, un groupe diphényleméthyle, éthoxycarbonyloxyéthyle ou pivaloyloxyméthyle.

12. Composé selon la revendication 11, dans lequel Z est un groupe tétrazolylthio, thiadiazolylthio, triazinylthio ou triazolylthio substitués chacun par 1 ou 2 groupes alkyle en $C_1$ à $C_3$.

13. Composé selon la revendication 11, dans lequel Z est un groupe 1H-tétrazol-5-yle 1-substitué.

14. Composé selon la revendication 13, dans lequel le substituant est un groupe méthyle ou vinyle.

15. Composé selon la revendication 13, dans lequel le substituant est un groupe carbamoylméthyle ou 2-carbamoyléthyle.

16. Composé selon la revendication 13, dans lequel le substituant est un groupe cyanométhyle ou cyanoéthyle.

17. Composé selon la revendication 13, dans lequel le substituant est un groupe 2-oxoimidazolidin-1-yle.

18. Composé selon la revendication 13, dans lequel le substituant est un groupe 2-diméthylaminoéthyle.

19. Composé selon la revendication 13, dans lequel le substituant est un groupe chlorométhyle ou difluorométhyle.

20. Composé selon la revendication 13, dans lequel le substituant est un groupe 2-sulfamoyléthyle.

21. Composé selon la revendication 13, dans lequel le substituant est un groupe 2-méthanesulfonylaminoéthyle.

22. Composé selon la revendication 11, dans lequel Z est un groupe acétoxy, carbamoyloxy, carbamoyloxy N-protégé, ou halo.

23. Procédé de préparation d'un composé selon la revendication 1, qui comprend l'amidation d'un dérivé de l'acide 7β-amino-7α-méthoxy-3-(méthyle substitué par Z)-1-déthia-1-oxa-3-céphème-4-carboxylique représenté par la formule suivante:

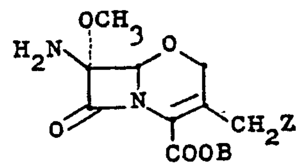

(dans laquelle B et Z sont tels que définis dans la revendication 1)
ou son dérivé réactif avec l'acide (méthylthio fluoré) acétique représenté par la formule: $ASCH_2COOH$ (dans laquelle A est tel que défini dans la revendication 1) ou son dérivé réactif.

24. Procédé de préparation d'un composé selon la revendication 1 qui comprend la substitution d'un dérivé de l'acide 7β-(méthylthio fluoré) acétamido-7α-méthoxy-3-halométhyl-1-déthia-1-oxa-3-céphème-4-carboxylique représenté par la formule suivante:

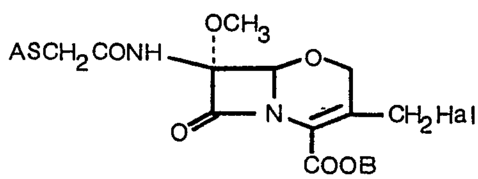

(dans laquelle A et B sont tels que définis dans la revendication 1 et Hal est un atome d'halogène) avec un thiol hétérocyclique représenté par la formule suivante:

HZ

(dans laquelle Z est un groupe thio hétérocyclique tel que défini dans la revendication 6) ou son dérivé réactif.

25. Procédé de préparation d'un composé selon la revendication 1, qui comprend la méthoxylation d'un dérivé de l'acide 7ξ-(méthyle fluoré)thioacétamido-3-(méthyle substitué par Z)-1-déthia-1-oxa-3-céphème-4-carboxylique représenté par la formule suivante:

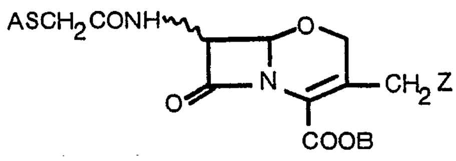

(dans laquelle A, B et Z sont tels que définis dans la revendication 1)
avec un réactif de N-halogénation, un fixateur d'halogènure d'hydrogène et du méthanol, successivement.

26. Procédé de préparation d'un sel selon la revendication 1, qui comprend la neutralisation d'un dérivé de l'acide 7β-(méthylthio fluoré)-acétamido-7α-méthoxy-3-(méthyle substitué par Z)-1-déthia-1-oxa-3-céphème-4-carboxylique représenté par la formule suivante:

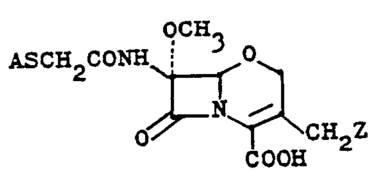

(dans laquelle A et Z sont tels que définis dans la revendication 1)
ou son dérivé réactif avec une base.

27. Procédé de préparation d'un acide carboxylique selon la revendication 1, qui comprend la désestérification d'un ester de l'acide 7β-(méthylthio fluoré) acétamido-7α-méthoxy-3-(méthyle substitué par Z)-1-déthia-1-oxa-3-céphème-4-carboxylique représententé par la formule suivante:

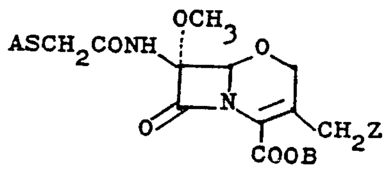

(dans laquelle A et Z sont tels que définis dans la revendication 1 et B est un groupe formant un ester).

28. Composition antibactérienne comprenant une quantité efficace du composé selon la revendication 1, et un support classique.

29. Procédé pour lutter contre les bactéries in vitro, qui comprend la mise en conctact d'une quantité efficace du composé selon la revendication 1, avec les bactéries.